(19) Europäisches Patentamt
European Patent Office
Office européen des brevets

(11) EP 2 144 915 B1

(12) **EUROPEAN PATENT SPECIFICATION**

(45) Date of publication and mention of the grant of the patent:
**19.03.2014 Bulletin 2014/12**

(21) Application number: **08748940.7**

(22) Date of filing: **16.04.2008**

(51) Int Cl.:
***C07D 491/20*** (2006.01) ***A61P 23/00*** (2006.01)
***A61P 25/00*** (2006.01) ***A61K 31/438*** (2006.01)

(86) International application number:
**PCT/EP2008/003043**

(87) International publication number:
**WO 2008/125349 (23.10.2008 Gazette 2008/43)**

(54) **SPIRO-PYRANO-PYRAZOLE DERIVATIVES**

SPIRO-PYRAN-PYRAZOL-DERIVATE

DÉRIVÉS DE SPIRO-PYRANO-PYRAZOLE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **16.04.2007 EP 07384021**

(43) Date of publication of application:
**20.01.2010 Bulletin 2010/03**

(73) Proprietor: **Laboratorios del Dr. Esteve S.A.**
**08041 Barcelona (ES)**

(72) Inventors:
- **WÜNSCH, Bernhard**
  **48161 Münster (DE)**
- **SCHEPMANN, Dirk**
  **48147 Münster (DE)**
- **SCHLÄGER, Torsten**
  **22297 Hamburg (DE)**
- **ZAMANILLO-CASTANEDO, Daniel**
  **E-08041 Barcelona (ES)**

(74) Representative: **Peters, Hajo**
**ZACCO GmbH**
**Bayerstrasse 83**
**80335 München (DE)**

(56) References cited:
**US-A1- 2006 047 127**

- **MAIER C A ET AL: "Novel Spiropiperidines as Highly Potent and Subtype Selective .sigma.-Receptor Ligands. Part 1" JOURNAL OF MEDICINAL CHEMISTRY, AMERICAN CHEMICAL SOCIETY. WASHINGTON, US, vol. 45, no. 2, 2002, pages 438-448, XP002388303 ISSN: 0022-2623 cited in the application**
- **SCHOW S R ET AL: "Novel sigma receptor ligands 2" BIOORGANIC AND MEDICINAL CHEMISTRY LETTERS 1993 UNITED KINGDOM, vol. 3, no. 2, 1993, pages 221-224, XP002450664 ISSN: 0960-894X**
- **BERARDI F ET AL: "novel potent s1 ligands: N-[w-(tetralin-1-yl)alkyl]piperidine derivatives" JOURNAL OF MEDICINAL CHEMISTRY, AMERICAN CHEMICAL SOCIETY. WASHINGTON, US, vol. 39, 1996, pages 4255-4260, XP002218227 ISSN: 0022-2623**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 144 915 B1

Printed by Jouve, 75001 PARIS (FR)

## Description

### FIELD OF THE INVENTION

The present invention relates to compounds having pharmacological activity towards the sigma (σ) receptor, and more particularly to some spiro-pyrano-pyrazole derivatives, to processes of preparation of such compounds, to pharmaceutical compositions comprising them, and to said compounds for use in therapy and prophylaxis, in particular for the treatment of psychosis.

### BACKGROUND OF THE INVENTION

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

There is still a need to find compounds that have pharmacological activity towards the sigma receptor, being both effective and selective, and having good "drugability" properties, i.e. good pharmaceutical properties related to administration, distribution, metabolism and excretion.

The closest technique known comprises benzimidazoles of WO2003035065 for the inhibition of kinases. Tetrahydro-pyranopyrazole compounds displaying cannabinoid modulating activity are disclosed in WO2007001939 and FR2875230. None of the them presents spiro-pyrano-pyrazole variants or analogues.

Spiropiperidines are known as potent ligands to sigma receptors (Maier et al, J Med Chem, 2002, 45, 438-448 and Maier et al, J Med Chem, 2002, 45, 4923-4930). However, such spiropiperidines show benzofuran and benzopyran rings.

### SUMMARY OF THE INVENTION

We have now found a family of structurally distinct spiro[benzopyran] or spiro[benzofuran] Derivatives which are particularly selective inhibitors of the sigma receptor.

The present specification relates to non-claimed compounds of general formula (I),

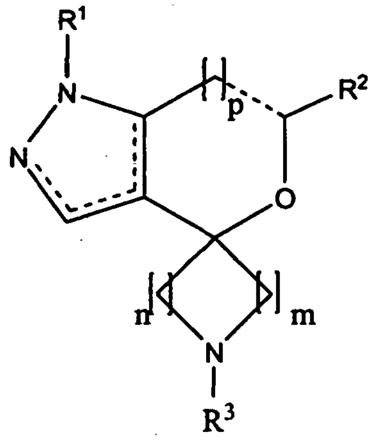

(I)

wherein

m is selected from 1, 2 or 3, n is selected from 1, 2 or 3, and m + n is either 3, 4 or 5;

p is selected from 0 or 1;

the dotted line ......... is either a double or a single bond;

if p is 1, the dotted line ......... is either a double or a single bond;

if p is 0, the dotted line ......... is a single bond;

$R^1$ is selected from hydrogen; at least mono-substituted, linear or branched $C_{1-6}$-aliphatic group; optionally at least monosubstituted aryl; optionally at least mono-subtituted alkyl-aryl;

$R^2$ is selected from hydrogen; an optionally at least monosubstituted, linear or branched $C_{1-6}$-aliphatic group; O-R with R being H or an optionally at least monosubstituted, linear or branched $C_{1-6}$-aliphatic group;

$R^3$ is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl; or COOR' with R' being either H or $C_{1-4}$-alkyl;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

A first aspect of the invention is directed to compounds of general formula Ia,

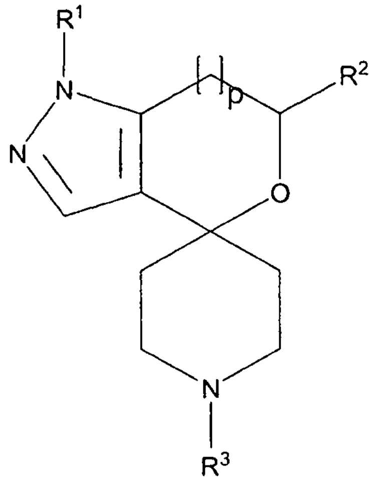

Ia

wherein

p is selected from 0 or 1;

R' is selected from linear or branched $C_{1-4}$-alkyl; or optionally at least monosubstituted aryl;

$R^2$ is selected from H; OH or a linear or branched $OC_{1-4}$-alkyl group;

$R^3$ is selected from hydrogen; $C_{1-10}$-alkyl, linear or branched; $C_{3-8}$-alkenyl, linear or branched; optionally at least mono-substituted $C_{1-6}$-alkyl-aryl; optionally at least mono-substituted $C_{1-6}$-alkyl-heterocyclyl; or optionally at least mono-substituted $C_{1-6}$-alkyl-$C_{4-8}$-cycloalkyl, wherein the cycloalkyl group can be saturated or unsaturated,

optionally in form of one of the stereoisomers, preferably enantiomers or diastereomers, a racemate or in form of a mixture of at least two of the stereoisomers, preferably enantiomers and/or diastereomers, in any mixing ratio,

or a corresponding salt thereof, or a corresponding solvate thereof,

wherein aryl or alkyl-aryl, cycloalkyl or alkyl-cycloalkyl, heterocyclyl or alkyl-heterocyclyl groups,are substituted on the ring-system of the aryl or alkyl-aryl, cycloalkyl or alkyl-cycloalkyl; heterocyclyl or alkyl-heterocyclyl by substituents selected from OH, SH, =O, halogen (F, Cl, Br, I), CN, $NO_2$, ,COOH; $NR_xR_y$, with $R_x$ and $R_y$ independently being either H or a saturated or unsaturated, linear or branched, substituted or unsubstituted $C_{1-6}$-alkyl; a saturated or unsaturated, linear or branched, substituted or unsubstituted - O-$C_{1-6}$-alkyl (alkoxy); a saturated or unsaturated, linear or branched, substituted or unsubstituted -S-$C_{1-6}$-alkyl; a saturated or unsaturated, linear or branched, substituted or unsubstituted C(O)- $C_{1-6}$-alkyl-group; a saturated or unsaturated, linear or branched, substituted or unsubstituted C(O)-O-$C_{1-6}$-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl,

and

wherein alkyls or alkenyls can be substituted or unsubstituted, wherein substitutents of alkyls or alkenyls are selected from the group consisting of F, Cl, Br, I, $NH_2$, SH or OH.

In the context of this invention aliphatic group or aliphatic radical includes alkyl, alkenyl and alkinyl.

In the context of this invention, alkyl radical or group is understood as meaning saturated, linear or branched hydrocarbons, which can be unsubstituted or mono- or polysubstituted. Alkenyl and alkinyl groups, on the other hand include groups like e.g. -CH=CH-$CH_3$ or -C≡C-$CH_3$, while the saturated alkyl encompasses e.g. -$CH_3$ and-$CH_2$-$CH_3$. In these radicals, $C_{1-2}$-alkyl represents C1- or C2-alkyl, $C_{1-3}$-alkyl represents C1-, C2- or C3-alkyl, $C_{1-4}$-alkyl represents C1-, C2-, C3- or C4-alkyl, $C_{1-5}$-alkyl represents C1-, C2-, C3-, C4-, or C5-alkyl, $C_{1-6}$-alkyl represents C1-, C2-, C3-, C4-, C5- or C6-alkyl, $C_{1-7}$-alkyl represents C1-, C2-, C3-, C4-, C5-, C6- or C7-alkyl, $C_{1-8}$-alkyl represents C1-, C2-, C3-, C4-,

C5-, C6-, C7- or C8-alkyl, $C_{1-10}$-alkyl represents C1-, C2-, C3-, C4-, C5-, C6-, C7-, C8-, C9- or C10-alkyl and $C_{1-18}$-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also $CHF_2$, $CF_3$ or $CH_2OH$ etc.

In the context of this invention cycloalkyl radical or group is understood as meaning saturated and unsaturated (but not aromatic) cyclic hydrocarbons (without a heteroatom in the ring), which can be unsubstituted or mono- or polysubstituted. Furthermore, $C_{3-4}$-cycloalkyl represents C3- or C4-cycloalkyl, $C_{3-5}$-cycloalkyl represents C3-, C4- or C5-cycloalkyl, $C_{3-6}$-cycloalkyl represents C3-, C4-, C5- or C6-cycloalkyl, $C_{3-7}$-cycloalkyl represents C3-, C4-, C5-, C6- or C7-cycloalkyl, $C_{3-8}$-cycloalkyl represents C3-, C4-, C5-, C6-, C7- or C8-cycloalkyl, $C_{4-5}$-cycloalkyl represents C4- or C5-cycloalkyl, $C_{4-6}$-cycloalkyl represents C4-, C5- or C6-cycloalkyl, $C_{4-7}$-cycloalkyl represents C4-, C5-, C6- or C7-cycloalkyl, $C_{5-6}$-cycloalkyl represents C5- or C6-cycloalkyl and $C_{5-7}$-cycloalkyl represents C5-, C6- or C7-cycloalkyl. However, mono- or polyunsaturated, preferably monounsaturated, cycloalkyls also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantly.

In the context of this invention alkyl-cycloalkyl is understood as meaning a cycloalkyl group (see above) being connected to another atom through a $C_{1-6}$-alkyl group (see above), whereas the $C_{1-6}$-alkyl-group is always saturated and unsubstituted, and linear or branched.

In connection with alkyl or aliphatic group - unless defined otherwise - the term substituted in the context of this invention is understood as meaning replacement of at least one hydrogen radical by F, Cl, Br, I, $NH_2$, SH or OH, "polysubstituted" (more than once substituted) radicals being understood as meaning that the replacement takes effect both on different and on the same atoms several times with the same or different substituents, for example three times on the same C atom, as in the case of $CF_3$, or at different places, as in the case of e.g. $-CH(OH)-CH=CH-CHCl_2$. optionally at least monosubstituted" means either "monosubstituted", "polysubstituted" or - if the option is not fulfilled - "unsubstituted".

The term $(CH_2)_{3-6}$ is to be understood as meaning $-CH_2-CH_2-CH_2-$, $-CH_2-CH_2-CH_2-CH_2-$, $-CH_2-CH_2-CH_2-CH_2-CH_2-$ and $-CH_2-CH_2-CH_2-CH_2-CH_2CH_2-$, $(CH_2)_{1-4}$ is to be understood as meaning $-CH_2-$, $-CH_2-CH_2-$, $-CH_2-CH_2-CH_2-$ and $-CH_2-CH_2-CH_2-CH_2-$, $(CH_2)_{4-5}$ is to be understood as meaning $-CH_2-CH_2-CH_2-CH_2-$ and $-CH_2-CH_2-CH_2-CH_2-CH_2-$, etc.

An aryl radical or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention alkyl-aryl is understood as meaning an aryl group (see above) being connected to another atom through a $C_{1-6}$-alkyl-group (see above), whereas the $C_{1-6}$-alkyl-group is always saturated and unsubstituted, and linear or branched.

A heterocyclyl radical or group is understood as meaning heterocyclic ring systems, saturated or unsaturated ring which contains one or more heteroatoms from the group consisting of nitrogen, oxygen and/or sulfur in the ring and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In the context of this invention alkyl-heterocylyl is understood as meaning a heterocyclyl group (see above) being connected to another atom through a $C_{1-6}$-alkyl group (see above), whereas the $C_{1-6}$-alkyl-group is always saturated and unsubstituted, and linear or branched.

In connection with aryl or alkyl-aryl, cycloalkyl or alkyl-cycloalkyl, heterocyclyl or alkyl-heterocyclyl, substituted is understood - unless defined otherwise - as meaning substitution of the ring-system of the aryl or alkyl-aryl, cycloalkyl or alkyl-cycloalkyl; heterocyclyl or alkyl-heterocyclyl by OH, SH, =O, halogen (F, Cl, Br, I), CN, $NO_2$, COOH; $NR_xR_y$, with $R_x$ and $R_y$ independently being either H or a saturated or unsaturated, linear or branched, substituted or unsubstituted $C_{1-6}$-alkyl; a saturated or unsaturated, linear or branched, substituted or unsubstituted $C_{1-6}$-alkyl; a saturated or unsaturated, linear or branched, substituted or unsubstituted $-O-C_{1-6}$alkyl (alkoxy); a saturated or unsaturated, linear or branched, substituted or unsubstituted $-S-C_{1-6}$alkyl; a saturated or unsaturated, linear or branched, substituted or unsubstituted - $C(O)-C_{1-6}$-alkyl-group; a saturated or unsaturated, linear or branched, substituted or unsubstituted "$C(O)-O-C_{1-6}$-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl. "Optionally at least monosubstituted" means either "monosubstituted", "polysubstituted" or - if the option is not fulfilled - "unsubstituted".

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids and in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by $^{13}C$- or $^{14}C$-enriched carbon or $^{15}N$-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, or solvates.

In a non-claimed embodiment of the compound according to general formula I $R^1$ is selected from hydrogen; at least mono-substituted, linear or branched $C_{1-6}$-aliphatic group; optionally at least monosubstituted aryl; especially $R^1$ is selected from linear or branched $C_{1-4}$-alkyl; or optionally at least monosubstituted aryl; more preferably $R^1$ is selected from $CH_3$ or phenyl.

In another non-claimed embodiment of the compound according to general formula I $R^2$ is selected from OR with R being H or an optionally at least monosubstituted, linear or branched $C_{1-6}$-aliphatic group; especially $R^2$ is selected from OR with R being H or a linear or branched $C_{1-4}$-alkyl group, more preferably $R^2$ is selected from OH or $OCH_3$.

In another non-claimed embodiment of the compound according to general formula I $R^3$ is selected from H; an optionally at least monosubstituted, linear or branched $C_{1-18}$-aliphatic group; an optionally at least monosubstituted alkyl-aryl; an optionally at least monosubstituted alkyl-heterocyclyl; or an optionally at least monosubstituted alkyl-cycloalkyl.

In a non-claimed embodiment of the compound according to general formula I m and n are selected from 1 or 2 and m+n are selected from 3 or 4.

In a highly preferred embodiment of the compound according to the invention the compounds are compounds according to general formula Ia

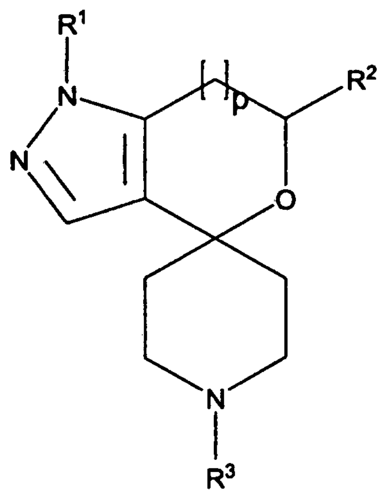

Ia

wherein

p is selected from 0 or 1;

$R^1$ is selected from $CH_3$ or phenyl;

$R^2$ is selected from H; OH or $OCH_3$;

$R^3$ is selected from hydrogen; $C_{1-10}$-alkyl, linear or branched; $C_{3-8}$-alkenyl, linear or branched; optionally at least mono-substituted $C_{1-6}$-alkyl-aryl; optionally at least mono-substituted $C_{1-6}$-alkyl-heterocyclyl; or optionally at least mono-substituted $C_{1-6}$-alkyl-$C_{4-8}$-cycloalkyl.

Referring to and repeating the definition given above the term "substituted" in the context of an alkyl group is understood as meaning replacement of at least one hydrogen radical by F, Cl, Br, I, $NH_2$, SH or OH. "Optionally at least monosubstituted" means either "monosubstituted", "polysubstituted" or - if the option is not fulfilled - "unsubstituted", with "polysubstituted" (more than once substituted) being understood as meaning that the replacement takes effect both on different and on the same atoms several times with the same or different substituents, for example three times on the same C atom, as in the case of $CF_3$, or at different places, as in the case of e.g. -CH(OHFCH=CH-$CHCl_2$.

Referring to and repeating the definition given above the term "substituted" in the context of an aryl or heterocyclyl is understood as meaning substitution of the ring-system of the aryl or heterocyclyl by OH, SH, =O, halogen (F, Cl, Br, I), CN, $NO_2$, COOH; $NR_xR_y$, with $R_x$ and $R_y$ independently being either H or a saturated or unsaturated, linear or branched, substituted or unsubstituted $C_{1-6}$-alkyl; a saturated or unsaturated, linear or branched, substituted or unsubstituted $C_{1-6}$-alkyl; a saturated or unsaturated, linear or branched, substituted or unsubstituted -O-$C_{1-6}$-alkyl (alkoxy); a saturated or unsaturated, linear or branched, substituted or unsubstituted -S-$C_{1-6}$alkyl; a saturated or unsaturated, linear or branched, substituted or unsubstituted - C(O)-$C_{1-6}$-alkyl-group; a saturated or unsaturated, linear or branched, substituted or unsubstituted -C(O)-O-$C_{1-6}$-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl. "Optionally at least monosubstituted" means either "monosubstituted", "polysubstituted" or - if the option is not fulfilled - "unsubstituted".

In another preferred embodiment of the compound according to the invention the compounds are compounds according to general formula Ia

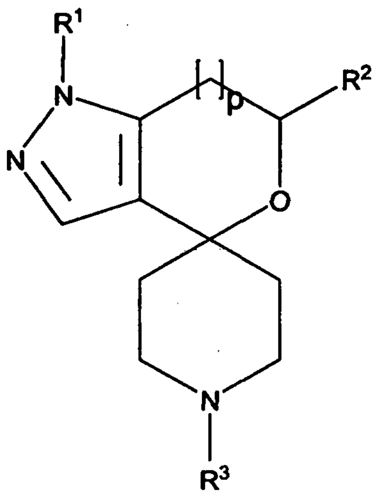

Ia

wherein

p is selected from 0 or 1;

$R^1$ is selected from $CH_3$ or phenyl;

$R^2$ is selected from H; OH or $OCH_3$;

$R^3$ is selected from $C_{1-10}$-alkyl, linear or branched; $C_{3-8}$-alkenyl, linear or branched; $C_{1-6}$-alkyl-aryl optionally at least mono-substituted with halogen, $C_{1-6}$-alkyl, O-$C_{1-6}$-alkyl, OH or $CF_3$; $C_{1-6}$-alkyl-heterocyclyl optionally at least mono-substituted with halogen, $C_{1-6}$-alkyl, O-$C_{1-6}$-alkyl, OH or $CF_3$; or $C_{1-6}$-alkyl-$C_{4-8}$-cycloalkyl optionally at least mono-substituted with halogen, $C_{1-6}$-alkyl, O-$C_{1-6}$-alkyl, OH or $CF_3$.

In another preferred embodiment of the compound according to the invention the compounds according to general formula I are selected from:

- 6'-Methoxy-1'-phenyl-4',6'-dihydro-1'H-spiro[piperidin-4,4'-furo[3,4-c]pyrazole];
- 1-Benzyl-6'-methoxy-1'-phenyl-4',6'-dihydro-1'H-spiro[piperidin-4,4'-furo[3,4-c]pyrazole];
- 1-Butyl-6'-methoxy-1'-phenyl-4',6'-dihydro-1'H-spiro[piperidin-4,4'-furo[3,4-c]pyrazole];
- 1-Benzyl-6'-methoxy-1'-phenyl-6',7'-dihydro-1'H-spiro[piperidin-4,4'-pyrano[4,3-c]pyrazole];
- 6'-Methoxy-1'-phenyl-1-propyl-6',7'-dihydro-1'H-spiro[piperidin-4,4'-pyrano[4,3-c]pyrazole];
- 1-Isopropyl-6'-methoxy-1'-phenyl-6',7'-dihydro-1'H-spiro[piperidin-4,4'-pyrano[4,3-c]pyrazole];
- 1-Butyl-6'-methoxy-1'-phenyl-6',7'-dihydro-1'H-spiro[piperidin-4,4'-pyrano[4,3-c]pyrazole];
- 1-Isobutyl-6'-methoxy-1'-phenyl-6',7'-dihydro-1'H-spiro[piperidin-4,4'-pyrano[4,3-c]pyrazole];
- 6'-Methoxy-1-pentyl-1'-phenyl-6',7'-dihydro-1'H-spiro[piperidin-4,4'-pyrano[4,3-c]pyrazole];
- 1-Isopentyl-6'-methoxy-1'-phenyl-6',7'-dihydro-1'H-spiro[piperidin-4,4'-pyrano[4,3-c]pyrazole];
- 6'-Methoxy-1-(3-methy)but-2-en-1-yl)-1'-phenyl-6',7'-dihydro-1'H-spiro[piperidin-4,4'-pyrano[4,3-c]pyrazole];

- 6'-Methoxy-1-octyl-1'-phenyl-6',7'-dihydro-1'H-spiro[piperidin-4,4'-pyrano[4,3-c]pyrazole];
- 1-(Cyclohexan-1-ylmethyl)-6'-methoxy-1'-phenyl-6',7'-dihydro-1'H-spiro[piperidin-4,4'-pyrano[4,3-c]pyrazole];
- 6'-Methoxy-1'-phenyl-1-(2-phenylethyl)-6',7'-dihydro-1'H-spiro[piperidin-4,4'-pyrano[4,3-c]pyrazole];
- 6'-Methoxy-1'-phenyl-1-(3-phenylpropyl)-6',7'-dihydro-1'H-spiro[piperidin-4,4'-pyrano[4,3-c]pyrazole];
- 6'-Methoxy-1'-phenyl-1-(4-phenylbutyl)-6',7'-dihydro-1'H-spiro[piperidin-4,4'-pyrano[4,3-c]pyrazole];
- 1-(Furan-2-ylmethyl)-6'-methoxy-1'-pheny)-6',7'-dihydro-1'H-spiro[piperidin-4,4'-pyrano[4,3-c]pyrazole];
- 6'-Methoxy-1-(4-methoxybenzyl)-1'-phenyl-6',7'-dihydro-1'H-spiro[piperidin-4,4'-pyrano(4,3-c]pyrazole];
- 1-(4-Fluorbenzyl)-6'-methoxy-1'-phenyl-6',7'-dihydro-1'H-spiro[piperidin-4,4'-pyrano[4,3-c]pyrazole];
- 1-Benzyl-6'-methoxy-1'-methyl-6',7'-dihydro-1'H-spiro[piperidin-4,4'-pyrano[4,3-c]pyrazole];
- 6'-Methoxy-1'-methyl-6',7'-dihydro-1'H-spiro[piperidin-4,4'-pyrano[4,3-c]pyrazole];
- 6'-Methoxy-1'-methyl-1-(3-phenylpropyl)-6',7'-dihydro-1'H-spiro[piperidin-4,4'-pyrano[4,3-c]pyrazole];
- 1-(4-Fluorbenzyl)-6'-methoxy-1'-methyl-6',7'-dihydro-1'H-spiro[piperidin-4,4'-pyrano[4,3-c]pyrazole];
- 1-Isopentyl-6'-methoxy-1'-methyl-6',7'-dihydro-1'H-spiro[piperidin-4,4'-pyrano[4,3-c]pyrazole];
- 6'-Methoxy-1'-methyl-1-propyl-6',7'-dihydro-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]-6'-ol;
- 1-Benzyl-1'-methyl-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]; or
- 1'-Methyl-6',7'-dihydro-1'H-spiro[piperidin-4,4'-pyrano[4,3-c]pyrazole];
- 1-Benzyl-6'-hydroxy-1'-phenyl-6',7'-dihydro-1'H-spiro[piperidin-4,4'-pyrano[4,3-c]pyrazole];
- 1-Benzyl-1'-phenyl-6',7'-dihydro-1'H-spiro[piperidin-4,4'-pyrano[4,3-c]pyrazole];
- 1'-phenyl-6',7'-dihydro-1'H-spiro[piperidin-4,4'-pyrano[4,3-c]pyrazole];
- 1-Isopentyl-1'-phenyl-6',7'-dihydro-1'H-spiro[piperidin-4,4'-pyrano[4,3-c]pyrazole];
- 1-(3-methylbut-2-en-1-yl)-1'-phenyl-6',7'-dihydro-1'H-spiro[piperidin-4,4'-pyrano[4,3-c]pyrazole];
- 1'-phenyt-1-(3-phenylpropyl)-6',7'-dihydro-1'H-spiro[piperidin-4,4'-pyrano[4,3-c]pyrazole];
- 1-Isopentyl-1'-methyl-6',7'-dihydro-1'H-spiro[piperidin-4,4'-pyrano[4,3-c]pyrazole];
- 1-(3-methylbut-2-en-1-yl)-1'-methyl-6',7'-dihydro-1'H-spiro[piperidin-4,4'-pyrano[4,3-c]pyrazole];
- 1'-methyl-1-(3-phenylpropyl)-6',7'-dihydro-1'H-spiro[piperidin-4,4'-pyrano[4,3-c]pyrazole]; or also
- 1-(Cyclohexan-1-ylmethyl)-1'-phenyl-6',7'-dihydro-1'H-spiro[piperidin-4,4'-pyrano[4,3-c]pyrazole];

optionally in form of one of the stereoisomers, preferably enantiomers or diastereomers, a racemate or in form of a mixture of at least two of the stereoisomers, preferably enantiomers and/or diastereomers, in any mixing ratio, or a

corresponding salt thereof, or a corresponding solvate thereof;
preferably from

- 6'-Methoxy-1'-phenyl-4',6'-dihydro-1'H-spiro[piperidin-4,4'-furo[3,4-c]pyrazole];
- 1-Benzyl-6'-methoxy-1'-phenyl-4',6'-dihydro-1'H-spiro[piperidin-4,4'-furo[3,4-c]pyrazole];
- 1-Butyl-6'-methoxy-1'-phenyl-4',6'-dihydro-1'H-spiro[piperidin-4,4'-furo[3,4-c]pyrazole];
- 1-Benzyl-6'-methoxy-1'-phenyl-6',7'-dihydro-1'H-spiro[piperidin-4,4'-pyrano[4,3-c]pyrazole];
- 6'-Methoxy-1'-phenyl-1-propyl-6',7'-dihydro-1'H-spiro[piperidin-4,4'-pyrano[4,3-c]pyrazole];
- 1-Isopropyl-6'-methoxy-1'-phenyl-6',7'-dihydro-1'H-spiro[piperidin-4,4'-pyrano[4,3-c]pyrazole];
- 1-Butyl-6'-methoxy-1'-phenyl-6',7'-dihydro-1'H-spiro[piperidin-4,4'-pyrano[4,3-c]pyrazole];
- 1-Isobutyl-6'-methoxy-1'-phenyl-6',7'-dihydro-1'H-spiro[pipeidin-4,4'-pyrano[4,3-c]pyrazole];
- 6'-Methoxy-1-pentyl-1'-phenyl-6',7'-dihydro-1'H-spiro[piperidin-4,4'-pyrano[4,3-c]pyrazole];
- 1-Isopentyl-6'-methoxy-1'-phenyl-6',7'-dihydro-1'H-spiro[piperidin-4,4'-pyrano[4,3-c]pyrazole];
- 6'-Methoxy-1-(3-methylbut-2-en-1-yl)-1'-phenyl-6',7'-dihydro-1'H-spiro[piperidin-4,4'-pyrano[4,3-c]pyrazole];
- 6'-Methoxy-1-octyl-1'-phenyl-6',7'-dihydro-1'H-spiro[piperidin-4,4'-pyrano[4,3-c]pyrazole];
- 1-(Cyclohexan-1-ylmethyl)-6'-methoxy-1'-phenyl-6',7'-dihydro-1'H-spiro[piperidin-4,4'-pyrano[4,3-c]pyrazole];
- 6'-Methoxy-1'-phenyl-1-(2-phenylethyl)-6',7'-dihydro-1'H-spiro[piperidin-4,4'-pyrano[4,3-c]pyrazole];
- 6'-Methoxy-1'-phenyl-1-(3-phenylpropyl)-6',7'-dihydro-1'H-spiro[piperidin-4,4'-pyrano[4,3-c]pyrazole];
- 6'-Methoxy-1'-phenyl-1-(4-phenylbutyl)-6',7'-dihydro-1'H-spiro[piperidin-4,4'-pyrano[4,3-c]pyrazole];
- 1-(Furan-2-ylmethyl)-6'-methoxy-1'-phenyl-6',7'-dihydro-1'H-spiro[piperidin-4,4'-pyrano[4,3-c]pyrazole];
- 6'-Methoxy-1-(4-methoxybenzyl)-1'-phenyl-6',7'-dihydro-1'H-spiro[piperidin-4,4'-pyrano[4,3-c]pyrazole];
- 1-(4-Fluorbenzyl)-6'-methoxy-1'-phenyl-6',7'-dihydro-1'H-spiro[piperidin-4,4'-pyrano[4,3-c]pyrazole];
- 1-Benzyl-6'-methoxy-1'-methyl-6',7'-dihydro-1'H-spiro[piperidin-4,4'-pyrano[4,3-c]pyrazole];
- 6'-Methoxy-1'-methyl-6',7'-dihydro-1'H-spiro[piperidin-4,4'-pyrano[4,3-c]pyrazole];
- 6'-Methoxy-1'-methyl-1-(3-phenylpropyl)-6',7'-dihydro-1'H-spiro[piperidin-4,4'-pyrano[4,3-c]pyrazole];
- 1-(4-Fluorbenzyl)-6'-methoxy-1'-methyl-6',7'-dihydro-1'H-spiro[piperidin-4,4'-pyrano[4,3-c]pyrazole];
- 1-Isopentyl-6'-methoxy-1'-methyl-6',7'-dihydro-1'H-spiro[piperidin-4,4'-pyrano[4,3-c]pyrazole];
- 6'-Methoxy-1'-methyl-1-propyl-6',7'-dihydro-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]-6'-ol;
- 1-Benzyl-1'-methyl-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]; or

- 1'-Methyl-6',7'-dihydro-1'H-spiro[piperidin-4,4'-pyrano[4,3-c]pyrazole];

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

The term "pharmacological tool" refers to the property of compounds of the invention through which they are particularly selective ligands for Sigma receptors which implies that compound of formula (I), described in this invention, can be used as a model for testing other compounds as sigma ligands, ex. a radiactive ligands being replaced, and can also be used for modeling physiological actions related to sigma receptors.

The compounds of the present invention represented by the above described formula (I) may include enantiomers depending on the presence of chiral centres or isomers depending on the presence of multiple bonds (e.g. Z, E). The single enantiomers or diastereoisomers and mixtures thereof fall within the scope of the present invention.

In general the processes are described below in the experimental part. The starting materials are commercially available or can be prepared by conventional methods.

Herein described is also a process for the production of a compound according to the invention according to general formula I, wherein a compound of formula III

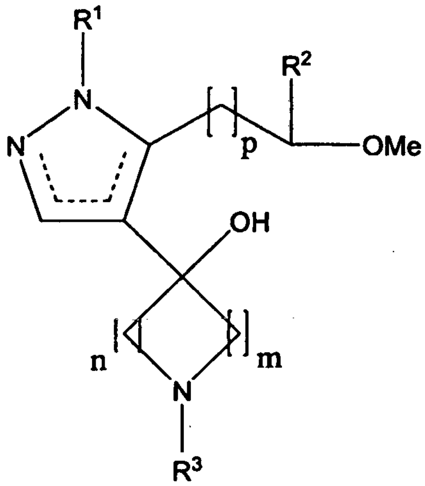

,

wherein $R^1$, $R^2$, $R^3$, m, n and p and the dotted line are as defined above is reacted with acid, preferably p-toluol sulphonic acid, to form a compound according the formula I. It is preferred if in this process in the compound according to formula III

- $R^3$ is C(O)OR' or benzyl; and/or
- $R^2$ is $OCH_3$; and/or
- $R^1$ is $C_{1-6}$alkyl or aryl; and/or
- m and n are 2.

A preferred aspect of the invention is also a process for the production of a compound according to the invention according to general formula Ia, wherein a compound of formula IIIa

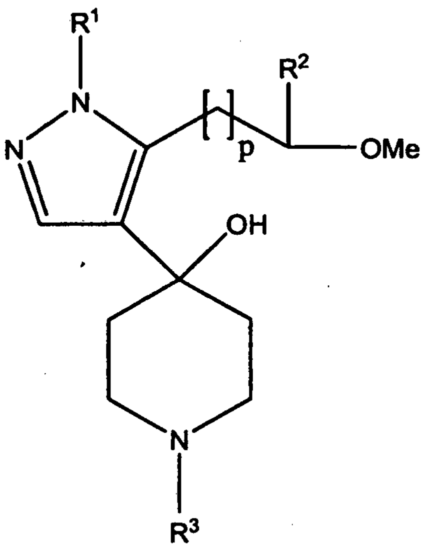

,

wherein $R^1$, $R^2$, $R^3$ and p are as defined above is reacted with acid, preferably p-toluol sulphonic acid, to form acompound according the formula Ia. It is preferred if in the compound according to general formula IIIa

- $R^3$ is C(O)OR' or benzyl; and/or
- $R^2$ is $OCH_3$; and/or
- $R^1$ is $C_{1-6}$alkyl or aryl.

It is further preferred if in the process above as a next step a compound according to formula I or Ia in which p is 0 and $R^3$ is C(O)OR' is reacted with a KOH solution in water to form a compound according to formulas I or Ia with $R^3$ being H.

It is further preferred if in the process above as a next step a compound according to formula I or Ia in which p is 1 and $R^3$ is benzyl is reacted with $NH_4^+$ $HCOO^-$ with a Pd/C catalysator to form a compound according to formulas I or Ia with $R^3$ being H.

It is further preferred if in the process above as a next step a compound according to formula I or Ia in which $R^3$ is hydrogen is reacted with a compound $R^3$-X with X being a leaving group and $K_2CO_3$ under reflux to form a compound according to formulas I or Ia with $R^3$ being as defined above, except H.

The obtained reaction products may, if desired, be purified by conventional methods, such as crystallisation and chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in pharmaceutical composition. In the case of salts and solvates the additional ionic and solvent moieties must also be non-toxic. The compounds of the invention may present different polymorphic forms, it is intended that the invention encompasses all such forms.

Another aspect of the invention refers to a pharmaceutical composition which comprises a compound according to the invention or a pharmaceutically acceptable salt, isomer or solvate thereof, and a pharmaceutically acceptable carrier, adjuvant or vehicle. The present invention thus provides pharmaceutical compositions comprising a compound of this invention, or a pharmaceutically acceptable salt, or stereoisomers thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example

magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Another aspect of the invention refers to the use of a compound according to the invention in the manufacture of a medicament.

Another aspect of the invention refers to the use of a compound according to the invention in the manufacture of a medicament for the treatment or prophylaxis of a sigma receptor mediated disease or condition. A further related aspect of the invention refers to the use of a compound according to the invention for the treatment or prophylaxis of a sigma receptor mediated disease or condition. A preferred embodiment of this is this use wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer.

A preferred embodiment of this is this use wherein the disease is pain, especially neuropathic pain, inflammatory pain or other pain conditions, allodynia and/or hyperalgesia, especially mechanical allodynia.

Another aspect of the invention refers to the use of a compound according o the invention as pharmacological tool or as anxiolytic or immunosuppressant.

The term "pharmacological tool" refers to the property of compounds of the invention through which they are particularly selective ligands for Sigma receptors which implies that compound of formula I, described in this invention, can be used as a model for testing other compounds as Sigma ligands, ex. a radiactive ligands being replaced, and can also be used for modeling physiological actions related to Sigma receptors.

The compounds of the invention may be synthesized following one of the three Reaction Schemes (A, B, C or D) set out below, wherein R is equivalent to $R^3$:

**Reaction Scheme A:**

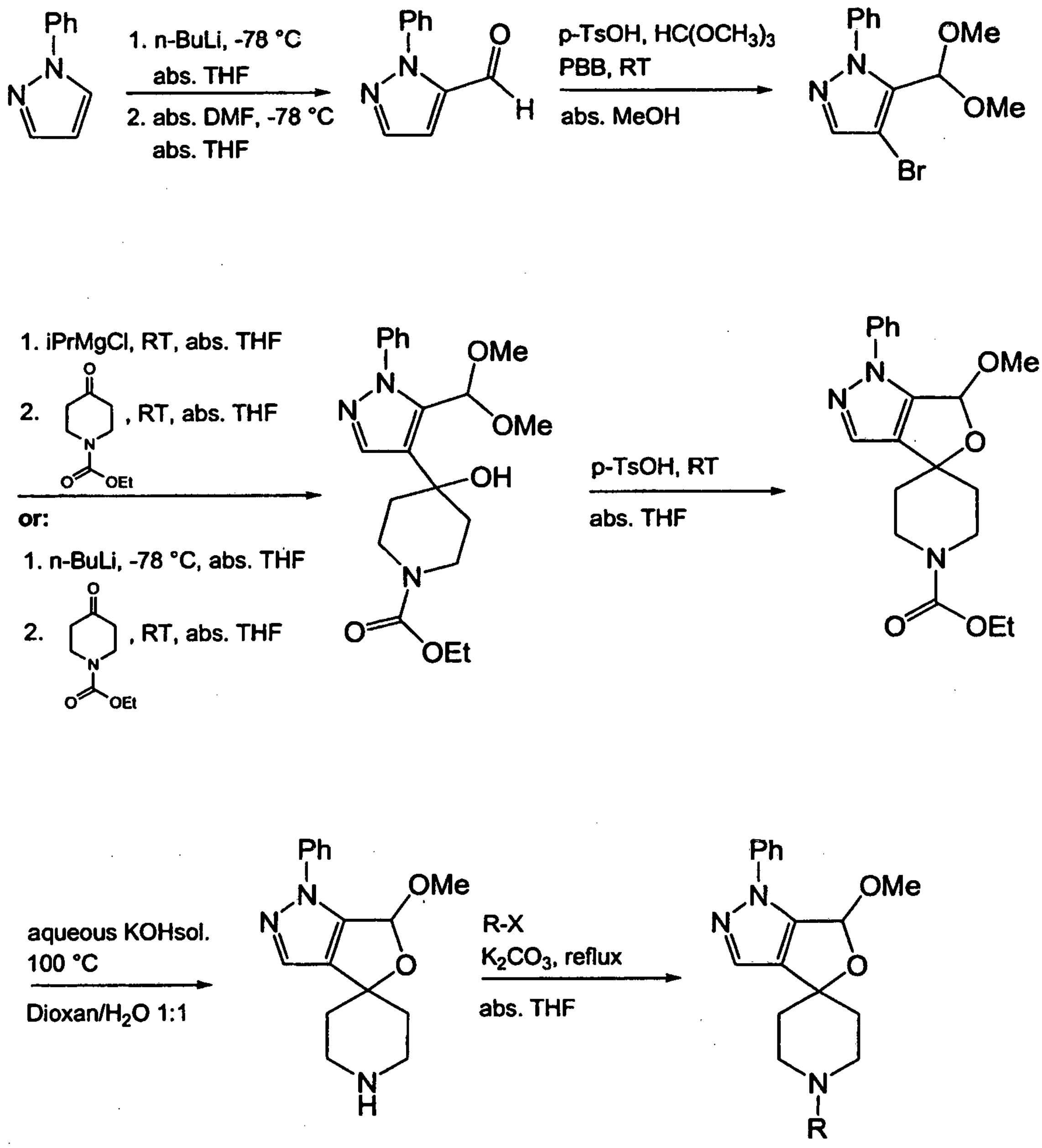

**Reaction Scheme B:**

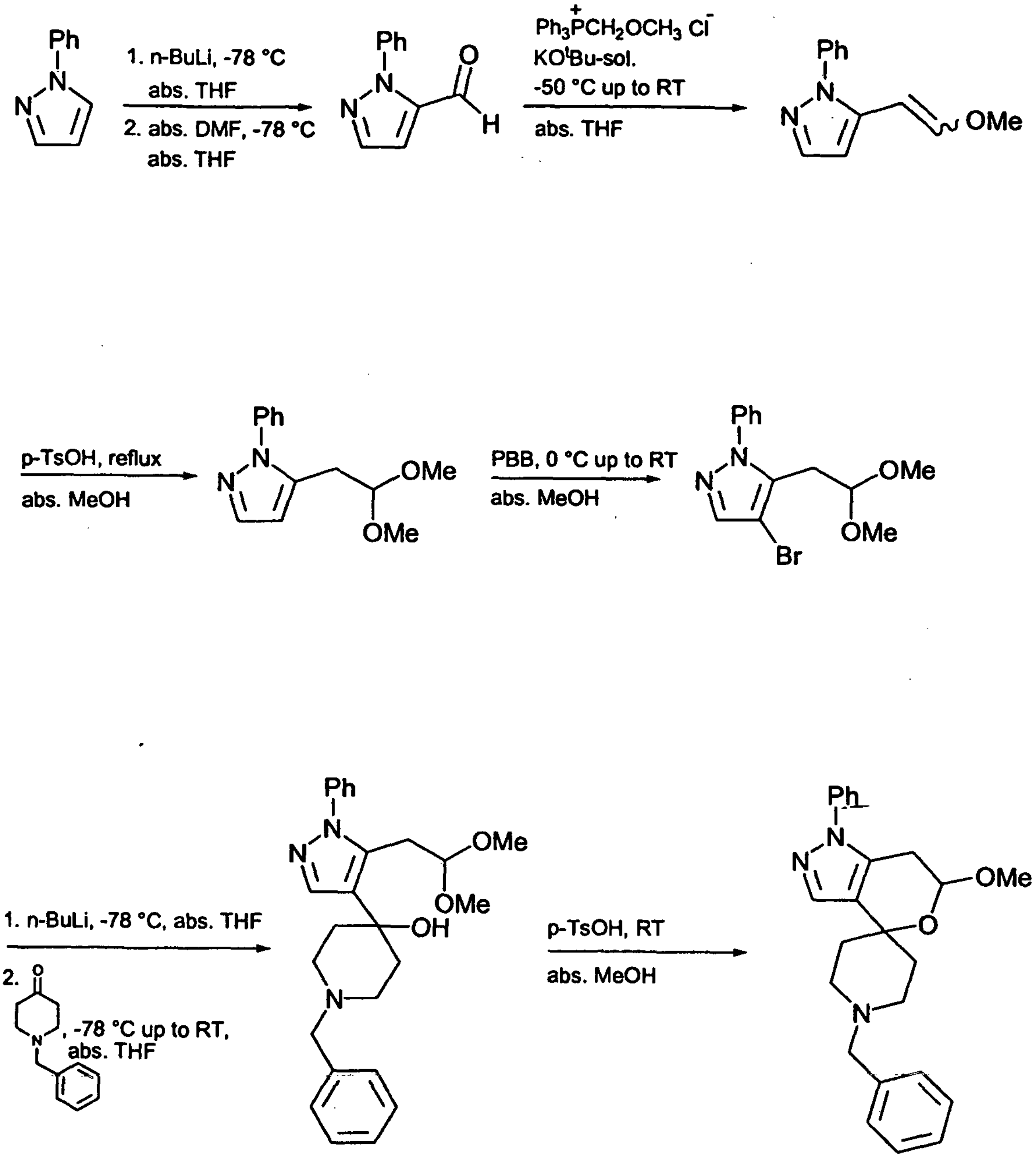

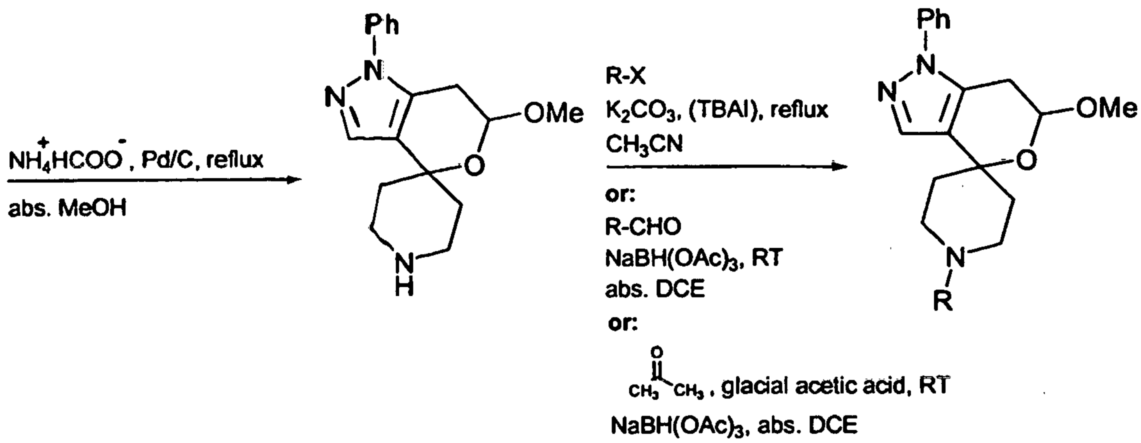


## Reaction Scheme C:

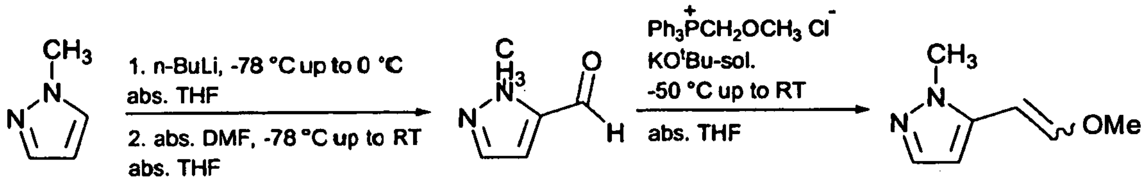

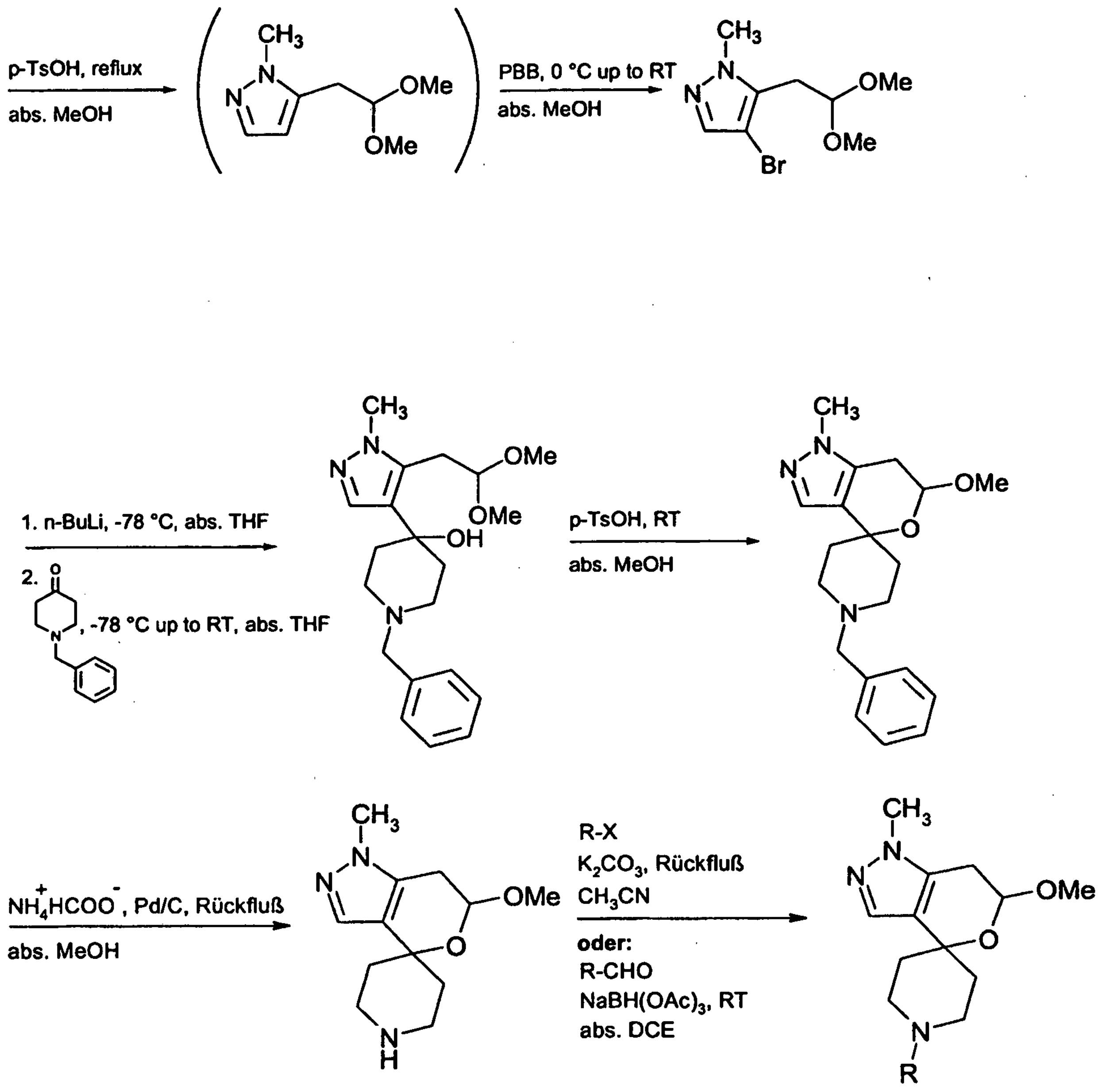


## Reaction Scheme D:

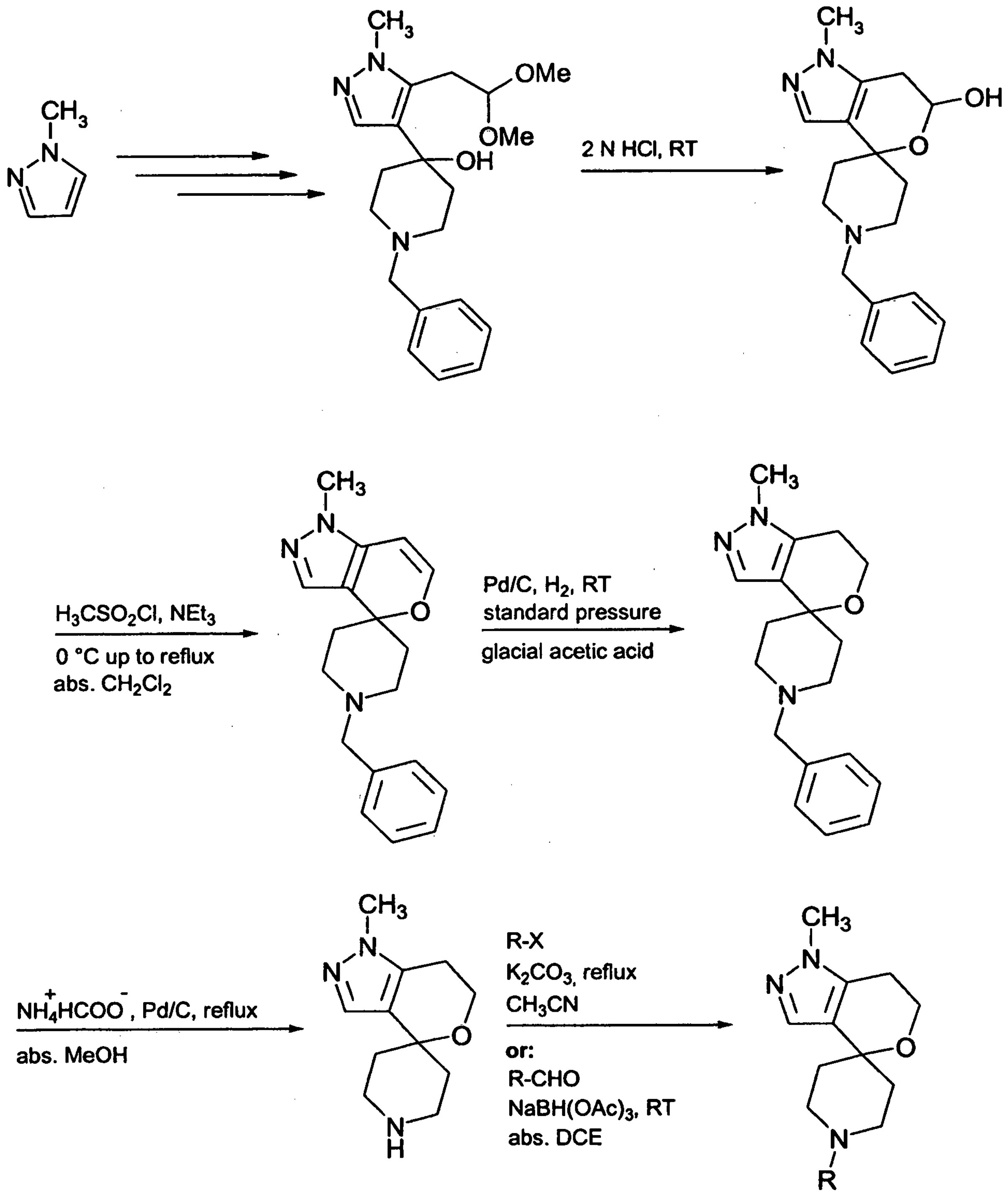


The present invention is illustrated below with the aid of examples. These illustrations are given solely by way of example.

## EXAMPLES:

### General Experimental Part (Methods and Equipment of the synthesis and analysis

All solvents used for synthesis were p. a. quality.

The separation of mixtures of substances using thin-layer chromatography was done on glass plates layered by silica gel. Analysis of the plates was done after treatment with iodine gas or incubation with Dragendorffs reagent under UV light.

As a rule synthesized products were purified using flash-chromatography.

Melting points were measured using capillaries. As sometimes the products were mixtures of diastereoisomers, themelting point: had to be expressed as a range.

IR-spectra were measured using the FT-IR-480 Plus Fourier Transform Spectrometer with ATR (Fa. Jasco). All substances were either measured directly as solids or in oil.

NMR-Spectra were measured using Mercury-400BB (Fa. Varian) at a temperature of 21°C. δ, measured in ppm, is based on the signal TMS measured in comparison to the residue signal ($CHCl_3$) of the solvent ($CDCl_3$):

$^1$H-NMR-Spectroscopy:

$$\delta\ (TMS) = \delta\ (CHCl_3) - 7.26$$

$^{13}$C-NMR-Spectroscopy:

$$\delta\ (TMS) = \delta\ (CHCl_3) - 77.0$$

Mass-spectra (MS) were measured using the GCQ Finnigan MAT (Fa. Finnigan) with Xcalibur Version 1.1. The method of ionisation is shown in brackets: EI = electronic ionisation (70 eV); CI = Chemical ionisation (Isobutane or $NH_3$, 170 eV).

Elementary analysis was done with VarioEL (Fa. Elementar).

Before using HPLC the maximum absorption of the substances was measured using the UV/Vis-Spectra done with a 50Bio Cary Spektrophotometer (Fa. Varian).

For determination of the purity and for the separation of the diastereomers a HPLC Hitachi L6200A Intelligent Pump with a UV-Detector (Merck) was used.

Synthesis was done in the synthetic microwave Discover (Fa. CEM).

Some reactions were done using protective gas.

Reactions at -78°C were done in an acetone bath in a Dewar.

<u>**Example A: 1-Phenylpyrazole-5-carbaldehyd**</u>

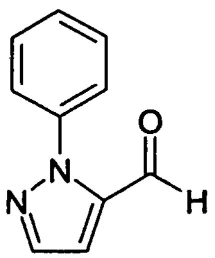

Experimental procedure:

Under $N_2$-atmosphere a solution of n-butyllithium in heptane (2.61 M, 5.3 mL, 13.9 mmol) is slowly added to a cooled (-78 °C) solution of 1-phenyl-1H-pyrazole (2.0 g, 13.9 mmol) in abs. THF (60 mL). The reaction mixture was stirred for 2 h at -78 °C. Subsequently a Solution of abs. DMF (1.1 mL, 13.9 mmol) in abs. THF (8 mL) is slowly added at -78 °C. This mixture was stirred for 1 h at -78 °C, before it was slowly heated to room temperature and was stirred for a further 18 h. Then it was hydrolysed with water (10 mL) and subsequently extracted three times with $CH_2Cl_2$. The organic phases were dried ($Na_2SO_4$), filtered and the solvent was removed under vacuum. The crude product (2.48 g) was purified using flash-chromatography (Ø = 6 cm, h = 12 cm, n-hexane:ethylacetate = 8:2, 30 mL, $R_f$ = 0.24).
Yellow Oil, that crystalized in the cold to a yellow solid, melting point: 31 °C,
Yield: 2.15 g (90%)

$C_{10}H_8N_2O$ (172.2)
MS (EI): m/z (rel.Int) = 172 [$M^+$, 100], 144 [M - CO, 69].
IR (neat): $\tilde{v}$ ($cm^{-1}$) = 3063 (C-H $_{aromat.}$), 2923 (C-H $_{aliphat.}$), 2854 (C-H), 1683 (C=O), 1596, 1517, 1499 (C=C), 763, 694 (C-H).
$^1$H-NMR ($CDCl_3$): $\delta$ (ppm) = 7.11 (d, J = 2.1 Hz, 1 H, Pyrazole-4-*CH*), 7.46 - 7.56 (m, 5H, Phenyl-*CH*), 7.76 (d, J = 2.0 Hz, 1H, Pyrazole-3-*CH*), 9.88 (s, 1 H, CHO).
$^{13}$C-NMR ($CDCl_3$): $\delta$ (ppm) = 112.5 (1 C, Pyrazole-4-*C*H), 125.8 (2 C, Phenyl-*C*H, ortho), 129.4 (1 C, Phenyl-*C*H, para), 129.6 (2 C, Phenyl-*C*H, meta), 139.0 (1 C, Phenyl-*C*, quartär), 140.3 (1 C, Pyrazole-5-C), 140.7 (1 C, Pyrazole-3-*C*H), 180.2 (1 C, CHO).

Example B: 4-Brom-1-phenylpyrazole-5-carbaldehyd-dimethylacetal

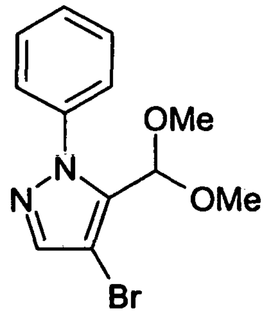

Experimental procedure:

The aldehyde (B) (2.30 g, 13.4 mmol), trimethylortoformiate (4.4 mL, 40.1 mmol) and p-toluolsulfonic acid monohydrate (127.1 mg, 0.67 mmol) were dissolved in abs. methanol (260 mL). After slow addition of pyridiniumbromide-perbromide (PBB) (4.3 g, 13.4 mmol) the solution was stirred for 90 h at room temperature. Subsequently a saturated $NaHCO_3$-solution (25 mL) was added, diluted with water and 3x extracted with $CH_2Cl_2$. The organic phases were dried ($K_2CO_3$) and the solvent removed under vacuum. The crude product (4.08 g) was purified using flash-chromatography (Ø = 8 cm, h = 12 cm, n-hexane:ethylacetate = 9:1, 40 mL, $R_f$ = 0.20).
Colourless solid, melting point: 33 °C, yield: 3.67 g (93%)
$C_{12}H_{13}BrN_2O_2$ (297.2)

| | C | H | N |
|---|---|---|---|
| Calc. | 48.5 | 4.41 | 9.43 |
| Found | 48.5 | 4.17 | 9.32 |

MS (EI): m/z (rel. Int.) = 298 [$^{82}$Br-$M^+$, 28], 296 [$^{79}$Br-$M^+$, 25], 267 [$^{82}$Br-M- $OCH_3$, 93], 265 [$^{79}$Br-M- $OCH_3$, 100], 235 [$^{82}$Br-M- 2x $OCH_3$, 49], 233 [$^{79}$Br-M- 2x $OCH_3$, 47].
IR (neat): $\tilde{v}$ ($cm^{-1}$) = 3062 (C $_{aromat}$), 2933 (C-H $_{aliphad}$), 2830 (C-H), 1597, 1501 (C=C), 1090, 1065 (C-O), 761, 692 (C-H).
$^1$H-NMR ($CDCl_3$): $\delta$ (ppm) = 3.37 (s, 6 H, ArCH(O*$CH_3$)$_2$*), 5.36 (s, 1 H, Ar*CH*($OCH_3$)$_2$), 7.39 - 7.49 (m, 3 H, Phenyl-C*H*), 7.53 - 7.59 (m, 2 H, Phenyl-CH, ortho), 7.63 (s, 1 H, Pyrazole-3C*H*).
$^{13}$C-NMR ($CDCl_3$): $\delta$ (ppm) = 54.7 (2 C, ArCH($OCH_3$)$_2$), 95.9 (1 C, Pyrazole-4-C), 98.5 (1 C, Ar*C*H($OCH_3$)$_2$), 125.6 (2 C, Phenyl-CH, ortho), 128.7 (1 C, Phenyl-*C*H, para), 129.1 (2 C, Phenyl-*C*H, meta), 136.3 (1 C, Phenyl-C, quartär), 140.1 (1 C, Pyrazole-5-C), 141.2 (1 C, Pyrazole-3-*C*H).

Example C: Ethyl-4-(5-dimethoxymethyl-1-phenylpyrazole-4-yl)-4-hydroxy-piperidin-1-carboxylat

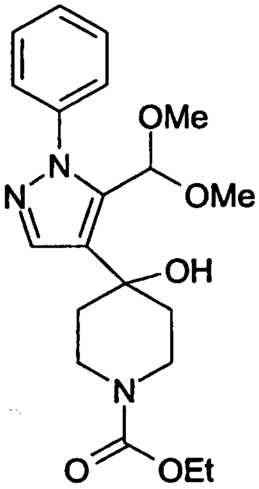

Experimental procedure:

Method 1:

To a solution of the bromated acetal (B) (1.50 g, 5.05 mmol) in abs. THF (100 mL) it was added under $N_2$-atmosphere isopropyl magnesiumchloride in THF (2 M, 2.8 mL, 5.60 mmol). The reaction mixture was stiired for 90 h at room temperature. Subsequently a solution of 4-Oxo-piperidine-1-carboxylic acid ethyl ester (1.12 g, 6.56 mmol) in abs. THF (65 mL) was added dropwise. After 24 h of stirring at room temperature water was added until no further precipitate was developing. The precipitate was filtered off and the mixture was extracted 3x with $CH_3Cl_2$. The organic phases were dried over $K_2CO_3$, filtered and reduced in vacuum. The crude product (2.35 g) was purified using flash-chromatography (Ø = 8 cm, h = 15 cm, Cyclohexan:Ethylacetat = 1:1, 40 mL, $R_f$ = 0,13).
Colourless Oil, Yield 623.4 mg (32%)

Method 2:

Under $N_2$-atmosphäre n-butyllithium in heptane (2.61 M, 130.0 μL, 0.34 mmol) was slowly added at -78 °C to a Solution of the bromated acetals B (100 mg, 0.34 mmol) in abs THF (7 mL). The reaction mixture was stirred for 15 minutes. Subsequently a solution of 4-Oxo-piperidine-1-carboxylic acid ethyl ester (58.21 mg, 0.34 mmol) in abs. THF (7 mL) was added dropwise at -78 °C. After 2 h of stiring at -78 °C the mixture was heated to room temperature and immediately mixed with water until no further precipitate is formed. Following extraction with $CH_2Cl_2$ the organic phases were dried over $K_2CO_3$ and filtered. The solvent was removed under vacuum and the crude product (0.14 g) was purified using flash-chromatography (Ø = 3 cm, h = 15 cm, n-Hexane:Ethylacetate = 3:7, 20 mL, $R_f$ = 0.23).
Colourless Oil, Yield 0.030 g (23%)
$C_{20}H_{27}N_3O_5$(389.5)
**MS** (EI): m/z (rel. Int.) = 358 [M - $OCH_3$, 44], 343 [M - $CH_3CH_2OH$, 55], 326 [M - 2x $OCH_3$, 100].
**IR** (neat): $\tilde{\nu}$($cm^{-1}$) = 3448 (O-H), 2980, 2933 (C-H $_{aliphat.}$), 1693 (C=O), 1598, 1545, 1500 (C=C), 1093, 1072 (C-O), 765, 696 (C-H).
**$^1$H-NMR** (DMSO-$d_6$): δ (ppm) = 1.17 (t, J = 7.1 Hz, 3H, $OCH_2CH_3$), 1.77 (br d, J = 12.5 Hz, 2H, $N(CH_2CH_{2\,eq.})_2$), 1.88 (td, J = 12.9/4.5 Hz, 2H, $N(CH_2CH_{2\,ax.})_2$), 3.10-3.21 (m, 2H, $N(CH_2CH_2)_2$)*, 3.20 (s, 6H, $ArCH(OCH_3)_2$), 3.74 - 3.87 (m, 2H, $N(CH_2CH_2)_2$), 4.02 (q, J = 7.1 Hz, 2H, $OCH_2CH_3$), 5.09 (s, 1H, $ArCH(OCH_3)_2$), 7.37 (tt, J = 7.2/1.8 Hz, 1H, Phenyl-C*H*, para), 7.40 - 7.45 (m, 2H, Phenyl-C*H*, meta), 7.48-7.52 (m, 2H, Phenyl-C*H*, ortho), 7.58 (s, 1H, Pyrazole-3-C*H*).
* The both protons of the piperidine rings neighbouring the nitrogen are overlaid by the signal of the methoxy group. They became visible producing an NMR-spectrum at 50°C.

**Example 1: Ethyl-6'-methoxy-1'-phenyl-4',6'-dihydro-1'H-spiro[piperidin-4,4'-furo[3,4-c]pyrazole]-1-carboxylate**

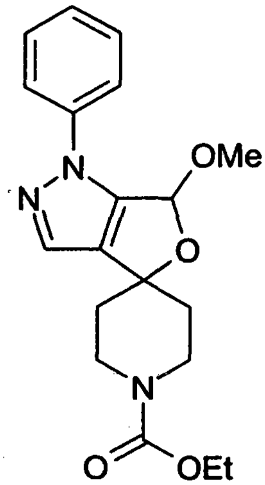

Experimental procedure:

Under $N_2$-atmosphere a solution of 0.1 M p-toluolsulphonsic acid monohydrate (900μL, 0.09 mmol) predried over $Na_2SO_4$ at room temperature was added to a solution example C (704.4 mg, 1.81 mmol) in abs. THF (36 mL). Subsequently it was stirred for 24 h at room temperature. Following the addition of 0.1 M NaOH- solution up to reaching pH 10 it was repeatedly extracted with $CH_2Cl_2$. Subsequently the organic phases were dried over $K_2CO_3$, filtered and the Solvent removed in vacuum. The crude product (0.700 g) was purified using flash-chromatography (Ø = 5 cm, h = 15 cm, n-Hexane:Ethylacetate = 1:1, 30 mL, $R_f$ = 0.43).
Colourless Oil, Yield 233 mg (36%)
$C_{19}H_{23}N_3O_4$ (357.5)
**MS** (EI): m/z (rel. Int) = 357 [M*, 61], 342 [M - $CH_3$, 70], 325 [M - $HOCH_3$, 100], 297 [M - $OCH_3$-$CH_3CH_2$, 41].
**IR** (neat): $\tilde{\nu}$(cm$^{-1}$) = 2954, 2930 (C-H $_{aliphat.}$), 2829 (C-H), 1692 (C=O), 1600, 1566, 1514 (C=C), 1098, 1083 (C-O), 756, 711 (C-H).
$^1$H-NMR (DMSO-$d_6$): δ (ppm) = 1.18 (t, J = 7.1 Hz, 3H, $OCH_2CH_3$), 1.66-1.90 (m, 4H, $N(CH_2CH_2)_2$), 3.34 (s, 3H, $OCH_3$), 3.43 - 3.65 (m, 4H, $N(CH_2CH_2)_2$), 4.05 (q, J = 7.1 Hz, 2H, $OCH_2CH_3$), 6.38 (s, 1H, Ar-C*H*), 7.31 (t, J = 7.1 Hz, 1H, Phenyl-C*H*, para), 7.46 - 7.53 (m, 2H, Phenyl-CH, meta), 7.66 -7.71 (m, 2H, Phenyl-CH, ortho), 7.79 (s, 1H, Pyrazole-3-C*H*).

**Example** 2: 6'-Methoxy-1'-phenyl-4',6'-dihydro-1'H-spiro[piperidin-4,4'-furo[3,4-c]pyrazole]

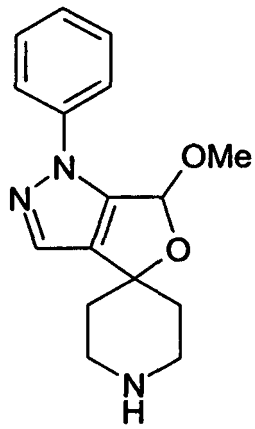

Experimental procedure:

To a solution of example 1 (343 mg, 0.96 mmol) in dioxane/$H_2O$ 1:1 (96 mL) an aqueous KOH-solution (2 M, 30 mL) was added. The solution was heated for 18 h at 100 °C. After diluting with water, it was extracted with ethylacetate,

dried over $K_2CO_3$, filtered and subsequently the solvent was removed in vacuum. The crude product (298 mg) was purified using flash-chromatography (0 = 4 cm, h = 15 cm, Methanol + 2% $NH_{3\ conc.}$, 30 mL, $R_f$ = 0.21) and subsequently nach after removing te solvent up to dryness and another dissolution in ethylacetate was filtered through a membrane filter. Further reduction resulted in a slightly yellow solid.
Slightly yellow solid, melting point: 81 °C, Yield 210 mg (77%)
$C_{16}H_{19}N_3O_2$ (285.4)

| | C | H | N |
|---|---|---|---|
| Calc. | 67.4 | 6.71 | 14.7 |
| Found | 67.1 | 6.73 | 14.5 |

**MS** (EI): m/z (rel. Int) = 285 [M*, 60], 270 [M - $CH_3$, 100], 77 [Phenyl, 10].
**IR** (neat): $\tilde{\nu}$($cm^{-1}$) = 3309 (N-H), 3063 (C-H $_{aromat.}$), 2942 (C-H $_{aliphat.}$), 2826 (C-H), 1599, 1563, 1512 (C=C), 1080, 1061 (C-O), 755, 710 (C-H).
**$^1$H-NMR** (DMSO-$d_6$): δ (ppm) = 1.65 - 1.77 (m, 4H, $N(CH_2CH_2)_2$), 2.67 - 2.78 (m, 2H, $N(CH_2CH_2)_2$), 2.87 - 2.97 (m, 2H, $N(CH_2CH_2)_2$), 3.34 (s, 3H, $OCH_3$), 6.35 (s, 1 H, ArC*H*), 7.30 (tt, J = 7.4/1.2 Hz, 1H, Phenyl-CH, para), 7.45 - 7.52 (m, 2H, Phenyl-CH, meta), 7.66 - 7.71 (m, 2H, Phenyl-CH, ortho), 7.73 (s, 1H, Pyrazole-3-C*H*).

The Signal of the NH-protons was not detectable.

**Example** 3: 1-Benzyl-6'-methoxy-1'-phenyl-4',6'-dihydro-1'H-spiro[piperidin-4,4'-furo[3,4-c]pyrazole]

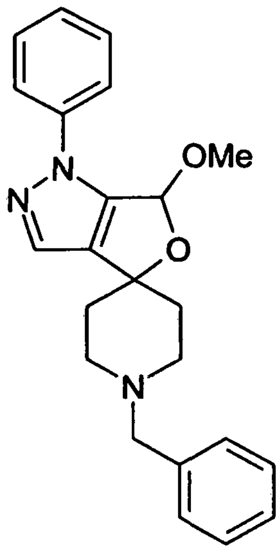

Experimental procedure:

Benzyl bromide (29.9 µL, 0.25 mmol) and $K_2CO_3$ (232.2 mg, 1.68 mmol) were added to a solution of example 2 (60.0 mg, 0.21 mmol) in abs. THF (7 mL). This mixture was heated under reflux for 24 h. Then it was filtered and the Solvent removed under vacuum. The crude product (99.7 mg) was purified using flash-chromatography (Ø = 3 cm, h = 15 cm, n-Hexane:Ethylacetate = 1:1, 20 mL, $R_f$ = 0.20).
Yellow resin, Yield 56 mg (71%)
$C_{23}H_{25}N_3O_2$ (375.5)

| | C | H | N |
|---|---|---|---|
| Calc. | 73.6 | 6.71 | 11.2 |
| Found | 73.6 | 6.82 | 11.1 |

MS (EI): m/z (rel. Int) = 375 [M*, 60], 360 [M - $CH_3$, 100], 344 [M - $OCH_3$, 17], 284 [M - Benzyl, 17], 91 [Benzyl, 93].
**IR** (neat): $\tilde{\nu}$ ($cm^{-1}$) = 3061 (C-H $_{aromat.}$), 2923 (C-H $_{aliphat.}$), 2804 (C-H), 1600, 1565, 1512 (C=C), 1080, 1061 (C-O), 755, 738 (C-H).
**$^1$H-NMR** ($CDCl_3$): δ (ppm) =1.86-2.12 (m, 4H, $N(CH_2CH_2)_2$), 2.49 - 2.80 (m, 4H, $N(CH_2CH_2)_2$), 3.46 (s, 3H, $OCH_3$), 3.62 (s, 2H, $NCH_2Ph$), 6.16 (s, 1 H, ArC*H*), 7.24 - 7.46 (m, 9H, aromat. C*H*), 7,73 (dd, J = 8.4/0.8 Hz, 2H, Phenyl-CH, ortho).

**Example** 4: 1-Butyl-6'-methoxy-1'-phenyl-4',6'-dihydro-1'H-spiro[piperidin-4,4'-**furo[3,4-c]pyrazole]**

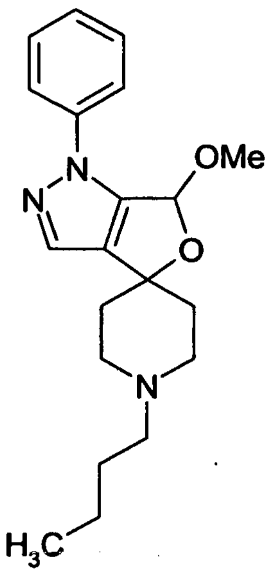

Experimental procedure:

Butyl-bromide (48.4 μL, 0.45 mmol) and $K_2CO_3$ (410.5 mg, 2.97 mmol) were added to a solution example 3 (106.0 mg, 0.37 mmol) in abs. THF (12 mL). This mixture was heated under reflux for 49 h. Then it was filtered and the solvent removed under vacuum. The crude product (133.3 mg) was purified using flash-chromatography (Ø = 3 cm, h = 18 cm, Ethylacetat + 10% Methanol, 20 mL, $R_f$ = 0.18).
Yellow Oil, Yield 74 mg (59%)
$C_{20}H_{27}N_3O_2$(341.5)

| | C | H | N |
|---|---|---|---|
| Calc. | 70.4 | 7.97 | 12.3 |
| Found | 70.3 | 8.01 | 12.2 |

**MS** (EI): m/z (rel. Int) = 341 [$M^+$, 5], 298 [M - Propyl, 100].
**IR** (neat): $\tilde{\nu}$ ($cm^{-1}$) = 3064 (C-H $_{aromat.}$), 2927 (C-H $_{aliphat}$.), 2804 (C-H), 1601, 1564, 1513 (C=C), 1086, 1062 (C-O), 754, 712 (C-H).
**$^1$H-NMR** ($CDCl_3$): δ (ppm) = 0.94 (t, J = 7.3 Hz, 3H, $NCH_2CH_2CH_2CH_3$), 1.30 - 1.41 (m, 2H, $NCH_2CH_2CH_2CH_3$), 1.48 - 1.60 (m, 2H, $NCH_2CH_2CH_2CH_3$), 1.90 - 2.12 (m, 4H, $N(CH_2CH_2)_2$), 2.38 - 2.50 (m, 2H, $NCH_2CH_2CH_2CH_3$), 2.51 - 2.81 (m, 4H, $N(CH_2CH_2)_2$), 3.46 (s, 3H, $OCH_3$)), 6.16 (s, 1H, ArC*H*), 7.25 (t, J = 7.4 Hz, 1H, Phenyl-CH, para), 7.41 - 7.44 (m, 2H, Phenyl-C*H*, meta), 7.46 (s, 1H, Pyrazole-3-C*H*), 7.74 (d, J = 7.7 Hz, 2H, Phenyl-CH, ortho).

**Example** D: 5-(2-Methoxyvinyl)-1-phenylpyrazole

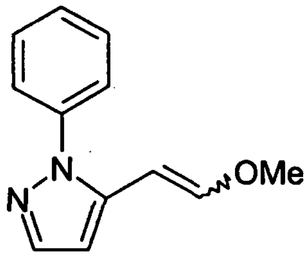

Experimental procedure:

Under $N_2$-atmosphere dried $MeOCH_2P^+Ph_3Cl^-$ (6.22 g, 18.1 mmol) was weighed out and suspended in abs. THF (60 mL) for 30 minutes. Subsequently the suspension was cooled to -50 °C and following that $KO^tBu$-Solution in THF (1M, 16.5 mL, 16.5 mmol) was slowly added dropwise. After stirring for another 15 minutes, the aldehyd of example A (1.42 g, 8.3 mmol) was dissolved in abs. THF (40 mL) and added dropwise at -50 °C. The reaction mixture was slowly heated to room temperature over night while stirring. After adittion of water (- 25 mL) it was extracted 3-times using ethylacetatet. The organic phases were dried over $K_2CO_3$, filtered and the solvent removed under vacuum. The crude product (7.59 g) was purified using flash-chromatography (Ø = 8 cm, h = 12 cm, n-hexane:ethylacetate = 9:1, 40 mL, $R_f$ = 0.10).
Colourless Oil, Yield 1.53 g (92%)
$C_{12}H_{12}N_2O$ (200.3)
MS (EI): m/z (rel. Int) = 200 [$M^+$, 93], 169 [M - $OCH_3$, 100].
IR (neat): $\tilde{\nu}$(cm$^{-1}$) = 3098 (C-H $_{aromat.}$), 2935 (C-H $_{aliphat.}$), 1644 (C=C $_{Alken}$), 1597, 1529 (C=C $_{aromat.}$), 1093 (C-O), 761, 696 (C-H).
$^1$H-NMR (DMSO-$D_6$): δ (ppm) = 3.56 (s, 3H, OC*H*$_3$, trans-Isomer), 3.77 (s, 3H, OC*H*$_3$, cis-Isomer), 5.18 (d, J = 6.8 Hz, 1 H, C*H*=CHOCH$_3$, cis-Isomer), 5.59 (d, J = 12.8 Hz, 1 H, C*H*=CHOCH$_3$, trans-Isomer), 6.37 (d, J = 6.8 Hz, 1H, CH=C*H*OCH$_3$, cis-Isomer), 6.47 (d, J = 1,6 Hz, 1 H, Pyrazole-4-CH, trans-Isomer), 6,63 (d, J = 1.6 Hz, 1H, Pyrazole-4-CH, cis-Isomer), 7.26 (d, J = 12.8 Hz, 1 H, CH=C*H*OCH$_3$, trans-Isomer), 7.39 - 7.54 (m, 10H, aromat. H, cis-Isomer (5H), trans-Isomer (5H)), 7.55 (d, J = 1.6 Hz, 1H, Pyrazole-3-CH, trans-Isomer), 7.58 (d, J = 1.2 Hz, 1H, Pyrazole-3-CH, cis-Isomer).

The ratio of the isomers cis/trans is 1:3.

**Example E: 2-(1-Phenylpyrazole-5-yl)acetaldehyddimethylacetal (11)**

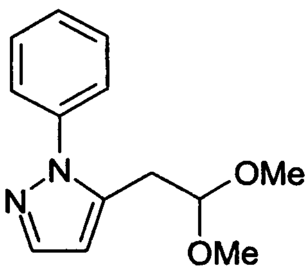

Experimental procedure:

Example D (1.0 g, 5.0 mmol) was dissolved in abs. methanol (80 mL). Subsequently p-Toluol sulphonic acid monohydrate (475 mg, 2.5 mmol) was added and heated for 72 h under reflux. After addition of a saturated solution of $NaHCO_3$ up to approximately pH 10, it was extracted 3-times with $CH_2Cl_2$. The pooled organic phases were dried over $K_2CO_3$, and filtered. Following removal of the solvent under vacuum the Crude product (1.15 g) was pruried using flash chromatography. (0 = 6 cm, h = 15, n-hexane:ethylacetate = 8:2, 40 mL, $R_f$ = 0.12).
Colourless Oil, Yield 1.04 g (90%)
$C_{13}H_{16}N_2O_2$ (232.3)
**MS** (EI): m/z (rel. Int) = 233 [$MH^+$, 10], 201 [M - $OCH_3$, 9], 171 [MH - 2x $OCH_3$, 58], 169 [M - $OCH_3$ - $HOCH_3$, 100].
**IR** (neat): $\tilde{\nu}$(cm$^{-1}$) = 3064 (C-H $_{aromat.}$), 2935 (C-H $_{aliphat.}$), 1599, 1535, 1500 (C=C), 1120, 1063 (C-O), 766, 696 (C-H).

**$^1$H-NMR** (DMSO-$D_6$): δ (ppm) = 2.92 (d, J = 5.9 Hz, 2H, $CH_2CH(OCH_3)_2$), 3.39 (s, 6H, $CH_2CH(OCH_3)_2$), 4.55 (t, J = 5.5 Hz, 1 H, $CH_2CH(OCH_3)_2$), 6.39 (d, J = 1.6 Hz, 1H, Pyrazole-4-C*H*), 7.40 - 7.55 (m, 5H, Phenyl-C*H*), 7.59 (d, J = 1.6 Hz, 1H, Pyrazole-3-C*H*).
**$^{13}$C-NMR** (DMSO-$D_6$): δ (ppm) = 30.5 (1C, $CH_2CH(OCH_3)_2$), 53.8 (2C, $CH_2CH(OCH_3)_2$), 103.6 (1C,$CH_2CH(OCH_3)_2$), 107.3 (1C, Pyrazole-4-CH), 126.0 (2C, Phenyl-CH, ortho), 128.6 (1C, Phenyl-CH, para), 129.9 (2C, Phenyl-CH, meta), 139.1 (1C, Phenyl-C, quartär), 140.2 (1C, Pyrazole-5-CH), 140.3 (1C, Pyrazole-3-CH).

<u>**Example** F: 2-(4-Brom-1-phenylpyrazole-5-yl)acetaldehyddimethylacetal</u>

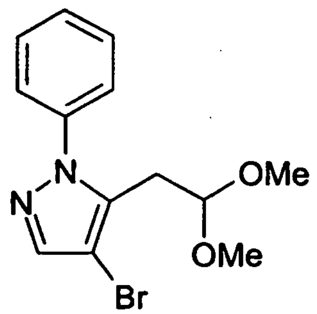

<u>Experimental procedure:</u>

To a solution of example E (9.9 g, 42.8 mmol) in abs. methanol (500 mL) were added trimethyl-orthoformiate (7.0 mL, 64.3 mmol) and - cooling in ice at 0 °C - in batches Pyridinium bromide perbromide (PBB) (13.7 g, 42.8 mmol). The reaction mixture was stirred for 1 h at 0 °C, subsequently heated slowly to room temperature and then stirred for a further 4 h. After addition of a saturated solution of $NaHCO_3$ up to reaching approximately pH 10 and diluting with water, it was extracted 3 times with $CH_2Cl_2$. The organic phases were dried ($K_2CO_3$), filtered and the solvent was removed under vacuum. The crude product (13.3 g) was purified using flash-chromatography (Ø = 8 cm, h = 18 cm, n-hexane: ethylacetate = 8:2, 40 mL, $R_f$ = 0.30). Colourless Oil, Yield 12.7 g (95%)
$C_{13}H_{15}BrN_2O_2$(311.2)

| | C | H | N |
|---|---|---|---|
| Calc. | 50.2 | 4.86 | 9.00 |
| Found | 50.2 | 4.93 | 9.02 |

**MS** (EI): m/z (rel. Int.) = 313 [$^{81}$Br-MH$^+$, 7], 311 [$^{79}$Br-MH$^+$, 8], 281 [$^{81}$Br-M - $OCH_3$, 9], 279 [$^{79}$Br-M -$OCH_3$, 12], 249 [$^{81}$Br-MH - 2x - $HOCH_3$, 40], 247 [$^{79}$Br-M - 2x-$HOCH_3$, 36], 168 [M - Br - $OCH_3$ - $HOCH_3$, 100].
**IR** (neat): $\tilde{\nu}$(cm$^{-1}$) = 3065 (C-H $_{aromat.}$), 2933 (C-H $_{aliphat.}$), 1598, 1500 (C=C), 1119, 1072 (C-O), 766, 695 (C-H).
**$^1$H-NMR** (DMSO-$D_6$): δ (ppm) = 2.95 (d, J = 5.9 Hz, 2H, $CH_2CH(OCH_3)_2$), 3.10 (s, 6H, $CH_2CH(OCH_3)_2$), 4.40 (t, J = 5.7 Hz, 1 H, $CH_2CH(OCH_3)_2$), 7.45 - 7.58 (m, 5H, Phenyl-C*H*), 7.76 (s, 1H, Pyrazole-3-C*H*).
**$^{13}$C-NMR** (DMSO-$D_6$): δ (ppm) = 29.6 (1C, $CH_2CH(OCH_3)_2$), 54.2 (2C, $CH_2CH(OCH_3)_2$), 95.8 (1C, Pyrazole-4C), 102.7 (1C, $CH_2CH(OCH_3)_2$), 126.3 (2C, Phenyl-*C*H, ortho), 129.3 (1C, Phenyl-*C*H, para), 130.0 (2C, Phenyl-*C*H, meta), 137.4 (1C, Phenyl-*C*, quartär), 140.2 (1C, Pyrazole-5*C*), 140.5 (1C, Pyrazole-3-*C*H).

<u>**Example G: 2-[4-(1-Benzyl-4-hydroxypiperidin-4-yl)-1-phenylpyrazole-5-yl]acetaldehyddimethylacetal**</u>

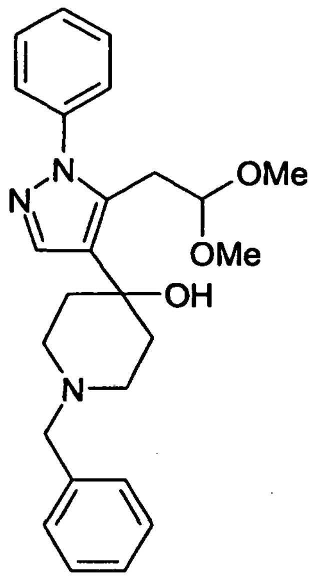

Experimental procedure:

Under $N_2$-atmosphere n-butyl lithium in n-hexane (1.53 M, 10.5 mL, 16.1 mmol) was added slowly at -78 °C to a solution of the bromated Example F (5.0 g, 16.1 mmol) in abs. THF (80 mL). The reaction mixture was stirred for 15 minutes. Subsequently a solution of 1-Benzyl-piperidin-4-one (3.3 g, 17.7 mmol) in abs. THF (10 mL) was added at -78 °C. After stirring for 4.5 h at -78 °C the micture was heated to room temperature, stirred for a further hour and subsequently mixed with water until no further precipitate is forming (40 mL). Following extraction with $CH_2Cl_2$ the organic phases were dried over $K_2CO_3$ and filtered. The solvent was removed under vacuum and the crude product (8.3 g) purified using flash-chromatography (Ø = 8 cm, h = 18 cm, ethylacetate:n-hexane = 8:2 + 2% N,N-Dimethylethylamine, 80 mL, $R_f$ = 0.23).
Yellow Oil, crystallizing into a slightly yellow solid, melting point: 107°C, Yield 4.6 g (67%).
$C_{25}H_{31}N_3O_3$ (421.6)
**MS** (ESI): m/z (rel. Int.) = 422 [$MH^+$, 100].
**IR** (neat): $\tilde{\nu}$ ($cm^{-1}$) = 3445 (O-H), 3062, 3026 (C-H $_{aromat.}$), 2938 (C-H $_{aliphat.}$), 1599, 1501 (C=C), 1117, 1071 (C-O), 740, 697 (C-H).
**$^1$H-NMR** (DMSO-$d_6$): δ (ppm) = 1.77 (d breit, J = 12.5 Hz, 2H, $N(CH_2CH_2)_2$), 1.92 (td, J = 12.5/3.1 Hz, 2H, $N(CH_2CH_2)_2$), 2.42 (t, J = 10.6 Hz, 2H, $N(CH_2CH_2)_2$), 2.58 (d breit, J = 11.0 Hz, 2H, $N(CH_2CH_2)_2$), 3.04 (s, 6H, $CH_2CH(OCH_3)_2$), 3.12 (d, J = 5.5 Hz, 2H, $CH_2CH(OCH_3)_2$), 3.49 (s, 2H, $NCH_2Ph$), 4.50 (t, J = 5.8 Hz, 1H, $CH_2CH(OCH_3)_2$), 4.66 (s, 1H, OH), 7.21 - 7.27 (m, 1H, aromat. CH, para), 7.32 (m, 4H, aromat. CH, meta), 7.40 - 7.46 (m, 3H, aromat. CH, para/ortho), 7.49 (d, J = 7.0 Hz, 2H, aromat CH, ortho), 7.50 (s, 1H, Pyrazole-3-CH).

**Example 5: 1-Benzyl-6'-methoxy-1'-phenyl-6',7'-dihydro-1'H-spiro[piperidin-4,4'-pyrano[4,3-c]pyrazole]**

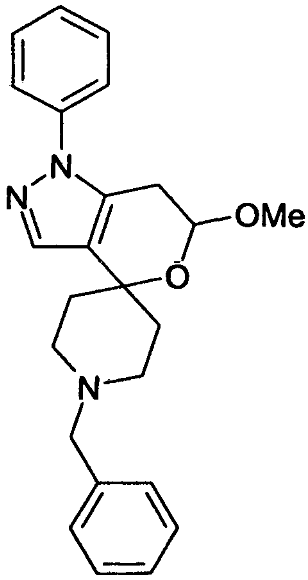

Experimental procedure:

Method 1:

To a solution of Example G (4.5 g, 10.7 mmol) in abs. Methanol (150 mL) p-Toluol sulphonic acid monohydrate (4.5 g, 23.5 mmol) was added and stirred at room temperature. After stirring for 21 h a solution of NaOH (0.5 M) was added upto reaching aproximately pH 10, dilluted with water and extracted 3 times with $CH_2Cl_2$. The pooled organic phases were dried over $K_2CO_3$, filtered and the solvent removed und vacuum. The crude product (4.9 g) was purified using flash-chromatography (0 = 8 cm, h = 18 cm, ethylacetate, 80 mL, $R_f$ = 0.27).
Coulorless solid,melting point: 151°C, Yield 3.0 g (73%).
$C_{24}H_{27}N_3O_2$ (389.5)

| | C | H | N |
|---|---|---|---|
| Calc. | 74.0 | 6.99 | 10.8 |
| Found | 73.7 | 6.91 | 10.5 |

**MS** (ESI): m/z (rel. Int.) = 390 [$MH^+$, 100].
**IR** (neat): $\tilde{\nu}$ ($cm^{-1}$) = 3028 (C-H $_{aromat.}$), 2919 (C-H $_{aliphat.}$), 1598, 1504 (C=C), 1114, 1059 (C-O), 739, 696 (C-H).
**$^1$H-NMR** ($CDCl_3$): δ (ppm) = 1.92 (dd, J = 14.1/3.1 Hz, 2H, $N(CH_2CH_2)_2$), 1.98 - 2.05 (m, 1H, $N(CH_2CH_{2\,axial})_2$), 2.09 (td, J = 12.5/3.7 Hz, 1H, $N(CH_2CH_{2\,axial})_2$), 2.45 (t breit, J = 11.7 Hz, 1H, $N(CH_2CH_2)_2$), 2.55 (t breit, J = 11.7 Hz, 1H, $N(CH_2CH_2)_2$), 2.77 (m, 2H, $N(CH_2CH_2)_2$), 2.88 (dd, J = 15.6/7.0 Hz, 1H, $CH_2CHOCH_3$), 2.96 (dd, J = 15.7/3.9 Hz, 1 H, $CH_2CHOCH_3$), 3.53 (s, 3H, $OCH_3$), 3.58 (s, 2H, $NCH_2Ph$), 4.84 (dd, J = 7.0/3.9 Hz, 1H, $CH_2CHOCH_3$), 7.23 - 7.28 (m, 1H, Phenyl-CH), 7.29 - 7.39 (m, 5H, Phenyl-CH), 7.40 - 7.47 (m, 4H, Phenyl-CH), 7.49 (s, 1 H, Pyrazole-3-CH).
**$^{13}$C-NMR** ($CDCl_3$): δ (ppm) = 31.2 (1C, $CH_2CHOCH_3$), 36.7 (1C, $N(CH_2CH_2)_2$), 39.5 (1C, $N(CH_2CH_2)_2$), 49.5 (1C, $N(CH_2CH_2)_2$), 49.6 (1C, $N(CH_2CH_2)_2$), 56.9 (1C, $OCH_3$), 63.7 (1C, $NCH_2Ph$), 71.9 (1C, Spiro-C), 77.5 (1C, Pyrazole-4-C), 96.8 (1C, $CH_2CHOCH_3$), 122.8 (2C, Phenyl-CH), 124.4 (1C, Phenyl-C, quartär), 127.3, 128.5, 129.5 (8C, Phenyl-CH), 133.8 (1C, Phenyl-C, quartär), 135.9 (1C, Pyrazole-3-CH), 139.5 (1C, Pyrazole-5-C).

**Example 6: 6'-Methoxy-1'-phenyl-6',7'-dihydro-1'H-spiro[piperidin-4,4'-pyrano[4,3-c]pyrazole]**

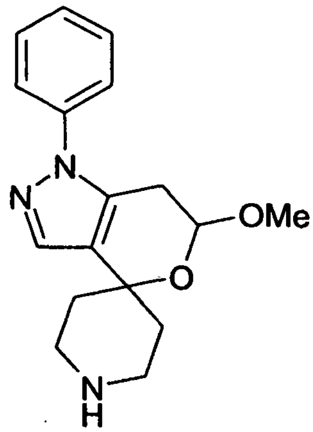

Experimental procedure:

Method 1:

To a solution of example 5 (80 mg, 0.21 mmol) in abs. Methanol (5 mL) were added dried ammonium formiate (64.8 mg, 1.28 mmol) and 10% Pd/C (16 mg) in one portion. Subsequently it was stirred for 10 minutes under reflux and subsequently the catalysator was filtered through a fluted filter. Following thorough rinsing with methanol wurde the solvent was removed under vacuum. The crude product (63 mg) was purified using flash-chromatography (Ø = 2 cm, h = 15 cm, Methanol + 2% $NH_3$ (conc.), 10 mL, $R_f$ = 0.10). The fractions were subsequently dried under vacuum to dryness. Then the residue was dissolved again in $CH_2Cl_2$ and filtered through cotton wool. A further evaporation resulted in a yellow oil.
Yellow Oil, Yield 54 mg (88%)
$C_{17}H_{21}N_3O_2$ (299.4)

| | C | H | N |
|---|---|---|---|
| Calc. | 68.2 | 7.07 | 14.0 |
| Found | 67.2 | 7.15 | 13.9 |

**MS** (ESI): m/z (rel. Int.) = 300 [$MH^+$, 100], 598 [2 x M, 46].
**IR** (neat): $\tilde{\nu}$ ($cm^{-1}$) = 3302 (N-H), 3061 (C-H $_{aromat.}$), 2921 (C-H $_{aliphat.}$), 2835 (C-H), 1598, 1576, 1503 (C=C), 1136 (C-O), 756, 694 (C-H).
**$^1$H-NMR** (DMSO-$d_6$): δ (ppm) = 1.55 - 1.68 (m, 2H, $N(CH_2CH_2)_2$), 1.79 -1.92 (m, 2H, $N(CH_2CH_2)_2$), 2.75 (t breit, J = 11.0 Hz, 2H, $N(CH_2CH_2)_2$), 2.80 - 2.95 (m, 2H, $N(CH_2CH_2)_2$)*, 2.82 (dd, J = 15.7/7.0 Hz, 1H, $CH_2CHOCH_3$), 2.91 (dd, J = 15.7/3.9 Hz, 1H, $CH_2CHOCH_3$), 3.31 (s breit, 1H, NH), 3.39 (s, 3H, $OCH_3$), 4.88 (dd, J = 6.7/3.5 Hz, 1H, $CH_2CHOCH_3$), 7.35 (t, J = 7.4 Hz, 1H, Phenyl-CH, para), 7.44 - 7.55 (m, 4H, Phenyl-CH, meta, ortho), 7.59 (s, 1 H, Pyrazole-3-CH).
* Signal is overlaid by the dd of the two methylene groups.

**Example 7: 6'-Methoxy-1'-phenyl-1-propyl-6',7'-dihydro-1'H-spiro[piperidin-4,4'-pyrano[4,3-c]pyrazole]**

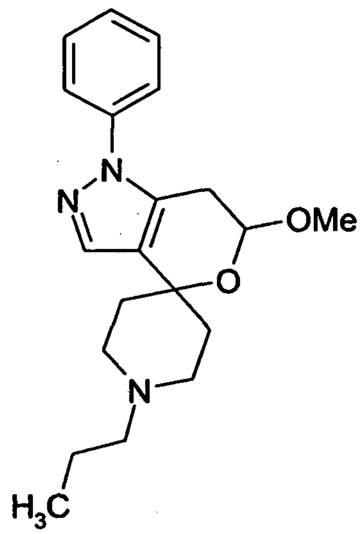

Experimental procedure:

To a solution of the crude Example 6 (100 mg, 0.33 mmol) in acetonitrile (7 mL) were added propyl bromide (36.4 µL, 0.40 mmol) and $K_2CO_3$ (369.3 mg, 2.67 mmol). The mixture was heated under reflux for 40 h. Subsequently the $K_2CO_3$ was filtered off and the solvent was removed under vacuum. The residue was dissolved in $CH_2Cl_2$, diluted with water and extracted with a saturated solution of $NaHCO_3$. The pooled organic phases were dried over $K_2CO_3$, filtered and the solvent removed under vacuum. The crude product (118 mg) was purified using flash-chromatography (0 = 3 cm, h = 18 cm, ethylacetate + 2% N,N-Dimethylethylamine, 20 mL, $R_f$ = 0.22).

Colourless resin, Yield 80.0 mg (70%)

$C_{20}H_{27}N_3O_2$ (341.5)

| | C | H | N |
|---|---|---|---|
| Calc. | 70.4 | 7.97 | 12.3 |
| Found | 69.9 | 8.01 | 12.1 |

**MS** (ESI): m/z (rel. Int.) = 342 [$MH^+$, 100].

**IR** (neat): $\tilde{\nu}$ ($cm^{-1}$) = 2954, 2872 (C-H $_{aliphat.}$), 2808 (C-H), 1599, 1505 (C=C), 1139 (C-O), 758, 694 (C-H).

**$^1$H-NMR** ($CDCl_3$): δ (ppm) = 0.94 (t, J = 7.4 Hz, 3H, $NCH_2CH_2CH_3$), 1.58 (m, 2H, $NCH_2CH_2CH_3$), 1.95 (dd, J = 13.9/2.5 Hz, 2H, $N(CH_2CH_2)_2$), 2.00 - 2.08 (m, 1H, $N(CH_2CH_2)_2$), 2.14 (td, J = 12.9/3.6 Hz, 1H, $N(CH_2CH_2)_2$), 2.40 (t, J = 7.8 Hz, 2H, $NCH_2CH_2CH_3$), 2.42 (t breit, J = 11.7 Hz, 1H, $N(CH_2CH_2)_2$)*, 2.52 (t breit, J = 11.7 Hz, 1H, $N(CH_2CH_2)_2$), 2.79 - 2.85 (m, 2H, $N(CH_2CH_2)_2$), 2.90 (dd, J = 15.7/6.3 Hz, 1H, $CH_2CHOCH_3$), 2.98 (dd, J = 15.7/3.9 Hz, 1 H, $CH_2CHOCH_3$), 3.56 (s, 3H, $OCH_3$), 4.85 (dd, J = 6.7/3.5 Hz, 1H, $CH_2CHOCH_3$), 7.29 - 7.34 (m, 1 H, Phenyl-CH, para), 7.41-7.48 (m, 4H, Phenyl-CH), 7.50 (s, 1H, Pyrazole-3-CH).

*The Signal is overlaid by the triplett of the methylene group of the n-propyl radical.

**$^{13}$C-NMR** ($CDCl_3$): δ (ppm) = 12.3 (1C, $NCH_2CH_2CH_3$), 20.5 (1C, $NCH_2CH_2CH_3$), 31.2 (1C, $CH_2CHOCH_3$), 36.6 (1C, $N(CH_2CH_2)_2$), 39.4 (1C, $N(CH_2CH_2)2$), 49.5 (1C, $N(CH_2CH_2)_2$), 49.7 (1C, $N(CH_2CH_2)_2$), 56.9 (1C, $OCH_3$), 61.2 (1C, $NCH_2CH_2CH_3$), 71.9 (1C, Spiro-C), 77.4 (1C, Pyrazole-4-C), 96.9 (1C, $CH_2CHOCH_3$), 122.8 (2C, Phenyl-CH, ortho), 127.3 (1C, Phenyl-CH, para), 129.5 (2C, Phenyl-CH, meta), 133.8 (1C, Phenyl-C, quartär), 135.9 (1C, Pyrazole-3-CH), 139.5 (1C, Pyrazole-5-C).

**Example 8: 1-Isopropyl-6'-methoxy-1'-phenyl-6',7'-dihydro-1'H-spiro[piperidin-4,4'-pyrano[4,3-c]pyrazole]**

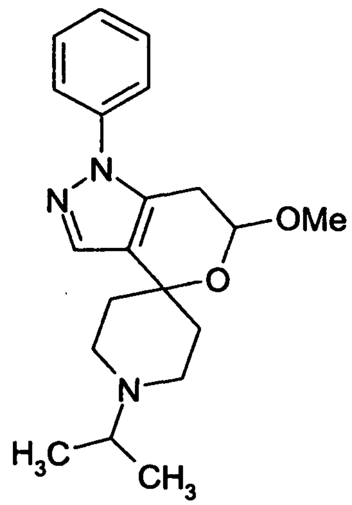

Experimental procedure:

Method 1:

To a solution of the crude example **6** (150 mg, 0.50 mmol) in acetonitrile (15 mL) $K_2CO_3$ (553 mg, 4.0 mmol), tetrabutyl ammoniumiodide (37.0 mg, 0.10 mmol) and isopropylbromide (56.4 μL, 0.60 mmol) were added. The mixture was heated for 48 h unter reflux. Subsequently the $K_2CO_3$ was filtered off and the solvent removed under vacuum. The residue was dissolved again in ethylacetate, diluted with water and several times extracted with ethylacetate. The pooled organic phases were dried over $K_2CO_3$, filtered and the solvent finally removed under vacuum. The crude product (218 mg) was purified using flash-chromatography (Ø = 4 cm, h = 18 cm, ethylacetate:methanol 3:7 + 2% N,N-Dimethylethylamine, 30 mL, $R_f$ = 0.15).
Colourless resin, Yield 53 mg (31%).

Method 2:

To a solution of crude example 6 (150 mg, 0.50 mmol) in abs. dichloroethane (5 mL) acetone (44.2 μL, 0.60 mmol), sodium triacetoxy-borhydride (148.7 mg, 0.70 mmol) and glacial acetic acid (28.7 μL, 0.50 mmol) were added. The reaction mixture was stirred for 61 h at room temperature. After alkalizing with a solution of NaOH (0.5 N, 2 mL) and diluting with water it was extracted 3 times with $CH_2Cl_2$. The pooled organic phases were subsequently dried over $K_2CO_3$, filtered and the solvent removed under vacuum. The crude product (151 mg) was purified using flash-chromatography (0 = 3 cm, h = 18 cm, ethylacetate:methanol 1:1, 20 mL, $R_f$ = 0.48).
Colourless resin, Yield 51 mg (30%).
$C_{20}H_{27}N_3O_2$ (341.5)

| | C | H | N |
|---|---|---|---|
| Calc. | 70.4 | 7.97 | 12.3 |
| Found | 69.8 | 8.00 | 11.9 |

MS (ESI): m/z (rel. Int.) = 342 [$MH^+$, 100].
IR (neat): $\tilde{v}$ ($cm^{-1}$) = 2960, 2920 (C-H $_{aliphat.}$), 2833 (C-H), 1599, 1505 (C=C), 1139, 1106 (C-O), 759, 695 (C-H).
**$^1$H-NMR** ($CDCl_3$): δ (ppm) = 1.13 (d, J = 6.3 Hz, 6H, $CH(CH_3)_2$), 1.88 - 2.15 (m, 4H, $N(CH_2CH_2)_2$), 2.60 - 2.86 (m, 5H, $N(CH_2CH_2)_2$ (4H), $NCH(CH_3)_2$ (1 H)), 2.90 (dd, J = 15.7/7.0 Hz, 1H, $CH_2CHOCH_3$), 2.98 (dd, J = 15.7/3.9 Hz, 1H, $CH_2CHOCH_3$), 3.56 (s, 3H, $OCH_3$), 4.86 (dd, J = 7.0/3.9 Hz, 1H, $CH_2CHOCH_3$), 7.30 - 7.36 (m, 1H, Phenyl-CH, para), 7.42 - 7.49 (m, 4H, Phenyl-CH), 7.51 (s, 1H, Pyrazole-3-CH).

**Example 9: 1-Butyl-6'-methoxy-1'-phenyl-6',7'-dihydro-1'H-spiro(piperidin-4,4'-pyrano[4.3-c]pyrazole]**

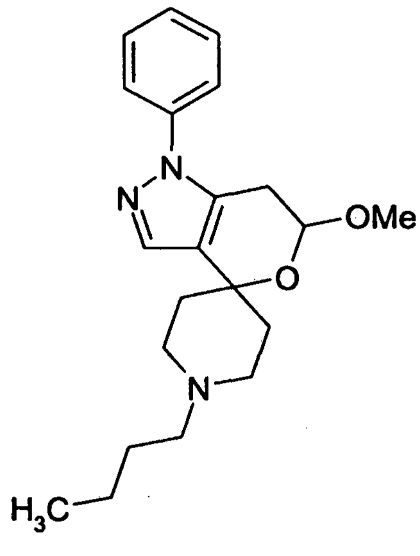

Experimental procedure:

To a solution of crude example 6 (95 mg, 0.32 mmol) in acetonitrile (11 mL) butyl bromide (40.9 µL, 0.38 mmol) and $K_2CO_3$ (350.8 mg, 2.54 mmol)were added. This mixture was heated for 21 h under reflux. Subsequently it was filtered and the solvent removed under vacuum. The crude product (110 mg) was purified using flash-chromatography (0 = 3 cm, h = 15 cm, ethylacetate + 2% N,N-Dimethylethylamine, 20 mL, $R_f$ = 0.28).
Slightly yellow solid, melting point: 78°C, Yield 90.6 mg (80%)
$C_{21}H_{29}N_3O_2$ (355.5)

| | C | H | N |
|---|---|---|---|
| Calc. | 71.0 | 8.22 | 11.8 |
| Found | 70.9 | 8.11 | 11.7 |

MS (ESI): m/z (rel. Int.) = 356 [$MH^+$, 100].
IR (neat): $\tilde{v}$ ($cm^{-1}$) = 2927, 2870 (C-H $_{aliphat.}$), 2807 (C-H), 1599, 1505 (C=C), 1139 (C-O), 758, 694 (C-H).
**$^1$H-NMR** ($CDCl_3$): δ (ppm) = 0.95 (t, J = 7.0 Hz, 3H, $NCH_2CH_2CH_2C\mathit{H}_3$), 1.31 - 1.41 (m, 2H, $NCH_2CH_2C\mathit{H}_2CH_3$), 1.50 - 1.60 (m, 2H, $NCH_2C\mathit{H}_2CH_2CH_3$), 1.95 (dd, J = 13.3/2.3 Hz, 2H, $N(CH_2C\mathit{H}_2)_2$), 2.00 - 2.08 (m, 1 H, $N(CH_2C\mathit{H}_2)_2$), 2.12 (td, J = 11.8/3.1 Hz, 1H, $N(CH_2C\mathit{H}_2)_2$), 2.42 (t, J = 7.8 Hz, 3H, $NC\mathit{H}_2CH_2CH_2CH_3$, $N(C\mathit{H}_2CH_2)_2$), 2.51 (t breit, J = 11.7 Hz, 1H, $N(C\mathit{H}_2CH_2)_2$), 2.78 - 2.88 (m, 2H, $N(C\mathit{H}_2CH_2)_2$), 2.90 (dd, J = 15.7/6.7 Hz, 1H, $C\mathit{H}_2CHOCH_3$), 2.98 (dd, J = 15.7/3.1 Hz, 1H, $C\mathit{H}_2CHOCH_3$), 3.56 (s, 3H, $OCH_3$), 4.86 (dd, J = 7.0/ 3.5 Hz, 1 H, $CH_2C\mathit{H}OCH_3$), 7.29 - 7.36 (m, 1H, Phenyl-C*H*), 7.41 - 7.51 (m, 5H, Phenyl-CH, Pyrazole-3-C*H*).
$^{13}$C-NMR ($CDCl_3$): δ (ppm) = 14.3 (1C, $NCH_2CH_2CH_2CH_3$), 21.1 (1C, $NCH_2CH_2CH_2CH_3$), 29.8 (1C, $NCH_2CH_2CH_2CH_3$), 31.2 (1C, $\mathit{C}H_2CHOCH_3$), 36.7 (1C, $N(CH_2\mathit{C}H_2)_2$), 39.5 (1C, $N(CH_2\mathit{C}H_2)_2$), 49.6 (1C, $N(\mathit{C}H_2CH_2)_2$), 49.7 (1C, N $(\mathit{C}H_2CH_2)_2$), 56.9 (1C, $O\mathit{C}H_3$), 59.0 (1C, $NCH_2CH_2CH_2CH_3$), 71.9 (1C, Spiro-*C*), 77.4 (1C, Pyrazole-4-C), 96.6 (1C, $CH_2CHOCH_3$), 122.8 (2C, Phenyl-CH, ortho), 127.3 (1C, Phenyl-CH, para), 129.5 (2C, Phenyl-CH, meta), 133.7 (1C, Phenyl-C, quartär), 135.9 (1C, Pyrazole-3-*C*H), 139.5 (1C, Pyrazole-5-C).

**Example 10:** 1-Isobutyl-6'-methoxy-1'-phenyl-6',7'-dihydro-1'H-spiro[piperidin-**4,4'-pyrano[4,3-c]pyrazole]**

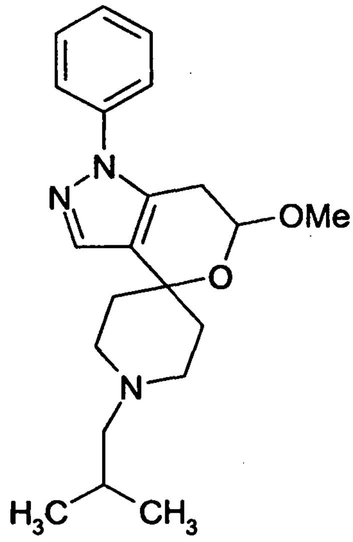

Experimental procedure:

To a solution of crude example 6 (80 mg, 0.27 mmol) in abs. dichloro ethane (1.5 mL) were added consecutively freshly distilled isobutyl-aldehyde (24.4 μL, 0.27 mmol) and Sodiumtriacetoxyborhydride (84.9 mg, 0.40 mmol). This reaction mixture was stirred for 1 h at room temperature. After addition of a saturated solution of $NaHCO_3$ (8 mL) and water (- 4 mL), it was extracted 3 times with $CH_2Cl_2$. Subsequently the pooled organic phases were dried over $K_2CO_3$ and filtered. After evaporation of the solvent under vacuum, the crude product (102 mg) was purified using flash-chromatography (∅ = 3 cm, h = 20 cm, n-hexane:ethylacetate 1:1 + 2% N,N-Dimethylethylamine, 20 mL, $R_f$ = 0.47).
Colourless solid, melting point: 143 °C, Yield 78 mg (82%)
$C_{21}H_{29}N_3O_2$ (355.5)

| | C | H | N |
|---|---|---|---|
| Calc. | 71.0 | 8.22 | 11.8 |
| Found | 70.7 | 8.18 | 11.7 |

MS (ESI): m/z (rel. Int.) = 356 [$MH^+$, 100].
IR (neat): $\tilde{v}$ ($cm^{-1}$) = 2961, 2863 (C-H $_{aliphat.}$), 2809 (C-H), 1600, 1504 (C=C), 1129 (C-O), 756, 696 (C-H).
**$^1$H-NMR** ($CDCl_3$): δ (ppm) = 0.94 (d, J = 6.7 Hz, 6H, $CH_2CH(C\mathit{H}_3)_2$), 1.78 - 1.90 (m, 1 H, $CH_2C\mathit{H}(CH_3)_2$), 1.93 (dd breit, J = 13.7/2.3 Hz, 2H, $N(CH_2C\mathit{H}_2)_2$), 1.98 - 2.07 (m, 1H, $N(CH_2C\mathit{H}_2)_2$), 2.05 - 2.14 (m, 1H, $N(CH_2C\mathit{H}_2)_2$)*, 2.17 (d, J = 7.0 Hz, 2H, $C\mathit{H}_2CH(CH_3)_2$), 2.38 (t breit, J = 11.3 Hz, 1H, $N(C\mathit{H}_2CH_2)_2$), 2.46 (t breit, J = 11.3 Hz, 1H, $N(C\mathit{H}_2CH_2)_2$), 2.69 - 2.79 (m, 2H, $N(C\mathit{H}_2CH_2)_2$), 2.91 (dd, J = 15.7/7.0 Hz, 1 H, $C\mathit{H}_2CHOCH_3$), 2.98 (dd, J = 15.7/3.1 Hz, 1H, $C\mathit{H}_2CHOCH_3$), 3.56 (s, 3H, $OC\mathit{H}_3$), 4.85 (dd, J = 7.0/3.5 Hz, 1 H, $CH_2C\mathit{H}OCH_3$), 7.30 - 7.37 (m, 1H, Phenyl-CH, para), 7.41 - 7.50 (m, 4H, Phenyl-C*H*), 7.52 (s, 1 H, Pyrazole-3-C*H*).
* The Signal is partly overlaid by the dublett of the i-butyl-group.
$^{13}$C-NMR ($CDCl_3$): δ (ppm) = 21.3 (2C, $NCH_2CH(CH_3)_2$). 25.9 (1C, $NCH_2CH(CH_3)_2$). 31.2 (1C, $CH_2CHOCH_3$), 36.7 (1C, $N(CH_2CH_2)_2$), 39.6 (1C, $N(CH_2\mathit{C}H_2)_2$), 49.8 (1C, $N(\mathit{C}H_2CH_2)_2$), 50.1 (1C, $N(CH_2CH_2)_2$), 56.9 (1C, $O\mathit{C}H_3$), 67.5 (1C, $N\mathit{C}H_2CH(CH_3)_2$), 72.1 (1C, Spiro-*C*), 77.4 (1C, Pyrazole-4-C), 96.8 (1C, $CH_2\mathit{C}HOCH_3$), 122.8 (2C, Phenyl-CH, ortho), 127.3 (1C, Phenyl-CH, para), 129.5 (2C, Phenyl-CH, meta), 133.8 (1C, Phenyl-*C*, quartär), 135.9 (1C, Pyrazole-3-*C*H), 139.5 (1C, Pyrazole-5-*C*).

**Example 11: 6'-Methoxy-1-pentyl-1'-phenyl-6',7'-dihydro-1'H-spiro[piperidin-4,4'-pyrano[4,3-c]pyrazole]**

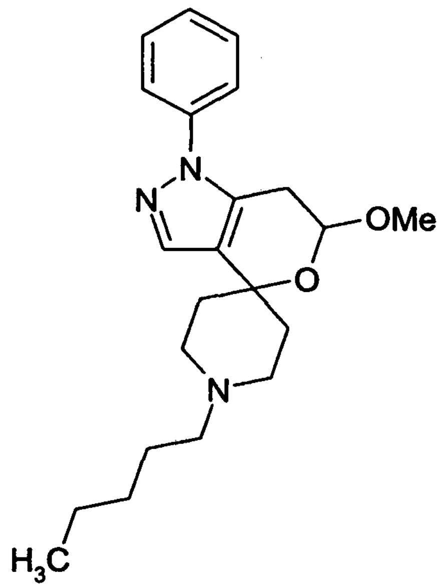

Experimental procedure:

To a solution of crude example 6 (100 mg, 0.33 mmol) in abs. Dichloroethane (2 mL) were added consecutively freshly distilled valeraldehyde (35.6 μL, 0.33 mmol) and Sodiumtriacetoxyborhydride (106.2 mg, 0.50 mmol). This reaction mixture was stirred for 1.5 h at room temperature. Folowing addition of a saturated solution of $NaHCO_3$-(10 mL) and water (~4 mL), it was extracted 3 times with $CH_2Cl_2$. Subsequently the pooled organic phases were dried over $K_2CO_3$ and filtered. After evaporation of the solvent under vacuum the crude product (134 mg) was purified by flash-chromatography (∅ = 3 cm, h = 18 cm, n-hexane:ethylacetate 1:1 + 2% N,N-dimethylethylamine, 20 mL, $R_f$ = 0.34).
Colourless solid, melting point: 87 °C, Yield 101 mg (80%)
$C_{22}H_{31}N_3O_2$ (369.6)

| | C | H | N |
|---|---|---|---|
| Calc. | 71.5 | 8.46 | 11.4 |
| Found | 71.3 | 8.51 | 11.2 |

MS (ESI): m/z (rel. Int.) = 370 [$MH^+$, 100].
IR (neat): $\tilde{v}$ ($cm^{-1}$) = 2925, 2858 (C-H $_{aliphat.}$), 2807 (C-H), 1599, 1505 (C=C), 1139, 1104 (C-O), 758, 695 (C-H).
**$^1$H-NMR** ($CDCl_3$): δ (ppm) = 0.85 (t, J = 7.0 Hz, 3H, $NCH_2(CH_2)_3CH_3$), 1.20-1.32 (m, 4H, $NCH_2CH_2CH_2CH_2CH_3$), 1.44 - 1.55 (m, 2H, $NCH_2CH_2CH_2CH_2CH_3$), 1.90 (dd breit, J = 14.1/2.3 Hz, 2H, $N(CH_2CH_2)_2$), 1.95 - 2.00 (m, 1H, $N(CH_2CH_2)_2$), 2.08 (t breit, J = 11.4 Hz, 1 H, $N(CH_2CH_2)_2$), 2.30 - 2.40 (m, 3H, $NCH_2$ $(CH_2)_3CH_3$, $N(CH_2CH_2)_2$), 2.43 (t breit, J = 11.0 Hz, 1H, $N(CH_2CH_2)_2$), 2.72 - 2.81 (m, 2H, $N(CH_2CH_2)_2$), 2.83 (dd, J = 15.7/6.3 Hz, 1H, $CH_2CHOCH_3$), 2.91 (dd, J = 15.7/3.9 Hz, 1 H, $CH_2CHOCH_3$), 3.50 (s, 3H, $OCH_3$), 4.79 (dd, J = 6.7/3.5 Hz, 1H, $CH_2CHOCH_3$), 7.22 - 7.28 (m, 1 H, Phenyl-C*H*, para), 7.35 - 7.42 (m, 4H, Phenyl-C*H*), 7.44 (s, 1H, Pyrazole-3-C*H*).
$^{13}$C-NMR ($CDCl_3$): δ (ppm) = 14.3 (1C, $NCH_2CH_2CH_2CH_2CH_3$), 22.9 (1C, $NCH_2CH_2CH_2CH_2CH_3$), 27.1 (1C, $NCH_2CH_2$-$CH_2CH_2CH_3$), 30.1 (1C, $NCH_2CH_2CH_2CH_2CH_3$), 31.2 (1C, $CH_2CHOCH_3$), 36.7 (1C, $N(CH_2CH_2)_2$), 39.4 (1C, $N(CH_2CH_2)_2$), 49.6 (1C, $N(CH_2CH_2)_2$), 49.7 (1C, $N(CH_2CH_2)_2$), 56.9 (1C, $OCH_3$), 59.3 (1C, $NCH_2CH_2CH_2CH_2CH_3$), 71.9 (1C, Spiro-*C*), 77.4 (1C, Pyrazole-4-C), 96.9 ($CH_2CHOCH_3$), 122.8 (2C, Phenyl-CH, ortho), 127.3 (1C, Phenyl-CH, para), 129.5 (2C, Phenyl-CH, meta), 133.7 (1C, Phenyl-C, quartär), 135.9 (1C, Pyrazole-3-CH), 139.5 (1C, Pyrazole-5-C).

**Example 12: 1-Isopentyl-6'-methoxy-1'-phenyl-6',7'-dihydro-1'H-spiro[piperidin-4,4'-pyrano[4,3-c]pyrazole]**

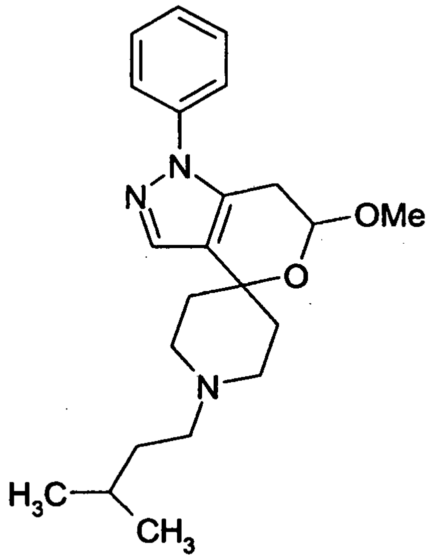

Experimental procedure:

To a solution of crude example 6 (56 mg, 0.19 mmol) in abs. Dichlorethan (2 mL) were added consecutively freshly distilled Isovaleraldehyde (20.1 μL, 0.19 mmol) and Sodiumtriacetoxyborhydride (59.5 mg, 0.28 mmol). This reaction mixture was stirred for 1.5 h at room temperature. After addition of a saturated solution of $NaHCO_3$ (8 mL) and water (- 5 mL), it was extracted 3 times with $CH_2Cl_2$. Subsequently the pooled organic phases were dried over $K_2CO_3$ and filtered. After removal of the solvent under vacuum the crude product (61 mg) was purified using flash-chromatography (∅ = 2 cm, h = 18 cm, Ethylacetat + 2% N,N-Dimethylethylamin, 10 mL, $R_f$ = 0.39).
Colourless resin, Yield 48 mg (70%)
$C_{22}H_{31}N_3O_2$ (369.6)

| | C | H | N |
|---|---|---|---|
| Calc. | 71.5 | 8.46 | 11.4 |
| Found | 71.2 | 8.65 | 11.2 |

MS (ESI): m/z (rel. Int.) = 370 [$MH^+$, 100].
IR (neat): $\tilde{v}$ ($cm^{-1}$) = 3061 (C-H $_{aromat.}$), 2951, 2922, 2867 (C-H $_{aliphat.}$), 2808 (C-H), 1599, 1505 (C=C), 1141 (C-O), 757, 694 (C-H).
**$^1$H-NMR** ($CDCl_3$): δ (ppm) = 0.93 (d, J = 6.7 Hz, 6H, $NCH_2CH_2CH$-($CH_3$)$_2$), 1.41 - 1.50 (m, 2H, $NCH_2CH_2CH$-($CH_3$)$_2$), 1.57-1.66 (m, 1H, $NCH_2CH_2CH(CH_3)_2$), 1.96 (dd breit, J = 13.7/2.0 Hz, 2H, $N(CH_2CH_2)_2$), 2.02 - 2.08 (m, 1H, N ($CH_2CH_2$)$_2$), 2.09 - 2.17 (m, 1H, $N(CH_2CH_2)_2$), 2.37 - 2.55 (m, 4H, $NCH_2CH_2CH(CH_3)_2$ (2H), $N(CH_2CH_2)_2$), (2H)), 2,79 - 2.83 (m, 2H, $N(CH_2CH_2)_2$), 2.91 (dd, J = 15.7/7.0 Hz, 1H, $CH_2CHOCH_3$), 2.99 (dd, J = 15.7/3.9 Hz, 1H, $CH_2CHOCH_3$), 3.56 (s, 3H, $OCH_3$), 4.86 (dd, J = 6.7/3.5 Hz, 1H, $CH_2CHOCH_3$), 7.30 - 7.35 (m, 1H, Phenyl-C*H*, para), 7.41 - 7.48 (m, 4H, Phenyl-C*H*), 7.50 (s, 1H, Pyrazole-3-C*H*).
**$^{13}$C-NMR** ($CDCl_3$): δ (ppm) = 23.0 (2C, $NCH_2CH_2CH(CH_3)_2$), 27.1 (2C, $NCH_2CH_2CH(CH_3)_2$), 31.2 (1C, $CH_2CHOCH_3$), 36.3 (1C, $NCH_2CH_2CH(CH_3)_2$), 36.7 (1C, $N(CH_2CH_2)_2$), 39.5 (1C, $N(CH_2CH_2)_2$), 49.7 (1C, $N(CH_2CH_2)_2$), 49.8 (1C, N ($CH_2CH_2$)$_2$). 56.9 (1C, $OCH_3$), 57.5 (1C, $NCH_2CH_2CH(CH_3)_2$), 71.9 (1C, Spiro-*C*), 77.4 (1C, Pyrazole-4-C), 96.9 (1C, $CH_2CHOCH_3$), 122.8 (2C, Phenyl-CH, ortho), 127.3 (1C, Phenyl-CH, para), 129.5 (2C, Phenyl-*C*H, meta), 133.7 (1C, Phenyl-*C*, quartär), 135.9 (1C, Pyrazole-3-*C*H), 139.5 (1C, Pyrazole-5-C).

**Example 13: 6'-Methoxy-1-(3-methylbut-2-en-1-yl)-1'-phenyl-6',7'-dihydro-1'H-spiro[piperidin-4,4'-pyrano[4,3-c] pyrazole]**

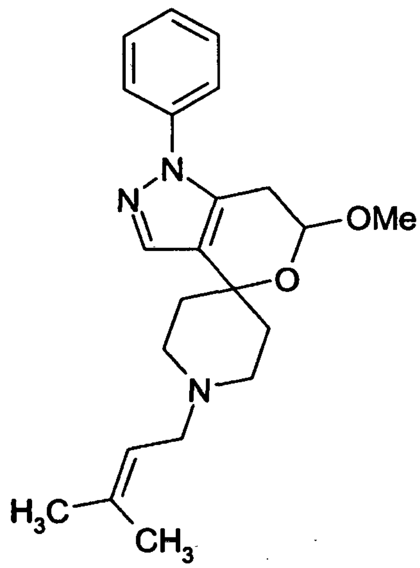

Experimental procedure:

To a solution of crude example 6 (100 mg, 0.33 mmol) in acetonitrile (5 mL) 1-Bromo-3-methyl-but-2-en (47.0 μL, 0.40 mmol) and $K_2CO_3$ (369.3 mg, 2.67 mmol) were added. This mixture was heated for 23 h under reflux. Subsequently it was filtered and the solvent removed under vacuum. The crude product (142 mg) was purified using flash-chromatography (0 = 3 cm, h = 18 cm, n-hexane:ethylacetate 3:7 + 2% N,N-Dimethylethylamine, 20 mL, $R_f$ = 0.16).
Colourless Oil, Yield 89 mg (72 %)
$C_{22}H_{29}N_3O_2$ (367.5)

| | C | H | N |
|---|---|---|---|
| Calc. | 71.9 | 7.95 | 11.4 |
| Found | 71.6 | 7.97 | 11.2 |

MS (ESI): m/z (rel. Int.) = 368 [$MH^+$, 100], 734 [2 x $M^+$, 12].
**IR** (neat): $\tilde{v}$ ($cm^{-1}$) = 3025 (C-H $_{aromat.}$), 2917 (C-H $_{aliphat.}$), 1599, 1505 (C=C), 1059, 1045 (C-O).
**$^1$H-NMR** ($CDCl_3$): δ (ppm) = 1.75 (s, 3H, ($H_3$C)$_2$C=CHCH$_2$N), 1.82 (s, 3H, ($H_3$C)$_2$C=CHCH$_2$N), 1.92 - 2.07 m, 2H, N (CH$_2$C$H_2$)$_2$), 2.09 - 2.24 (m, 2H, N(CH$_2$C$H_2$)$_2$), 2.48 (td, J = 11.8/2.8 Hz, 1H, N(C$H_2$CH$_2$)$_2$), 2.56 (td, J = 11.8/2.8 Hz, 1 H, N(C$H_2$CH$_2$)$_2$), 2.86 - 2.96 (m, 2H, N(C$H_2$CH$_2$)$_2$), 2.97 (dd, J = 15.7/6.7 Hz, 1H, C$H_2$CHOCH$_3$), 3,05 (dd, J = 15.7/3.8 Hz, 1 H, C$H_2$CHOCH$_3$), 3.11 (d, J = 7.2 Hz, 2H, (H$_3$C)$_2$C=CHC$H_2$N), 3.64 (s, 3H, OC$H_3$), 4.92 (dd, J = 6.7/3.8 Hz, 1H, CH$_2$C$H$OCH$_3$), 5.39 (t breit, J = 7.2 Hz, 1H, (H$_3$C)$_2$C=C$H$CH$_2$N), 7.36 - 7.43 (m, 1H, Phenyl-CH, para), 7.50 - 7.54 (m, 4H, Phenyl-C$H$), 7.56 (s, 1H, Pyrazole-3-C$H$).

**Example 14: 6'-Methoxy-1-octyl-1'-phenyl-6',7'-dihydro-1'H-spiro[piperidin-4,4'-pyrano[4,3-c]pyrazole]**

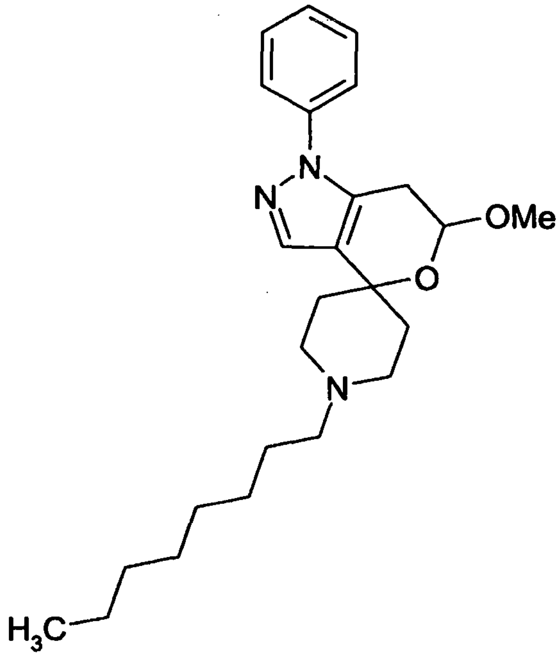

Experimental procedure:

To a solution of crude example 6 (80 mg, 0.27 mmol) in acetonitrile (5 mL) 1-Bromoctane (55.6 μL, 0.32 mmol) and $K_2CO_3$ (295 mg, 2.14 mmol) were added. This mixture was heated for 19 h under reflux. Subsequently the $K_2CO_3$ was filtered off and the solvent removed under vacuum. The crude product (105 mg) was purified using flash-chromatography (0 = 3 cm, h = 20 cm, n-hexane:ethylacetate 1:1 + 2% N,N-Dimethylethylamine, 20 mL, $R_f$ = 0.21).
Colourless solid, melting point: 86 °C, Yield 74 mg (67%)
$C_{25}H_{37}N_3O_2$ (411.7)

| | C | H | N |
|---|---|---|---|
| Calc. | 73.0 | 9.06 | 10.2 |
| Found | 72.7 | 9.07 | 10.1 |

MS (ESI): m/z (rel. Int.) = 412 [$MH^+$, 100].
IR (neat): $\tilde{\nu}$ ($cm^{-1}$) = 3001 (C-H $_{aromat.}$), 2951, 2919 (C-H $_{aliphat.}$), 2851, 2806 (C-H), 1596, 1504 (C=C), 1126, 1060 (C-O).
**$^1$H-NMR** ($CDCl_3$): δ (ppm) = 0.90 (t, J = 7.0 Hz, 3H, $NCH_2(CH_2)_6CH_3$), 1.22-1.38 (m, 10H, $NCH_2CH_2(CH_2)_5CH_3$), 1.50 - 1.60 (m, 2H, $NCH_2CH_2(CH_2)_5CH_3$), 1.95 (dd, J = 12.5/1.6 Hz, 2H, $N(CH_2CH_2)_2$), 2.00 - 2.09 (m, 1H, $N(CH_2CH_{2\ axial})_2$), 2.13 (td, J = 12.9/3.9 Hz, 1H, $N(CH_2CH_2$ axial$)_2$), 2.39 - 2.48 (m, 3H, $NCH_2(CH_2)_6CH_3$ (2H), $NCH_2CH_2)_2$ (1H)), 2.51 (t breit, J = 10.6 Hz, 1H, $N(CH_2CH_2)_2$), 2.80 - 2.90 (m, 2H, $N(CH_2CH_2)_2$), 2.92 (dd, J = 15.7/7.0 Hz, 1H, $CH_2CHOCH_3$), 2.98 (dd, J = 15.7/3.1 Hz, 1H, $CH_2CHOCH_3$), 3.57 (s, 3H, $OCH_3$), 4.87 (dd, J = 6.7/3.5 Hz, 1H, $CH_2CHOCH_3$), 7.33 (m, 1H, Phenyl-CH, para), 7.43 - 7.51 (m, 4H, Phenyl-CH), 7.51 (s, 1 H, Pyrazole-3-CH).
**$^{13}$C-NMR** ($CDCl_3$): δ (ppm) = 14.3 (1C, $NCH_2(CH_2)_6CH_3$), 22.9 (1C, $NCH_2(CH_2)_5CH_2CH_3$). 27.4 (1C, $NCH_2CH_2(CH_2)_5CH_3$), 28.0 (1C, $NCH_2(CH_2)_4CH_2CH_2CH_3$), 29.5 ($N(CH_2)_3CH_2CH_2CH_2-CH_3$), 29.8 (1C, $NCH_2(CH_2)_2CH_2(CH_2)3CH_3$). 31.2 (1C, $CH_2CHOCH_3$), 32.1 (1C, $NCH_2CH_2CH_2(CH_2)_4CH_3$), 36.7 (1C, $N(CH_2CH_2)_2$), 39.5 (1C, $N(CH_2CH_2)_2$). 49.6 (1C, $N(CH_2CH_2)2$), 49.7 (1C, $N(CH_2CH_2)_2$). 56.9 (1C, $OCH_3$), 59.4 (1C, $NCH_2(CH_2)_6CH_3$), 71.9 (1C, Spiro-C), 77.4 (1C, Pyrazole-4-C), 96.9 (1C, $CH_2CHOCH_3$), 122.8 (2C, Phenyl-CH, ortho), 127.3 (1C, Phenyl-CH, para), 129.5 (2C, Phenyl-CH, meta), 133.7 (1C, Phenyl-C, quartär), 135.9 (1C, Pyrazole-3-CH), 139.5 (1C, Pyrazole-5-C).

**Example 15: 1-(Cyclohexan-1-ylmethyl)-6'-methoxy-1'-phenvi-6',7'-dihydro-1'H-spiro[piperidin-4,4'-pyrano [4,3-c]pyrazole]**

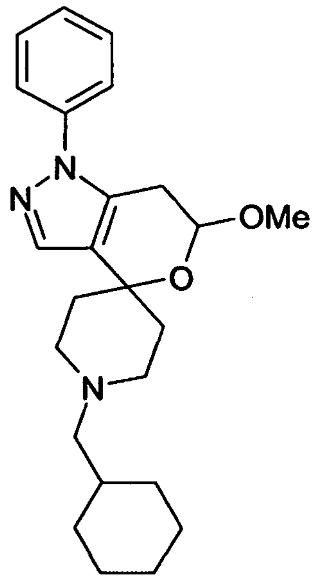

Experimental procedure:

To a solution of crude example 6 (80 mg, 0.27 mmol) in acetonitrile (5 mL) (Bromomethyl)cyclohexane (48.5 μL, 0.35 mmol) and $K_2CO_3$ (295 mg, 2.14 mmol) were added. This mixture was heated for 26 h under reflux. Subsequently $K_2CO_3$ was filtered off and the solvent removed under vacuum. The crude product (103 mg) was purified using flash-chromatography (∅ = 2.5 cm, h = 15 cm, n-hexane:ethylacetate 7:3 + 1 % N,N-Dimethylethylamine, 10 mL, $R_f$ = 0.18). Colourless solid, melting point: 151 °C, Yield 82 mg (77%)
$C_{24}H_{33}N_3O_2$ (395.6)

| | C | H | N |
|---|---|---|---|
| Calc. | 72.9 | 8.41 | 10.6 |
| Found | 72.7 | 8.39 | 10.4 |

MS (ESI): m/z (rel. Int.) = 396 [$MH^+$, 100], 813 [2 x M + $Na^+$, 7].
IR (neat): $\tilde{v}$ ($cm^{-1}$) = 2918 (C-H $_{aliphat.}$), 2848 (C-H), 1599, 1505 (C=C), 1114, 1059 (C-O).
**$^1$H-NMR** ($CDCl_3$): δ (ppm) = 0.84 - 0.97 (m, 2H, $NCH_2C_6H_{11}$), 1.18 - 1.29 (m, 4H, $NCH_2C_6H_{11}$), 1.47 - 1.59 (m, 1H, $NCH_2C_6H_{11}$), 1.62 - 1.87 (m, 4H, $NCH_2C_6H_{11}$), 1.90 - 1.98 (m, 2H, $N(CH_2CH_2)_2$), 2.02 - 2.15 (m, 2H, $N(CH_2CH_2)_2$), 2.21 (d, J = 7.0 Hz, 2H, $NCH_2C_6H_{11}$), 2.37 (td, J = 11.4/2.4 Hz, 1H, $N(CH_2CH_2)_2$), 2.45 (td, J = 11.7/2.4 Hz, 1H, N ($CH_2CH_2)_2$), 2.70 - 2.79 (m, 2H, $N(CH_2CH_2)_2$), 2.90 (dd, J = 15.7/7.0 Hz, 1 H, $CH_2CHOCH_3$), 2.97 (dd, J = 15.7/3.5 Hz, 1 H, $CH_2CHOCH_3$), 3.56 (s, 3H, $OCH_3$), 4.84 (dd, J = 6.7/3.5 Hz, 1H, $CH_2CHOCH_3$), 7.30 - 7.35 (m, 1H, Phenyl-CH, para), 7.41 - 7.48 (m, 4H, Phenyl-CH), 7.51 (s, 1 H, Pyrazole-3-CH).

**Example 16: 6'-Methoxy-1'-phenyl-1-(2-phenylethyl)-6',7'-dihydro-1'H-spiro(piperidin-4,4'-pyrano[4,3-c]pyrazole]**

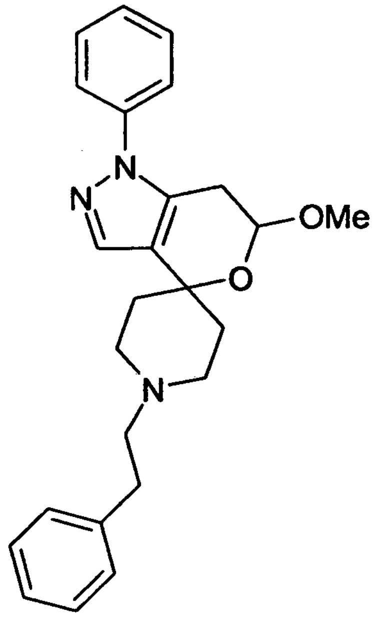

Experimental procedure:

To a solution of crude example 6 (80 mg, 0.27 mmol) in abs. dichloroethane (1.5 mL) were added consecutively freshly distilled phenyl-acetaldehyde (31.2 μL, 0.27 mmol) and sodiumtriacetoxyborhydride (84.9 mg, 0.40 mmol). This reaction mixture was stirred for 1.5 h at room temperature. After addition of a saturated solution of $NaHCO_3$ (6 mL) and water (~ 5 mL), it was extracted 3 times with $CH_2Cl_2$. Subsequently the pooled organic phases were dried over $K_2CO_3$ and filtered. After removal of the solvent under vacuum the crude product (125 mg) was purified using flash-chromatography (∅ = 3 cm, h = 18 cm, n-hexane:ethylacetate 1:1 + 2% N,N-Dimethylethylamine, 20 mL, $R_f$ = 0.39).
Slightly yellow resin, Yield 91 mg (84 %).
$C_{25}H_{29}N_3O_2$ (403.6)

| | C | H | N |
|---|---|---|---|
| Calc. | 74.4 | 7.24 | 10.4 |
| Found | 73.9 | 7.24 | 10.1 |

**MS** (ESI): m/z (rel. Int.) = 404 [$MH^+$, 100].
**IR** (neat): $\tilde{v}$ ($cm^{-1}$) = 3060, 3026 (C-H $_{aromat.}$), 2919 (C-$H_{aliphat.}$), 2809 (C-H), 1599, 1504 (C=C), 1137, 1113 (C-O), 758, 696 (C-H).
**$^1$H-NMR** ($CDCl_3$): δ (ppm) = 1.95 - 2.11 (m, 3H, $N(CH_2CH_2)_2$), 2.17 (t breit, J = 11.0 Hz, 1H, $N(CH_2CH_2)_2$), 2.54 (t breit, J = 11.0 Hz, 1H, $N(CH_2CH_2)_2$), 2.63 (t breit, J = 11.0 Hz, 1H, $N(CH_2CH_2)_2$), 2.68 - 2.76 (m, 2H,$NCH_2CH_2Ph$), 2.83 - 3.02 (m, 6H, $N(CH_2CH_2)_2$ (2H), $NCH_2CH_2Ph$ (2H), $CH_2CHOCH_3$) (2H)), 3.56 (s, 3H, $OCH_3$), 4.87 (dd, J = 6.7/3.5 Hz, 1H, $CH_2CHOCH_3$), 7.19 - 7.37 (m, 5H, Phenyl-C*H*), 7.41 - 7.50 (m, 5H, Phenyl-C*H*), 7.51 (s, 1 H, Pyrazole-3-C*H*).
**$^{13}$C-NMR** ($CDCl_3$): δ (ppm) = 31.3 (1C, $CH_2CHOCH_3$), 34.1 (1C, $NCH_2CH_2Ph$), 37.1 (1C, $N(CH_2CH_2)_2$), 39.8 (1C, N $(CH_2CH_2)_2$), 49.6 (1C, $N(CH_2CH_2)_2$), 49.8 (1C, $N(CH_2CH_2)_2$), 57.0 (1C, $OCH_3$), 61.1 (1C, $NCH_2CH_2Ph$), 71.8 (1C, Spiro-C), 77.5 (1C, Pyrazole-4-*C*), 97.0 (1C, $CH_2CHOCH_3$), 122.9 (2C, Phenyl-CH), 126.4 (1C, Phenyl-C, quartär), 127.4, 128.7, 129.0, 129.5 (8C, Phenyl-CH), 133.8 (1C, Phenyl-C, quartär), 135.9 (1C, Pyrazole-3-*C*H), 139.5 (1C, Pyrazole-5-C).

**Example 17: 6'-Methoxy-1'-phenyl-1-(3-phenylpropyl)-6',7'-dihydro-1'H-spiro[piperidin-4,4'-pyrano[4,3-c]pyrazole]**

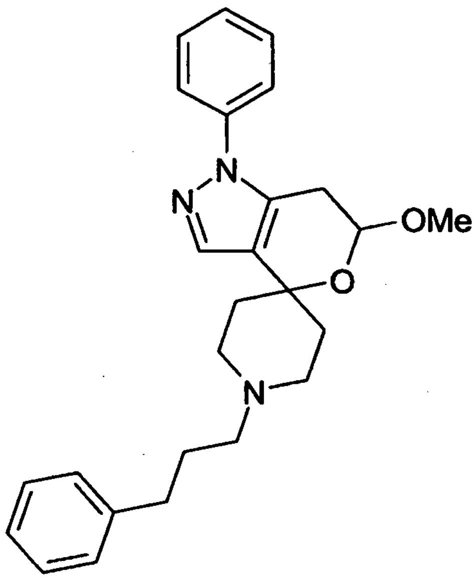

Experimental procedure:

To a solution of crude example 6 (120 mg, 0.40 mmol) in acetonitrile (10 mL) 1-Bromo-3-phenylpropane (72.8 μL, 0.48 mmol) and $K_2CO_3$ (443.1 mg, 3.21 mmol) were added. This mixture was heated for 19 h under reflux. Subsequently it was filtered and the solvent removed under vacuum. The crude product (158 mg) was purified using flash-chromatography (Ø = 3 cm, h = 15 cm, n-hexane:ethylacetate 1:1 + 2% N,N-Dimethylethylamine, 20 mL, $R_f$ = 0.14). Colourless resin, Yield 121 mg (72%)
$C_{26}H_{31}N_3O_2$ (417.6)

| | C | H | N |
|---|---|---|---|
| Calc. | 74.8 | 7.48 | 10.1 |
| Found | 74.3 | 7.50 | 9.82 |

MS (ESI): m/z (rel. Int.) = 418 [$MH^+$, 100].
**IR** (neat): $\tilde{v}$ ($cm^{-1}$) = 3060, 3025 (C-H $_{aromat.}$), 2941 (C-H $_{aliphat.}$), 2808 (C-H), 1599, 1504 (C=C), 1137, 1115 (C-O), 758, 695 (C-H).
**$^1$H-NMR** ($CDCl_3$): δ (ppm) = 1.85 - 2.16 (m, 6H, $N(CH_2CH_2)_2$ (4H), $NCH_2CH_2CH_2Ph$ (2H)), 2.38 - 2.58 (m, 4H, $NCH_2CH_2CH_2Ph$ (2H), $N(CH_2CH_2)_2$ (2H)), 2.68 (t, J = 7.8 Hz, 2H, $NCH_2CH_2CH_2Ph$), 2.77 - 2.87 (m, 2H, $N(CH_2CH_2)_2$), 2.90 (dd, J = 15.7/7.0 Hz, 1H, $CH_2CHOCH_3$), 2.97 (dd, J = 15.3/3.5 Hz, 1H, $CH_2CHOCH_3$), 3.55 (s, 3H, $OCH_3$), 4.85 (dd, J = 7.0/3.1 Hz, 1 H, $CH_2CHOCH_3$), 7.17 - 7.36 (m, 6H, Phenyl-C*H*), 7.41 - 7.48 (m, 4H, Phenyl-C*H*), 7.50 (s, 1H, Pyrazole-3-CH).
**$^{13}$C-NMR** ($CDCl_3$): δ (ppm) = 29.0 (1C, $NCH_2CH_2CH_2Ph$), 31.2 (1C, $CH_2CHOCH_3$), 34.1 (1C, $NCH_2CH_2CH_2Ph$), 36.7 (1C, $N(CH_2CH_2)_2$), 39.5 (1C, $N(CH_2CH_2)_2$), 49.5 (1C, $N(CH_2CH_2)_2$), 49.7 (1C, $N(CH_2CH_2)_2$), 56.9 (1C, $OCH_3$), 58.6 (1C, $NCH_2CH_2CH_2Ph$), 71.8 (1C, Spiro-C), 77.5 (1C, Pyrazole-4-C), 96.9 (1C, $CH_2CHOCh_3$), 122.8 (2C, Phenyl-CH), 124.2 (1C, Phenyl-C, quartär), 126.0, 127.3, 128.56, 128.63, 129.5 (8C, Phenyl-CH), 133.7 (1C, Phenyl-C, quartär), 135.8 (1C, Pyrazole-3-*C*H), 139.5 (1C, Pyrazole-5-C).

**Example 18: 6'-Methoxy-1'-phenyl-1-(4-phenylbutyl)-6',7'-dihydro-1'H-spiro[piperidin-4,4'-pyrano[4,3-c]pyrazole]**

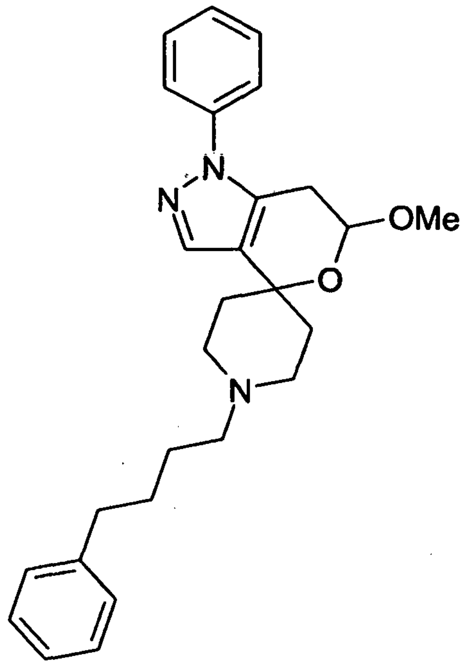

Experimental procedure:

To a solution of crude example 6 (120 mg, 0.40 mmol) in acetonitrile (8 mL) were added consecutively $K_2CO_3$ (443 mg, 3.21 mmol), tetrabutylammoniumiodide (29.6 mg, 0.08 mmol) and 1-Chlor-4-phenylbutane (79.1 $\mu$L, 0.48 mmol). The mixture was heated for 26 h under reflux. Subsequently the $K_2CO_3$ was filtered off and the solvent removed under vacuum. The residue was dissolved in ethylacetate, diluted with water and several times extracted. The pooled organic phases were dried over $K_2CO_3$, filtered and following that the solvent was removed under vacuum. The crude product (157 mg) was purified using flash-chromatography (0 = 3 cm, h = 15 cm, n-hexane:ethylacetate 1:1 + 2% N,N-Dimethylethylamine, 20 mL, $R_f$ = 0.17).
Colourless resin, Yield 83 mg (48%).
$C_{27}H_{33}N_3O_2$ (431.6)

| | C | H | N |
|---|---|---|---|
| Calc. | 75.1 | 7.71 | 9.74 |
| Found | 74.7 | 7.87 | 9.64 |

**MS** (ESI): m/z (rel. Int.) = 432 [$MH^+$, 100].
**IR** (neat): $\tilde{v}$ ($cm^{-1}$) = 3060, 3025 (C-$H_{aromat.}$), 2930, 2857 (C-$H_{aliphat.}$), 2807 (C-H), 1599, 1504 (C=C), 1137, 1115 (C-O), 758, 696 (C-H).
**$^1$H-NMR** ($CDCl_3$): $\delta$ (ppm) = 1.56 - 1.72 (m, 4H, $NCH_2CH_2CH_2CH_2Ph$), 1.91 - 2.17 (m, 4H, $N(CH_2CH_2)_2$), 2.38 - 2.55 (m, 4H, $N(CH_2CH_2)_2$ (2H), $NCH_2CH_2CH_2CH_2Ph$ (2H)), 2.66 (t, J = 7.4 Hz, 2H, $NCH_2CH_2CH_2CH_2Ph$), 2.77 - 2.86 (m, 2H, $N(CH_2CH_2)_2$), 2.90 (dd, J = 15.6/6.8 Hz, 1H, $CH_2CHOCH_3$), 2.98 (dd, J = 15.4/3.4 Hz, 1H, $CH_2CHOCH_3$), 3.55 (s, 3H, $OCH_3$), 4.85 (dd, J = 6.8/3.5 Hz, 1H, $CH_2CHOCH_3$), 7.15-7.21 (m, 3H, Phenyl-CH), 7.23 - 7.35 (m, 3H, Phenyl-CH), 7.42 - 7.48 (m, 4H, Phenyl-CH), 7.49 (s, 1H, Pyrazole-3-CH).
**$^{13}$C-NMR** ($CDCl_3$): $\delta$ (ppm) = 27.2, 29.7 (je 1C, $NCH_2CH_2CH_2CH_2Ph$), 31.2 (1C, $CH_2CHOCH_3$), 36.1 (1C, $NCH_2CH_2CH_2CH_2Ph$), 36.6 (1C, $N(CH_2CH_2)_2$), 39.4 (1C, $N(CH_2CH_2)_2$), 49.5 (1C, $N(CH_2CH_2)_2$), 49.7 (1C, $N(CH_2CH_2)_2$), 56.9 (1C, $OCH_3$), 59.0 (1C, $NCH_2CH_2CH_2CH_2Ph$), 71.8 (1C, Spiro-C), 77.5 (1C, Pyrazole-4-C), 96.9 (1C, $CH_2CHOCH_3$), 122.8 (2C, Phenyl-CH), 124.3 (1C, Phenyl-C, quartär), 125.9, 127.3, 128.5, 128.6, 129.5 (8C, Phenyl-CH), 133.7 (1C, Phenyl-C, quartär), 135.8 (1C, Pyrazole-3-CH), 139.5 (1C, Pyrazole-5-C).

**Example 19: 1-(Furan-2-ylmethyl)-6'-methoxy-1'-phenyl-6',7'-dihydro-1'H-spiro[piperidin-4,4'-pyrano[4,3-c] pyrazole]**

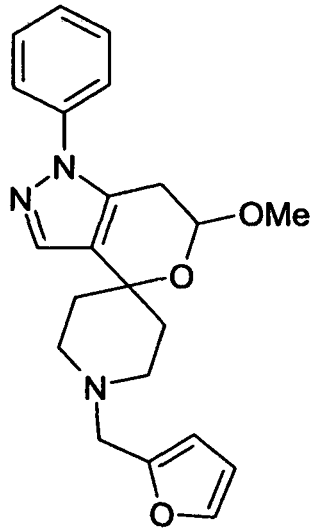

Experimental procedure:

To a solution of crude example 6 (100 mg, 0.33 mmol) in abs. Dichloroethane (2 mL) were added consecutively freshly distilled Furan-2-carbaldehyde (27.7 μL, 0.33 mmol) and sodiumtriacetoxyborhydride (106.2 mg, 0.50 mmol). This reaction mixture was stirred for 7 h at room temperature. After addition of a saturated solution of $NaHCO_3$ (6 mL) and water (~ 8 mL), it was extracted 3 times with $CH_2Cl_2$. Subsequently the pooled organic phases were dried over $K_2CO_3$ and filtered. After removal of the solvent under vacuum the crude product (112 mg), was prified using flash-chromatography (Ø = 3 cm, h = 18 cm, n-hexane:ethylacetate 1:1 + 2% N,N-Dimethylethylamine, 20 mL, $R_f$ = 0.33). Colourless resin, Yield 99 mg (78 %).
$C_{22}H_{25}N_3O_3$ (379.5)

| | C | H | N |
|---|---|---|---|
| Calc. | 69.6 | 6.64 | 11.1 |
| Found | 69.1 | 6.66 | 10.6 |

**MS** (ESI): m/z (rel. Int.) = 380 [$MH^+$, 100].
**IR** (neat): $\tilde{v}$ ($cm^{-1}$) = 3111 (C-$H_{aromat.}$), 2919 (C-$H_{aliphat.}$), 2812 (C-H), 1598, 1504 (C=C), 1139, 1111 (C-O), 758, 695 (C-H).
**$^1$H-NMR** ($CDCl_3$): δ (ppm) = 1.91 - 2.07 (m, 3H, $N(CH_2CH_2)_2$), 2.14 (td, J = 12.9/4.4 Hz, 1H, $N(CH_2CH_2)_2$), 2.50 (td, J = 11.5/2.9 Hz, 1H, $N(CH_2CH_2)_2$), 2.59 (td, J = 11.7/2.4 Hz, 1H, $N(CH_2CH_2)_2$), 2.77 - 2.86 (m, 2H, $N(CH_2CH_2)_2$), 2.88 (dd, J = 15.7/7.0 Hz, 1 H, $CH_2CHOCH_3$), 2.97 (dd, J = 15.7/3.1 Hz, 1 H, $CH_2CHOCH_3$), 3.53 (s, 3H, $OCH_3$), 3.61 (d, J = 14.1 Hz, 1H, $NCH_2$Furyl), 3.65 (d, J = 14.1 Hz, 1H, $NCH_2$Furyl), 4.84 (dd, J = 6.9/3.3 Hz, 1H, $CH_2CHOCH_3$), 6.24 (d, J = 2.7 Hz, 1H, Furan-3-C*H*). 6.32 - 6.36 (m, 1 H, Furan-4-C*H*), 7.30 - 7.36 (m, 1H, Phenyl-CH, para), 7.39 - 7.50 (m, 6H, Phenyl-CH (4), Pyrazole-3-CH (1 H), Furan-5-C*H* (1 H)).
**$^{13}$C-NMR** ($CDCl_3$): δ (ppm) = 31.2 (1C, $CH_2CHOCH_3$), 36.5 (1C, $N(CH_2CH_2)_2$), 39.3 (1C, $N(CH_2CH_2)_2$), 49.2 (1C, $N(CH_2CH_2)_2$), 49.3 (1C, $N(CH_2CH_2)_2$), 55.4 (1C, $NCH_2$Furyl), 56.9 (1C, $OCH_3$), 71.6 (1C, Spiro-*C*), 77.5 (1C, Pyrazole-4-*C*), 96.8 (1C, $CH_2CHOCH_3$), 109.1 (1C, Furan-3'-*C*H), 110.3 (1C, Furan-4'-*C*H), 122.8 (2C, Phenyl-CH, ortho), 124.3 (1C, Phenyl-*C*, quartär), 127.3 (1C, Phenyl-CH, para), 129.5 (2C, Phenyl-CH, meta), 133.8 (1C, Furan-2'-C), 135.8 (1C, Pyrazole-3-CH), 139.5 (1C, Pyrazole-5-*C*), 142.5 (1C, Furan-5'-CH).

**Example 20: 6'-Methoxy-1-(4-methoxybenzyl)-1'-phenyl-6',7'-dihydro-1'H-spiro[piperidin-4,4'-pyrano[4,3-c] pyrazole]**

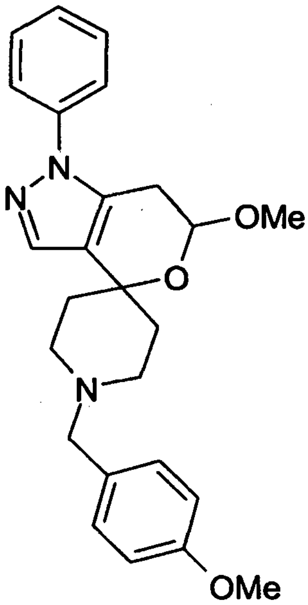

Experimental procedure:

To a solution of crude example 6 (80 mg, 0.27 mmol) in abs. dichloroethane (1.5 mL) were added consecutively freshly distillied p-Methoxy-benzaldehyde (32.4 μL, 0.27 mmol) and sodiumtriacetoxyborhydride (84.9 mg, 0.40 mmol). This reaction mixture was stirred for 17 h at room temperature. After adding a saturated solution of $NaHCO_3$ (8 mL) and water (- 5 mL), it was extracted 3 times with $CH_2Cl_2$. Subsequently the pooled organic phases were dried over $K_2CO_3$ and filtered. Following removal of the solvent under vacuum the crude product (126 mg) was purified using flash-chromatography (Ø = 3 cm, h = 20 cm, n-hexane:ethylacetate 1:1 + 2% N,N-Dimethylethylamine, 20 mL, $R_f$ = 0.13). Colourless resin, Yield 79 mg (70 %).
$C_{25}H_{29}N_3O_3$ (419.6)

| | C | H | N |
|---|---|---|---|
| Calc. | 71.6 | 6.97 | 10.0 |
| Found | 71.5 | 7.12. | 9.79 |

**MS** (ESI): m/z (rel. Int.) = 420 [$MH^+$, 100], 838 [2 x M, 67].
**IR** (neat): $\tilde{v}$ ($cm^{-1}$) = 2916 (C-$H_{aliphat.}$), 2833 (C-H), 1599, 1505 (C=C), 1114, 1059 (C-O).
**$^1$H-NMR** ($CDCl_3$): δ (ppm) = 1.89 - 1.96 (m, 2H, N($CH_2$C$H_2$)$_2$), 1.99 - 2.06 (m, 1H, N($CH_2$C$H_{2\,axial}$)$_2$), 2.05 - 2.15 (m, 1 H, N($CH_2$C$H_{2axial}$)$_2$), 2.45 (t breit, J = 11.7 Hz, 1 H, N(C$H_2$$CH_2$)$_2$), 2.55 (t breit, J = 11.7 Hz, 1H, N(C$H_2$$CH_2$)$_2$), 2.73 - 2.82 (m, 2H, N(C$H_2$$CH_2$)$_2$), 2.90 (dd, J = 15.7/6.3 Hz, 1 H, C$H_2$$CHOCH_3$), 2.98 (dd, J = 15.7/3.9 Hz, 1H, C$H_2$$CHOCH_3$), 3.55 (s, 5H, OC$H_3$ (3H), N$CH_2$Ph (2H)), 3.81 (s, 3H, Ph$OCH_3$), 4.85 (dd, J = 6.7/3.5 Hz, 1H, $CH_2$C*H*$OCH_3$), 6.88 (d, J = 8.6 Hz, 2H, Benzyl-C*H*, ortho), 7.24 - 7.36 (m, 3H, Phenyl-C*H*, para (1H), Benzyl-C*H*, meta (2H)), 7.41 - 7.48 (m, 4H, Phenyl-CH, ortho + meta), 7.50 (s, 1H, Pyrazole-3-C*H*).
**$^{13}$C-NMR** ($CDCl_3$): δ (ppm) = 31.2 (1C, C$H_2$$CHOCH_3$), 36.7 (1C, N($CH_2$C$H_2$)$_2$), 39.5 (1C, N($CH_2$C$H_2$)$_2$), 49.3 (1C, N (C$H_2$$CH_2$)$_2$), 49.4 (1C, N(C$H_2$$CH_2$)$_2$), 55.5 (1C, PhenylOC$H_3$), 56.9 (1C, $OCH_3$), 63.0 (1C, NC$H_2$Ph), 71.9 (1C, Spiro-*C*), 77.5 (1C, Pyrazole-4-C), 96.8 (1C, $CH_2$$CHOCH_3$), 113.8 (2C, Benzyl-2',6'-*C*H), 114.2 (1C, Benzyl-4'-*C*, quartär), 122.8 (2C, Phenyl-CH, ortho), 124.4 (1C, Phenyl-C, quartär), 127.3 (1C, Phenyl-CH, para), 129.5 (2C, Phenyl-CH, meta), 130.7 (2C, Benzyl-3',5'-CH), 133.8 (1C, Benzyl-1'-*C*), 135.9 (1C, Pyrazole-3-CH), 139.5 (1C, Pyrazole-5-C).

**Example 21: 1-(4-Fluorbenzyl)-6'-methoxy-1'-phenyl-6',7'-dihydro-1'H-spiro[piperidin-4,4'-pyrano[4,3-c]pyrazole]**

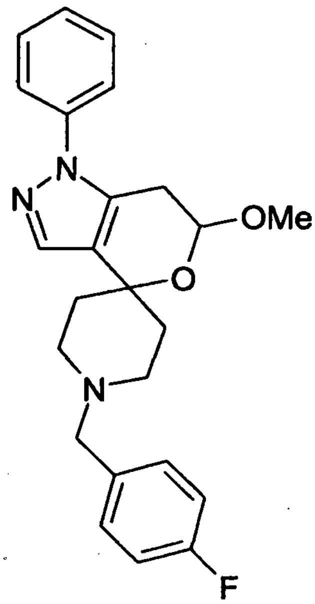

Experimental procedure:

To a solution of crude example 6 (100 mg, 0.33 mmol) in abs. dichloroethane (2 mL) were added consecutively the freshly distilled p-fluorobenzaldehyde (36.0 μL, 0.33 mmol) and sodiumtriacetoxyborhydride (106.2 mg, 0.50 mmol). This reaction mixture was stirred for 19 h at room temperature. Following addition of a saturated solution of $NaHCO_3$-Solution (10 mL) and water (- 8 mL), it was extracted 3 times with $CH_2Cl_2$. Subsequently the pooled organic phases were dried over $K_2CO_3$ and filtered. After rermoval of th solvent under vacuum the crude product (145 mg) was purified using flash-chromatography (Ø = 3 cm, h = 20 cm, n-hexane:ethylacetate 1:1 + 2% N,N-dimethylethylamine, 20 mL, $R_f$ = 0.20). Colourless solid, melting point: 158 °C, Yield 107 mg (79 %).
$C_{24}H_{26}FN_3O_2$ (407.5)

| | C | H | N |
|---|---|---|---|
| Calc. | 70.7 | 6.43 | 10.3 |
| Found | 70.5 | 6.34 | 10.3 |

**MS** (ESI): m/z (rel. Int.) = 408 [$MH^+$, 100], 814 [2 x M, 13], 837 [2 x M + $Na^+$].
**IR** (neat): $\tilde{v}$ ($cm^{-1}$) = 3062 (C-H $_{aromat.}$), 2941, 2917 (C-H $_{aliphat.}$), 2814 (C-H), 1599, 1505 (C=C), 1113, 1058 (C-O).
**$^1$H-NMR** ($CDCl_3$): δ (ppm) = 1.86 - 1.98 (m, 2H, $N(CH_2C\textit{H}_2)_2$), 2.00 - 2.06 (m, 1H, $N(CH_2C\textit{H}_{2\ axial})_2$), 2.09 (td, J = 12.9/4.4 Hz, 1H, $N(CH_2C\textit{H}_{2\ axial})_2$), 2.45 (t breit, J = 11.0 Hz, 1H, $N(C\textit{H}_2CH_2)_2$), 2.56 (t breit, J = 11.0 Hz, 1H, $N(C\textit{H}_2CH_2)_2$), 2.71 - 2.81 (m, 2H, $N(C\textit{H}_2CH_2)_2$), 2.90 (dd, J = 15.3/6.7 Hz, 1 H, $C\textit{H}_2CHOCH_3$), 2.98 (dd, J = 15.3/3.5 Hz, 1 H, $C\textit{H}_2CHOCH_3$), 3.55 (s, 5H, $OC\textit{H}_3$ (3H), $NC\textit{H}_2Ph$ (2H)), 4.86 (dd, J = 6.7/3.5 Hz, 1H, $CH_2C\textit{H}OCH_3$), 7.03 (t, J = 8.6 Hz, 2H, Phenyl-C*H*), 7.30 - 7.38 (m, 3H, Phenyl-C*H*), 7.41 - 7.48 (m, 4H, Phenyl-C*H*), 7.50 (s, 1H, Pyrazole-3-C*H*).
**$^{13}$C-NMR** ($CDCl_3$): δ (ppm) = 31.2 (1C, $\textit{C}H_2CHOCH_3$), 36.7 (1C, $N(CH_2\textit{C}H_2)_2$), 39.4 (1C, $N(CH_2\textit{C}H_2)_2$), 49.3 (1C, $N(\textit{C}H_2CH_2)_2$), 49.4 (1C, $N(\textit{C}H_2CH_2)_2$), 56.9 (1C, $OCH_3$), 62.7 (1C, $N\textit{C}H_2Ph$), 71.8 (1C, Spiro-C), 77.4 (1C, Pyrazole-4-C), 96.8 (1C, $CH_2CHOCH_3$), 115.1, 115.3 (2C, Benzyl-2',6'-*C*H), 122.8 (2C, Phenyl-CH, ortho), 124.3 (1C, Benzyl-4'-*C*, quartär), 127.3 (1C, Phenyl-CH, para), 129.4 (2C, Phenyl-CH, meta), 130.9 (2C, Benzyl-3'5'-*C*H), 133.7 (1C, Phenyl-C, quartär), 134.3 (1C, Benzyl-1'-*C*, quartär), 135.8 (1C, Pyrazole-3-*C*H), 139.4 (1C, Pyrazole-5-C).

**Example H: 1-Methylpyrazole-5-carbaldehyde**

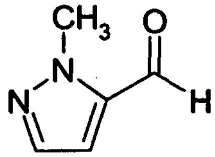

Experimental procedure:

Under $N_2$-atmosphere a Solution of n-butyllithium in hexane (1.5 M, 35.7 mL, 53.6 mmol) was added slowly to a solution of 1-Methylpyrazole (4.0 g, 48.7 mmol) in abs. THF (70 mL), which was cooled to -78 °C with a mixture of dry ice and acetone. The reaction mixture was heated to 0 °C and stirred for 30 min. Subsequently it was again cooled cooled to -78 °C with a mixture of dry ice and acetone and abs. DMF (4.5 mL, 58.5 mmol) was slowly added. This mixture was stirred for 1 h at -78 °C, subsequently heated to 0 °C, stirred for a further 2 h, heated to room temperature and finally stirred for a further 16 h. Then it was hydrolysed with water (- 20 mL) and subsequently extreacted three times with $CH_2Cl_2$. The pooled organic phases were dried ($K_2CO_3$), filtered and the solvent was removed under vacuum (30 °C, 200 mbar) upto a volume of approx. 10 mL. The crude product was purified using flash-chromatography (Ø = 8 cm, h = 18 cm, n-Hexan:Ethylacetat = 7:3, 65 mL, $R_f$ = 0.28).
Slightly yellow product, that was directly reacted further.
$C_5H_6N_2O$ (110.1)
**MS** (ESI): m/z (rel.Int) = 110 [$M^+$, 12].
**IR** (neat): $\tilde{\nu}$ ($cm^{-1}$) = 2952, 2836 (C-H $_{aliphat.}$), 1684 (C=O).
**$^1$H-NMR** ($CDCl_3$): δ (ppm) = 4.18 (s, 3H, NC*H*$_3$), 6.89 (d, J = 5.0 Hz, 1H, Pyrazole-4-C*H*), 7.52 (d, J = 5.0 Hz, 1H, Pyrazole-3-C*H*), 9.83 (s, 1H, ArC*H*O).

In the spectrum the signals of the solvent (ethylacetate) are also visible.

**Example I: 5-(2-Methoxyvinyl)-1-methylpyrazole**

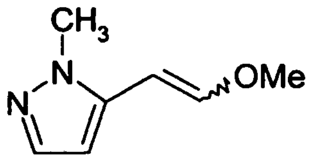

Experimental procedure:

Under $N_2$-atmosphere dry $MeOCH_2P^+Ph_3Cl^-$ (16.2 g, 47.2 mmol) was weight into and suspended in abs. THF (100 mL) for 30 minutes. Subsequently the suspension was cooled to -50 °C with a mixture of dry ice and acetone and subsequently a solution of KO$^t$Bu in THF (1M, 54.5 mL, 54.5 mmol) was added slowly. After a further 15 minutes of stirring, the aldehyde of Example H (4 g, 36.3 mmol) was dissolved in abs. THF (30 mL) and added dropwise at -50 °C. The reaction mixture was slowly brought to room temperature over night while stirring. After addition of water (- 40 mL) it was extracted 3 times with $CH_2Cl_2$. The pooled organic phases were dried over $K_2CO_3$, filtered and the solvent removed under vacuum (35 °C, 150 mbar) upto a volume of approx. 15 mL. The crude product was purified using flash-chromatography (Ø = 8 cm, h = 18 cm, n-hexane:ethylacetate = 5:5, 65 mL, $R_f$ = 0.20).
Slightly yellow product, that was directly reacted further.
$C_7H_{10}N_2O$ (138.2)
**MS** (ESI): m/z (rel.Int) = 139 [$MH^+$, 22], 294 [2 x M + $NH_4^+$, 100].
**IR** (neat): $\tilde{\nu}$ ($cm^{-1}$) = 2940 (C-H$_{aliphat.}$), 1644 (C=C $_{Alken}$), 1100 (C-O).
**$^1$H-NMR** ($CDCl_3$): δ (ppm) = 3.70 (s, 3H, OC*H*$_3$, trans-Isomer), 3.79, 3.80, 3.81 (je s, 9H, $NCH_3$, cis-Isomer + trans-Isomer (6H), OC*H*$_3$ cis-Isomer (3H)), 5.23 (d, J = 6.7 Hz, 1H, C*H*=$CHOCH_3$, cis-Isomer), 5.60 (d, J = 12.9 Hz, 1H, C*H*=$CHOCH_3$, trans-Isomer), 6.11 (d, J = 1.6 Hz, 1 H, Pyrazole-4-CH, trans-Isomer), 6.20 (d, J = 6.7 Hz, 1H, CH=C*H*O$CH_3$,

cis-Isomer), 6.55 (d, J = 1.6 Hz, 1H, Pyrazole-4-CH, cis-Isomer), 6.96 (d, J = 12.9 Hz, 1 H, CH=C*H*$OCH_3$, trans-Isomer), 7.36 (d, J = 1.6 Hz, 1 H, Pyrazole-3-CH, trans-Isomer), 7.41 (d, J = 1.2 Hz, 1H, Pyrazole-3-C*H*, cis-Isomer).

In the spectrum the signals of the solvent (ethylacetate) are also visible. The ratio of the both isomers cis/trans is 2:3.

**Example J: 2-(4-Brom-1-methylpyrazole-5-yl)acetaldehyde-dimethylacetal**

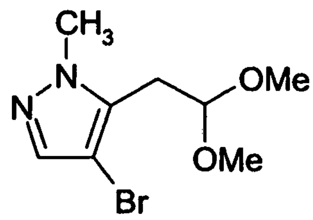

Experimental procedure:

To a solution of Example I (3.0 g, 21.7 mmol) in abs. MeOH (50 mL) was added p-toluolsulphonic acid monohydrate (4.1 g, 21.7 mmol) and heated for 115 h under reflux. Subsequently $HC(OCH_3)_3$ (3.6 mL, 32.6 mmol) was added and cooled to 0 °C. After adding PBB (6.9 g, 21.7 mmol) it was stirred for 1 h at 0 °C and then a further 18 h at room temperature. Subsequently following addition of 2N NaOH (- 20 mL) and $H_2O$ (~ 15 mL) it was extracted 3 times with $CH_2Cl_2$. The pooled organic phases were dried over $K_2CO_3$, filtered and the solvent removed under lowered pressure. The crude product received was purified using flash-chromatography. (Ø = 8 cm, h = 18 cm, n-hexane:ethylacetate = 7:3, 85 mL, $R_f$ = 0.22).

Colourless solid, melting point: 29 °C, Yield 3.5 g (29% in 4 steps, related to 4.0 g 1-Methylpyrazole).

$C_8H_{13}BrN_2O_2$ (249.1)

**MS** (ESI): m/z (rel.Int) = 249 [$^{79}$Br-MH$^+$, 19], 251 [$^{81}$Br-MH$^+$, 18], 271 [$^{79}$Br-M + Na$^+$, 97], 273 [$^{81}$Br-M + Na$^+$, 100], 520 [$^{79}$Br-2x MH + Na$^+$, 86], 522 [$^{81}$Br-2x MH + Na$^+$, 43].

**IR** (neat): $\tilde{v}$ (cm$^{-1}$ = 2936 (C-H $_{aliphat.}$), 2832 (C-H), 1118, 1070 (C-O).

**$^1$H-NMR** ($CDCl_3$): δ (ppm) = 2.96 (d, J = 5.5 Hz, 2H, C*H*$_2$CH($OCH_3$)$_2$), 3.37 (s, 6H, (OC*H*$_3$)$_2$), 3.86 (s, 3H, NC*H*$_3$), 4.45 (t, J = 5.5 Hz, 1H, $CH_2$C*H*($OCH_3$)$_2$), 7.40 (s, 1H, Pyrazole-3-C*H*).

**Example K: 2-[4-(1-Benzyl-4-hydroxypiperidin-4-yl)-1-methylpyrazole-5-yl]acetaldehyddimethylacetal**

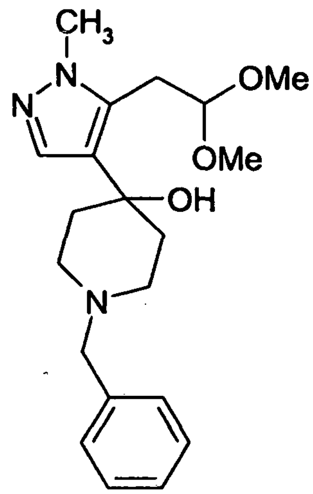

Experimental procedure:

Under $N_2$-atmosphere n-butyl lithium in n-hexane (1.5 M , 5.9 mL, 8.83 mmol) was slowly added at -78 °C to

a solution of the bromated Example J (2.0 g, 8.03 mmol) in abs THF (25 mL). The reaction mixture was stirred for 15 Minuten at -78 °C. Subsequently 1-Benzyl-piperidin-4-one (1.7 mL, 9.64 mmol) in abs. THF (4 mL) was added dropwise at -78 °C. After stirring for 4.5 h at -78 °C the mixture was heated to room temperature, stirred for a further 17 h stirred and subsequently diluted with water (~ 12 mL) until no further precipitate is forming. Following extraction with $CH_2Cl_2$ the pooled organic phases were dried over $K_2CO_3$ and filtered. The solvent was removed under vacuum and the crude product (3.6 g) purified using flash-chromatography (Ø = 8 cm, h = 15 cm, ethylacetate:n-hexane = 7:3 + 2% N,N-dimethylethylamine, 65 mL, $R_f$ = 0.06).
Slightly yellow oil, that crystallises after storage in the fridge to a slightly yellow solid,
melting point: 81 °C, Yield 1.93 g (67%).
$C_{20}H_{29}N_3O_3$ (359.5)
**MS** (ESI): m/z (rel.Int) = 360 [$MH^+$, 100].
**IR** (neat): $\tilde{v}$ ($cm^{-1}$) = 3404 (O-H), 3060, 3027 (C-$H_{aromat.}$), 2938, 2829 (C-H), 1117, 1064 (C-O).
**$^1$H-NMR** ($CDCl_3$): δ (ppm) = 1.69 - 1.76 (m, 2H, $N(CH_2CH_2)_2$), 1.95 - 2.04 (m, 2H, $N(CH_2CH_2)_2$), 2.35 - 2.48 (m, 2H, $N(CH_2CH_2)_2$), 2.61 - 2.72 (m, 2H, $N(CH_2CH_2)_2$), 3.14 (d, J = 5.6 Hz, 2H, $CH_2CH(OCH_3)_2$), 3.18 (s breit, 1 H, OH), 3.30 (s, 6H, $CH(OCH_3)_2$), 3.51 (s breit, 2H, $NCH_2$Phenyl), 3.74 (s, 3H, $NCH_3$), 4.42 (t, J = 5.6 Hz, 1 H, $CH_2CH(OCH_3)_2$), 7.23 - 7.31 (m, 6H, Phenyl-CH (5H), Pyrazole-3-CH (1 H)).

**<u>Example 22: 1-Benzyl-6'-methoxy-1'-methyl-6',7'-dihydro-1'H-spiro[piperidin-4,4'-pyrano[4,3-c]pyrazole]</u>**

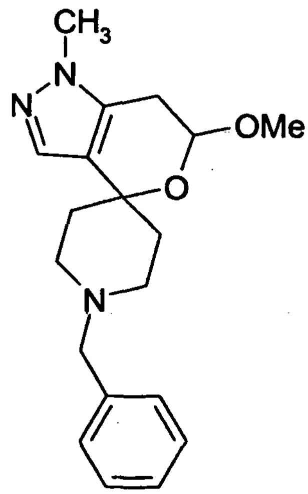

Experimental procedure:

To a solution Example K (1.0 g, 2.8 mmol) in abs. methanol (30 mL) p-toluol sulphonic acid monohydrate (5.3 g, 27.8 mmol) was added and stirred at room temperature. After stirring for 24 h it was neutralised with a solution of NaOH (2 M), diluted with water and extracted 3 times with $CH_2Cl_2$. The pooled organic phases were dried over $K_2CO_3$, filtered and the solvent removed under lowered pressure. The crude product (1.3 g) was purified using flash-chromatography (Ø = 8 cm, h = 18 cm, n-hexane:ethylacetate 1:1 + 2% N,N-dimethylethylamine, 65 mL, $R_f$ = 0.26 (EE + 2% N,N-dimethylethylamine)).
Colourless solid, melting point: 112 °C, Yield 374 mg (41 %).
$C_{19}H_{25}N_3O_2$ (327.5)

| | C | H | N |
|---|---|---|---|
| Calc. | 69.7 | 7.70 | 12.8 |
| Found | 69.4 | 7.70 | 13.0 |

MS (ESI): m/z (rel.Int) = 328 [$MH^+$, 100].
IR (neat): $\tilde{v}$ ($cm^{-1}$) = 3025 (C-$H_{aromat.}$), 2938, 2809 (C-$H_{aliphat.}$), 1576 (C=C), 1116, 1059, 1036 (C-O).
$^1$H-NMR ($CDCl_3$): δ (ppm) = 1.71 - 1.84 (m, 2H, $N(CH_2CH_2)_2$), 1.85 - 2.03 (m, 2H, $N(CH_2CH_2)_2$), 2.36 (td, J = 11.7/3.0

Hz, 1H, $N(CH_2CH_2)_2$), 2.46 (td, J = 11.7/2.9 Hz, 1H, $N(CH_2CH_2)_2$), 2.57 (dd, J = 15.3/7.2 Hz, 1 H, $CH_2CHOCH_3$), 2.62 - 2.72 (m, 2H, $N(CH_2CH_2)_2$), 2.77 (dd, J = 15.3/3.3 Hz, 1H, $CH_2CHOCH_3$), 3.49 (s, 3H, $CH_2CHOCH_3$), 3.67 (d, J = 13.2 Hz, 1H, $NCH_2$Phenyl), 3.70 (d, J = 13.2 Hz, 1H, $NCH_2$Phenyl), 3.66 (s, 3H, $NCH_3$), 4.82 (dd, J = 7.2/3.6 Hz, 1H, $CH_2CHOCH_3$), 7.20-7.31 (m, 6H, Phenyl-CH (5H), Pyrazole-3-CH (1 H)).

**Example 23: 6'-Methoxy-1'-methyl-6',7'-dihydro-1'H-spiro[piperidin-4,4'-pyrano[4,3-c]pyrazole]**

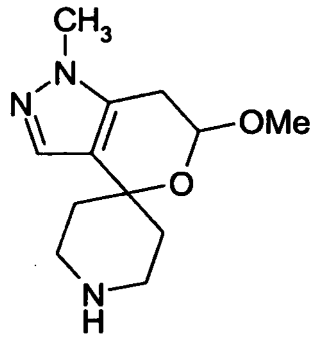

Experimental procedure:

To a solution of Example 22 (360 mg, 1.09 mmol) in abs. methanol (10 mL) were added consecutively dried ammoniumformiate (347 mg, 5.50 mmol) and 10% Pd/C (28 mg) each in one dose respectively. Following that it was stirred for 25 minutes under reflux and subsequently the catalysator filtered over a fluted filter. After thorough rinsing with methanol the solvent was removed under vacuum. The crude product (208 mg) was redissolved in acetonitrile and following a further removal of the solvent under vacuum a yellow oil is formed, which is used in following reactions without further purification.
Yellow Oil, Yield 208 mg (96%).
$C_{12}H_{19}N_3O_2$ (237.3)
MS (ESI): m/z (rel.Int) = 238 [$MH^+$, 100].
IR (neat): $\tilde{\nu}$ ($cm^{-1}$) = 3308 (N-H), 2956, 2911 (C-H $_{aliphat.}$), 1061 (C-O).
$^1$H-NMR ($CDCl_3$): $\delta$ (ppm) = 1.89 - 2.02 (m, 2H, $N(CH_2CH_2)_2$), 2.03 - 2.29 (m, 2H, $N(CH_2CH_2)_2$), 2.64 (dd, J = 15.5/6.0 Hz, 1 H, $CH_2CHOCH_3$), 2.81 (dd, J = 15.6/3.7 Hz, 1 H, $CH_2CHOCH_3$), 3.19 - 3.36 (m, 4H, $N(CH_2CH_2)_2$), 3.47 (s, 3H, $OCH_3$), 3.69 (s, 3H, $NCH_3$), 4.86 (dd, J = 6.0/3.8 Hz, 1 H, $CH_2CHOCH_3$), 7.27 (s, 1H, Pyrazole-3-CH).

**Example 24: 6'-Methoxy-1'-methyl-1-(3-phenylpropyl)-6',7'-dihydro-1'H-spiro[piperidin-4,4'-pyrano[4,3-c]pyrazole]**

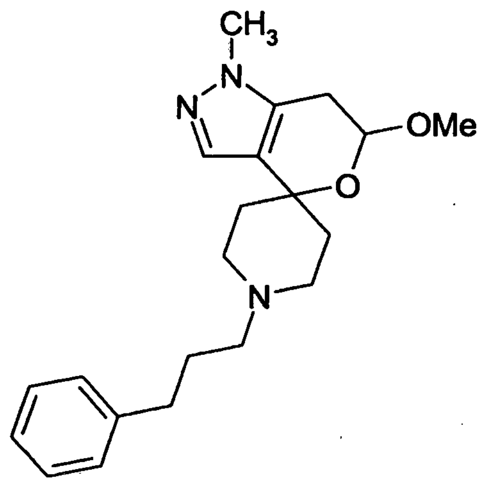

Experimental procedure:

To a solution of crude example 23 (64 mg, 0.27 mmol) in acetonitrile (5 mL) 1-Bromo-3-phenylpropane (48.9 μL, 0.32 mmol) and $K_2CO_3$ (298.2 mg, 2.16 mmol) were added. This mixture was heated for 21 h under reflux. Subsequently it was filtered and the solvent removed under vacuum. The crude product (106 mg) was purified using flash-chromatography (Ø = 2 cm, h = 18 cm, n-hexane:ethylacetate 3:7 + 2% N,N-dimethylethylamine, 10 mL, $R_f$ = 0.13).

Colourless solid, melting point: 115 °C, Yeld 74 mg (77%).

$C_{21}H_{29}N_3O_2$ (355.5)

| | C | H | N |
|---|---|---|---|
| Calc. | 71.0 | 8.22 | 11.8 |
| Found | 70.5 | 8.15 | 11.6 |

MS (ESI): m/z (rel.Int) = 356 [$MH^+$, 100], 378 [M + $Na^+$, 26].

IR (neat): $\tilde{v}$ ($cm^{-1}$) = 3025 (C-H $_{aromat.}$), 2940 (C-H $_{aliphat.}$), 1116, 1059 (C-O).

$^1$H-NMR ($CDCl_3$): δ (ppm) = 1.73 - 1.86 (m, 4H, $N(CH_2CH_2)_2$ (2H), $NCH_2CH_2CH_2Ph$ (2H)), 1.88 - 2.04 (m, 2H, $N(CH_2CH_2)_2$), 2.28 - 2.47 (m, 4H, $N(CH_2CH_2)_2$ (2H), $NCH_2CH_2CH_2Ph$ (2H)), 2.54 - 2.64 (m, 3H, $NCH_2CH_2CH_2Ph$ (2H), $CH_2CHOCH_3$ (1H)), 2.68 - 2.76 (m, 2H, $N(CH_2CH_2)_2$), 2.78 (dd, J = 15.3/3.3 Hz, 1H, $CH_2CHOCH_3$), 3.50 (s, 3H, $OCH_3$), 3.69 (s, 3H, $NCH_3$), 4.82 (dd, J = 7.2/3.6 Hz, 1H, $CH_2CHOCH_3$), 7.09 - 7.26 (m, 6H, Phenyl-CH (5H), Pyrazole-3-CH (1H)).

**Example 25: 1-(4-Fluorbenzyl)-6'-methoxy-1'-methyl-6'7'-dihydro-1'H-spiro[piperidin-4,4'-pyrano[4,3-c]pyrazole]**

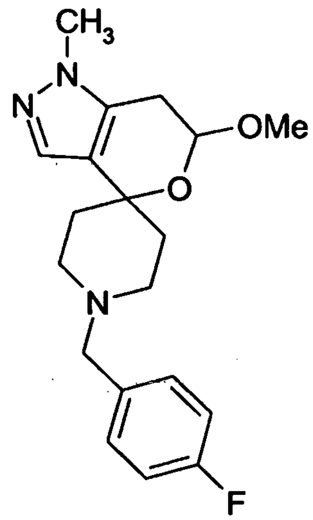

Experimental procedure:

To a solution of crude example 23 (90 mg, 0.38 mmol) in abs. dichloroethane (2 mL) were added cosecutively freshly distilled p-fluorobenzaldehyde (40.9 μL, 0.38 mmol) and sodiumtriacetoxyborhydride (120.6 mg, 0.57 mmol). This reaction mixture was stirred for 19 h at room temperature. After addition of a saturated solution of $NaHCO_3$ (- 6 mL) and water (- 8 mL), it was extracted 3 times with $CH_2Cl_2$. Subsequently the pooled organic phases were dried over $K_2CO_3$ and filtered. Following removal of the solvent under vacuum the crude product (136 mg) was purified using flash-chromatography (Ø = 3.5 cm, h = 15 cm, n-hexane:ethylacetate 1:1 + 2% N,N-dimethylethylamine, 20 mL, $R_f$ = 0.11). Colourless solid, melting point: 127 °C, Yield 87 mg (66 %).

$C_{19}H_{24}FN_3O_2$ (345.5)

| | C | H | N |
|---|---|---|---|
| Calc. | 66.1 | 7.00 | 12.2 |
| Found | | | |

MS (ESI): m/z (rel.Int) = 346 [$MH^+$, 100].
IR (neat): $\tilde{v}$ ($cm^{-1}$) = 2941 (C-H $_{aliphat.}$), 1602, 1507 (C=C), 1115, 1059 (C-O).
$^1$H-NMR ($CDCl_3$): δ (ppm) = 1.70 - 1.82 (m, 2H, $N(CH_2CH_2)_2$), 1.85 - 2.02 (m, 2H, $N(CH_2CH_2)_2$), 2.35 (td, J = 11.5/2.8 Hz, 1H, $N(CH_2CH_2)_2$), 2.45 (td, J = 11.7/2.9 Hz, 1 H, $N(CH_2CH_2)_2$), 2.57 (dd, J = 15.4/7.1 Hz, 1H, $CH_2CHOCH_3$), 2.62 - 2.71 (m, 2H, $N(CH_2CH_2)_2$), 2.76 (dd, J = 15.4/3.5 Hz, 1H, $CH_2CHOCH_3$), 3.47 (s, 2H, $NCH_2Ph$), 3.49 (s, 3H, $OCH_3$), 3.66 (s, 3H, $OCH_3$), 4.81 (dd, J = 7.2/3.6 Hz, 1H, $CH_2CHOCH_3$), 6.94 (d, J = 8.7 Hz, 2H, Phenyl-CH), 7.21 - 7.29 (m, 3H, Phenyl-CH (2H), Pyrazole-3-CH (1H)).

**Example 26: 1-Isopentyl-6'-methoxy-1'-methyl-6',7'-dihydro-1'H-spiro[piperidin-4,4-pyrano[4,3-c]pyrazole]**

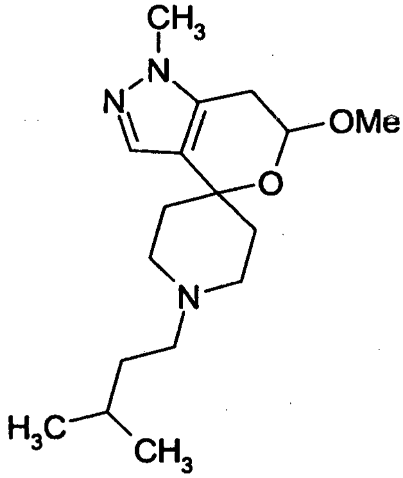

Experimental procedure:

To a solution of crude example 23 (73 mg, 0.31 mmol) in abs. dichloroethane (3 mL) were added consecutively freshly distilled Isovaleraldehyde (32.8 μL, 0.31 mmol) and Sodiumtriacetoxyborhydride (97.1 mg, 0.46 mmol). This reaction mixture was stirred for 19 h at room temperature. After adding of a saturated solution of $NaHCO_3$ (- 6 mL) and water (~ 5 mL), it was extracted 3 times with $CH_2Cl_2$. Subsequently the pooled organic phases were dried over $K_2CO_3$ and filtered. Following removal of the solvent under vacuum the crude product (114 mg), was prurified using flash-chromatography (Ø = 3.5 cm, h = 15 cm, n-hexane:ethylacetate 3:7 + 2% N,N-dimethylethylamine, 20 mL, $R_f$ = 0.08). Colourless solid, melting point: 85 °C, Yield 74 mg (79%).
$C_{17}H_{29}N_3O_2$ (307.5)

| | C | H | N |
|---|---|---|---|
| Calc. | 66.4 | 9.51 | 13.7 |
| Found | 66.1 | 9.52 | 13.3 |

**MS** (ESI): m/z (rel.Int) = 308 [$MH^+$, 100].
**IR** (neat): $\tilde{v}$ ($cm^{-1}$) = 2951 (C-H $_{aliphat.}$), 1060 (C-O).
**$^1$H-NMR** ($CDCl_3$): δ (ppm) = 0.77 (d, J = 6.6 Hz, 6H, $NCH_2CH_2CH(CH_3)_2$), 1.25 -1.32 (m, 2H, $NCH_2CH_2CH(CH_3)_2$), 1.40 - 1.51 (m, 1H, $NCH_2CH_2CH(CH_3)_2$), 1.66 - 1.75 (m, 2H, $N(CH_2CH_2)_2$), 1.80 - 1.91 (m, 2H, $N(CH_2CH_2)_2$), 2.20 - 2.30 (m, 3H, $N(CH_2CH_2)_2$) (1H), $NCH_2CH_2CH(CH_3)_2$) (2H)), 2.32 (td, J = 11.7/2.5 Hz, 1H, $N(CH_2CH_2)_2$), 2.50 (dd, J = 15.3/7.1 Hz, 1H, $CH_2CHOCH_3$), 2.59 - 2.66 (m, 2H, $N(CH_2CH_2)_2$), 2.68 (dd, J = 15.3/3.6 Hz, 1H, $CH_2CHOCH_3$), 3.42 (s, 3H,

$OCH_3$), 3.58 (s, 3H, $NCH_3$), 4.74 (dd, J = 7.1/3.6 Hz, 1H, $CH_2CHOCH_3$), 7.12 (s, 1H, Pyrazole-3-CH).

**Example 27: 6'-Methoxy-1'-methyl-1-propyl-6',7'-dihydro-1'H-spiro[piperidin-4,4'-pyrano[4,3-c]pyrazole]**

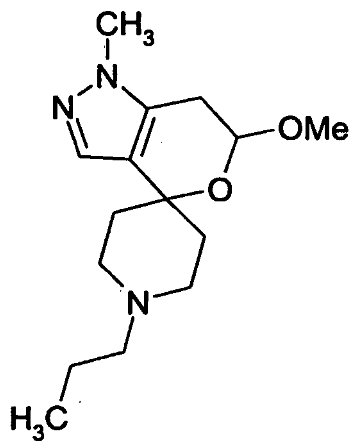

Experimental procedure:

To a solution of crude example 23 (90 mg, 0.38 mmol) in acetonitrile (5 mL) 1-bromopropane (44.8 µL, 0.49 mmol) and $K_2CO_3$ (419.3 mg, 3.03 mmol) were added. This mixture was heated for 47 h under reflux. Subsequently the $K_2CO_3$ was filtered off and the solvent removed under vacuum. The crude product (86 mg) was purified using flash-chromatography (Ø = 2.5 cm, h = 15 cm, n-hexane:ethylacetate 3:7 + 2% N,N-dimethylethylamine, 10 mL, $R_f$ = 0.11). Colourless solid, melting point: 94 °C, Yield 63 mg (60 %).
$C_{15}H_{25}N_3O_2$ (279.4)

| | C | H | N |
|---|---|---|---|
| Calc. | 64.5 | 9.02 | 15.0 |
| Found | 64.2 | 8.95 | 14.6 |

MS (ESI): m/z (rel.Int) = 280 [$MH^+$, 100].
**IR** (neat): $\tilde{v}$ ($cm^{-1}$) = 2948 (C-H $_{aliphat.}$), 2873 (C-H), 1122, 1057 (C-O).
$^1$H-NMR ($CDCl_3$): 6 (ppm) = 0.86 (t, J = 7.3 Hz, 3H, $NCH_2CH_2CH_3$), 1.42 - 1.56 (m, 2H, $NCH_2CH_2CH_3$), 1.73 - 1.85 (m, 2H, $N(CH_2CH_2)_2$), 1.88 - 2.04 (m, 2H, $N(CH_2CH_2)_2$), 2.26 - 2.34 (m, 3H, $N(CH_2CH_2)_2$) (1H), $NCH_2CH_2CH_3$) (2H)), 2.40 (td, J = 12.0/2.7 Hz, 1H, $N(CH_2CH_2)_2$), 2.58 (dd, J = 15.5/7.2 Hz, 1H, $CH_2CHOCH_3$), 2.66-2.74 (m, 2H, $N(CH_2CH_2)_2$), 2.77 (dd, J = 15.5/3.6 Hz, 1 H, $CH_2CHOCH_3$), 3.51 (s, 3H, $OCH_3$), 3.67 (s, 3H, $NCH_3$), 4.82 (dd, J = 7.2/3.3 Hz, 1H, $CH_2CHOCH_3$), 7.19 (s, 1H, Pyrazole-3-CH).

**Example 28: 1-Benzyl-1'-methyl-6',7'-dihydro-1'H-spiro[piperidin-4,4'-pyrano[4,3-c]pyrazole]-6'-ol**

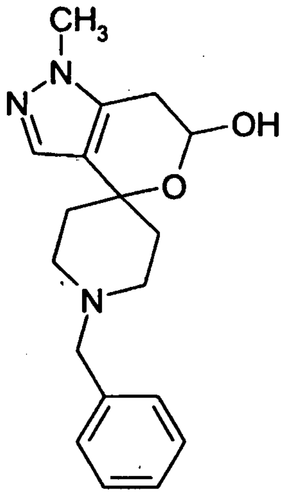

Experimental procedure:

A solution of Example K (1.0 g, 2.78 mmol) in aqueous HCl (2 N, 60 mL) was stirred for 47 h at room temperature. Subsequently it was neutralised with - 60 mL of a solution of NaOH (2 N), diluted with water and extracted 3 times with $CH_2Cl_2$. The pooled organic phases were dried over $K_2CO_3$, filtered and the solvent removed under lowered pressure. The crude product (1.01 g) was purified using flash-chromatography (Ø = 6 cm, h = 15 cm, ethylacetate + 2% N,N-dimethylethylamine, 65 mL, $R_f$= 0.08).
Colourless solid, melting point: 165 °C, Yield 673 mg (77 %).
$C_{18}H_{23}N_3O_2$ (313.4)

| | C | H | N |
|---|---|---|---|
| Calc. | 69.0 | 7.40 | 13.4 |
| Found | 68.7 | 7.36 | 12.9 |

MS (ESI): m/z (rel.Int) = 313 [$M^+$, 18], 312 [$(M - H)^+$, 100].
**IR** (neat): $\tilde{v}$ ($cm^{-1}$) = 3072 (O-H), 2921 (C-H $_{aliphat.}$), 2835 (C-H), 1109, 1043 (C-O).
$^1$H-NMR ($CDCl_3$): δ (ppm) = 1.70 - 1.86 (m, 2H, $N(CH_2CH_2)_2$), 1.87 - 2.03 (m, 2H, $N(CH_2CH_2)_2$), 2.36 (td, J = 11.4/3.1 Hz, 1 H, $N(CH_2CH_2)_2$), 2.45 (td, J = 11.6/2.9 Hz, 1H, $N(CH_2CH_2)_2$), 2.55 (dd, J = 15.3/7.2 Hz, 1 H, $CH_2CHOCH_3$), 2.59 - 2.70 (m, 2H, $N(CH_2CH_2)_2$), 2.83 (dd, J = 15.3/3.3 Hz, 1H, $CH_2CHOCH_3$), 3.61 (d, J = 14.1 Hz, 1H, $NCH_2Ph$), 2.64 (d, J = 14.3 Hz, 1H, $NCH_2Ph$), 3.68 (s, 3H, $NCH_3$), 5.27 (dd, J = 7.3/3.5 Hz, 1 H, $CH_2CHOCH_3$), 7.17 - 7.30 (m, 6H, Phenyl-CH (5H), Pyrazole-3-CH (1H)).

**Example 29: 1-Benzyl-1'-methyl-1'H-spiro[piperidin-4,4'-pyrano[4,3-c]pyrazole]**

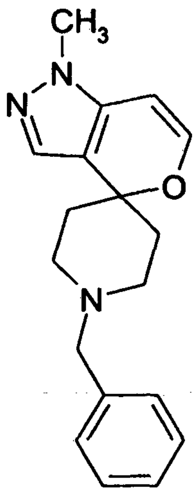

Experimental procedure:

Example 28 (400 mg, 1.28 mmol) was dissolved in abs. $CH_2Cl_2$ (18 mL) under $N_2$-atmosphere. Subsequently it was cooled to 0 °C and consecutively $NEt_3$ (425.7 µL, 3.06 mmol) and $MeSO_2Cl$ (118.9 µL, 1.53 mmol) were slowly added. The reaction solution was stirred for 2 h at room temperature and subsequently for 1 h under reflux, before being neutralised with a saturated solution of $NaHCO_3$ (~ 16 mL) and extracted for 3 times with $CH_2Cl_2$. The pooled organic phases were dried over $K_2CO_3$, filtered and the solvent removed in vacuum. The crude product (415 mg) was purified using flash-chromatography (0 = 4 cm, h = 15 cm, n-hexane:ethylacetate 3:7 + 2% N,N-dimethylethylamine, 30 mL, $R_f$ = 0.21).
Colourless solid, melting point: 115 °C, Yield 296 mg (79%).
$C_{18}H_{21}N_3O$ (295.4)

| | C | H | N |
|---|---|---|---|
| Calc. | 73.2 | 7.17 | 14.2 |
| Found | 73.0 | 6.91 | 14.1 |

MS (ESI): m/z (rel.Int) = 296 [$MH^+$, 100].
**IR** (neat): $\tilde{v}$ ($cm^{-1}$) = 3091, 3006 (C-H $_{aromat.}$), 2961, 2939, 2916 (C-H $_{aliphat.}$), 1611 (C=C), 1045 (C-O).
**$^1$H-NMR** ($CDCl_3$): δ (ppm) = 1.83 (t, J = 11.2 Hz, 2H, $N(CH_2C\mathit{H_2})_2$), 2.02 - 2.11 (m, 2H, $N(CH_2C\mathit{H_2})_2$), 2.37 (t, J = 11.2 Hz, 2H, $N(C\mathit{H_2}CH_2)_2$), 2.57 - 2.66 (m, 2H, $N(C\mathit{H_2}CH_2)_2$), 3.50 (s, 2H, $NC\mathit{H_2}Ph$), 3.67 (s, 3H, $NC\mathit{H_3}$), 5.56 (dd, J = 6.0/0.7 Hz, 1 H, ArCH=CH), 6.40 (d, J = 6.0 Hz, 1H, ArCH=C*H*), 7.08 (d, J = 0.7 Hz, 1 H, Pyrazole-3-C*H*), 7.17 - 7.31 (m, 5H, Phenyl-C*H*).

**Example 30: 1-Benzyl-1'-methyl-6',7'-dihydro-1'H-spiro[piperidin-4,4'-pyrano[4,3-c]pyrazole]**

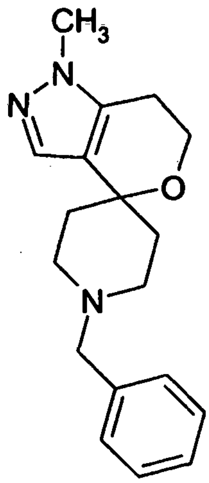

Experimental procedure:

50 mg (0.17 mmol) of Example 29 were dissolved in glacial acetic acid (5 mL) and 10 % Pd/C (35 mg) added. It was stirred for 1h at room temperature under $H_2$-atmosphere (Ballon), filtered through a fluted filter and thoroughly rinsed with HCl (2N) and $H_2O$. Subsequently the filtrate was strongly alkalized with a solution of NaOH (2N) and extracted with $CH_2Cl_2$. The pooled organic phases were dried over $K_2CO_3$, filtered and the solvent removed under vacuum abdestilliert. The crude product (52 mg) was purified using flash-chromatography (Ø = 2 cm, h = 15 cm, n-hexane:ethylacetate 1:1 + 2% N,N-dimethylethylamine, 10 mL, $R_f$ = 0.09).
Colourless oil, Yield 37 mg (74 %).
$C_{18}H_{23}N_3O$ (297.4)

| | C | H | N |
|---|---|---|---|
| Calc. | 72.7 | 7.80 | 14.1 |
| Found | 72.4 | 7.88 | 13.9 |

**MS** (ESI): m/z (rel.Int) = 298 [$MH^+$, 100].
**IR** (neat): $\tilde{v}$ ($cm^{-1}$) = 3026 (C-H $_{aromat.}$), 2936 (C-H $_{aliphat.}$), 2810 (C-H). 1577, 1508 (C=C), 1070 (C-O).
**$^1$H-NMR** ($CDCl_3$): $\delta$ (ppm) = 1.81 - 1.96 (m, 4H, $N(CH_2CH_2)_2$), 2.41 (td, J = 11.5/3.1 Hz, 2H, $N(CH_2CH_2)_2$), 2.62 (t, J = 5.7 Hz, 2H, $CH_2CH_2O$), 2.69 (dt, J = 11.7/3.9 Hz, 2H, $N(CH_2CH_2)_2$), 3.57 (s, 2H, $NCH_2Ph$), 3.74 (s, 3H, $NCH_3$), 3.91 (t, J = 5.7 Hz, 2H, $CH_2CH_2O$), 7.23 - 7.28 (m, 1H, Phenyl-CH, para), 7.29 - 7.37 (m, 5H, Phenyl-CH (4H), Pyrazole-3-CH (1H)).

**Example 31: 1'-Methyl-6',7'-dihydro-1'H-spiro[piperidin-4,4'-pyrano[4,3-c]pyrazole]**

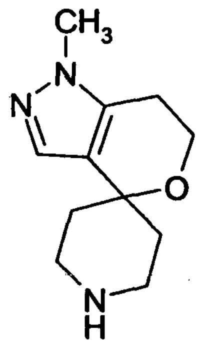

Experimental procedure:

To a solution of Example 30 (345 mg, 1.16 mmol) in abs. methanol (15 mL) were added consecutively dried ammoniumformiate (365.8 mg, 5.80 mmol) and 10% Pd/C (27.6 mg) each in one dose respectively. Subsequently it was stirred for 25 minutes under reflux and following that the catalysator filtered off through a fluted filter. After thorough rinsing with methanol the solvent was removed under vacuum. The crude product (colourless solid) was subsequently redissolved in $H_2O$ and extracted with $CH_2Cl_2$ : MeOH 2:1. After drying the poled organic phases over $K_2CO_3$ the solvent was again removed under vacuum. A yellow oil was formed, which was used in following reactions without further purification.

Yellow oil, $R_f$ = 0.09 (MeOH + 2 % $NH_3$ conc.), Yield 216 mg (90 %).

$C_{11}H_{17}N_3O$ (207.3)

**MS** (ESI): m/z (rel.Int) = 208 [$MH^+$, 100].

**IR** (neat): $\tilde{\nu}$ ($cm^{-1}$) = 2967 (C-H $_{aliphat.}$), 1510 (N-H), 1066 (C-O).

**$^1$H-NMR** ($CDCl_3$): δ (ppm) = 1.75 - 1.88 (m, 4H, $N(CH_2CH_2)_2$), 2.63 (t, J = 5.5 Hz, 2H, $ArCH_2CH_2O$), 2.92 (dt, J = 11.7/3.9 Hz, 2H, $N(CH_2CH_2)_2$), 3.04 (td, J = 11.7/3.9 Hz, 2H, $N(CH_2CH_2)_2$), 3.75 (s, 3H, $NCH_3$), 3.92 (t, J = 5.5 Hz, 2H, $ArCH_2CH_2O$), 7.28 (s, 1H, Pyrazole-3-C*H*).

In addition more compounds according to the invention are synthesized using the method of Reaction Scheme B in an analoguous way as in examples 7 to 21 using methods known in the art as also exemplified in the parallel cases of examples 28, 29, 30 and 31 (above). This reaction and the resulting compounds (examples 35, 36 and 37) are described in Scheme E:

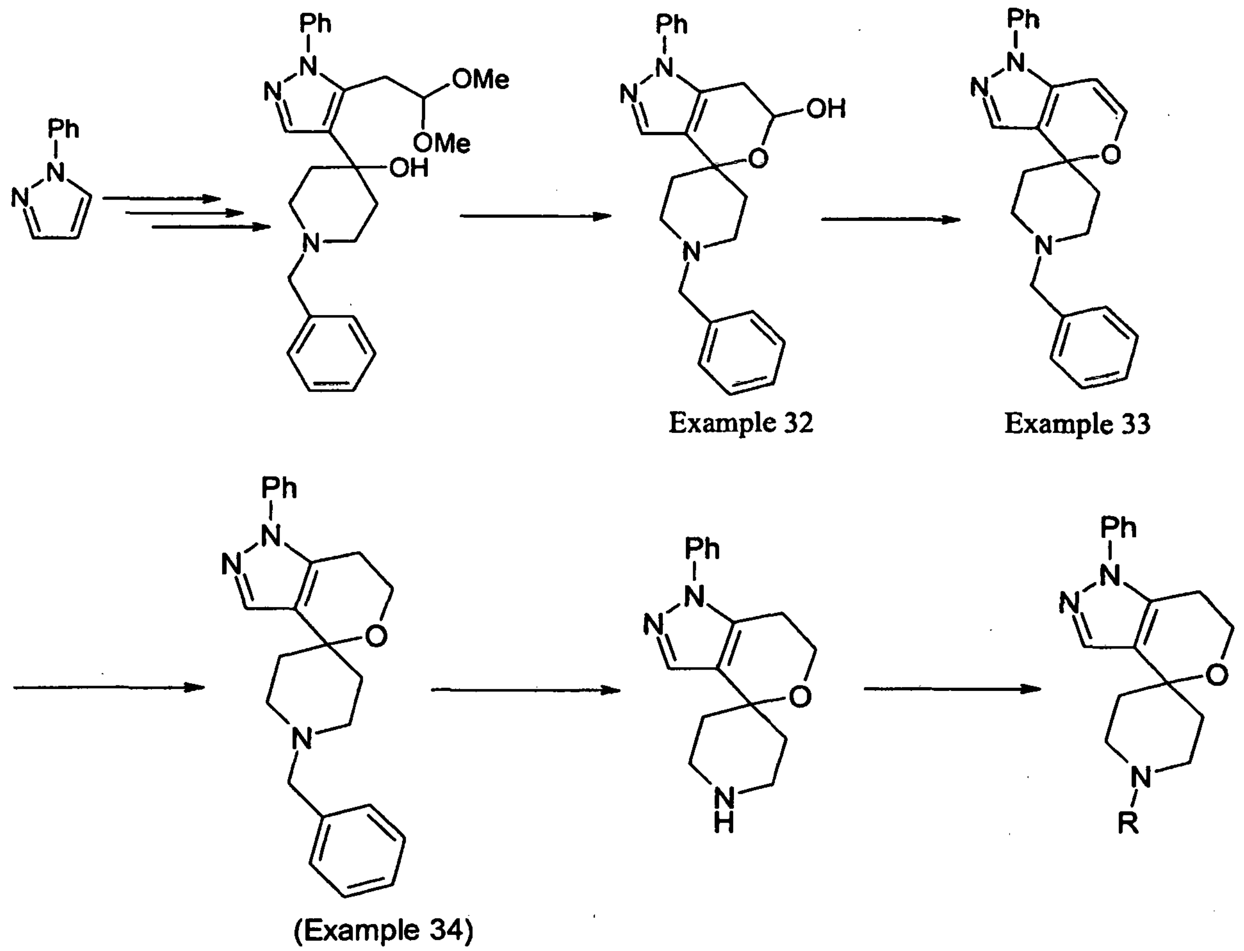

Example 32

Example 33

(Example 34)

with R being here either:

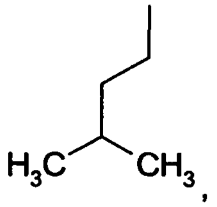
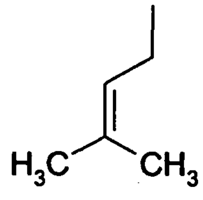
or 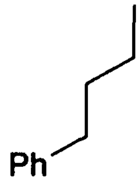.

(Example 35), (Example 36) or (Example 37).

Also Example 38 (1-(Cyclohexan-1-ylmethyl)-1'-phenyl-6',7'-dihydro-1'H-spiro[piperidin-4,4'-pyrano[4,3-c]pyrazole])

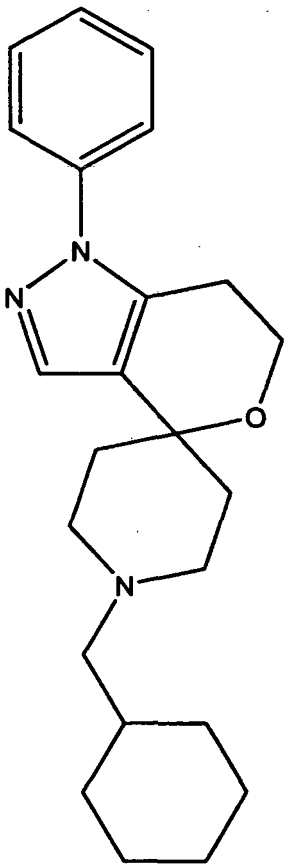

may be synthesized following Reaction Scheme E (above) as described for Example 35 (1-Isopentyl-1'-phenyl-6',7'-dihydro-1'H-spiro[piperidin-4,4'-pyrano[4,3-c]pyrazole]), Example 36 (1-(Methylbut-2-en-1-yl)-1'-phenyl-6',7'-dihydro-1'H-spiro[piperidin-4,4'-pyrano[4,3-c]pyrazole]) and Example 37 (1-Phenylpropyl-1'-phenyl-6',7'-dihydro-1'H-spiro[piperidin-4,4'-pyrano[4,3-c]pyrazole]). Besides that, the Reaction Scheme E also shows the synthesis of Example 32 (1-Benzyl-1'-phenyl-6',7'-dihydro-1'H-spiro[piperidin-4,4'-pyrano[4,3-c]pyrazole]-6'-ol), Example 33 (1-Benzyl-1'-phenyl-1'H-spiro[piperidin-4,4'-pyrano[4,3-c]pyrazole]) and Example 34 (1-Benzyl-1'-phenyl-6',7'-dihydro-1'H-spiro[piperidin-4,4'-pyrano[4,3-c]pyrazole]) as indicated in the Scheme which also serve as intermediates for the further synthesis of other compounds according to the invention like examples 35 to 38.

In addition more compounds according to the invention are synthesized using the method of Reaction Schemes C and D in an analoguous way as in examples 24 to 30 and 31. This reaction (going through Examples 28 to 31) and the resulting compounds, Example 39 (1-Isopentyl-1'-methyl-6',7'-dihydro-1'H-spiro[piperidin-4,4'-pyrano[4,3-c]pyrazole]), Example 40 (1-(Methylbut-2-en-1-yl)-1'-methyl-6',7'-dihydro-1'H-spiro[piperidin-4,4'-pyrano[4,3-c]pyrazole]), and Example 41(1-Phenylpropyl-1'-methyl-6',7'-dihydro-1'H-spiro[piperidin-4,4'-pyrano[4,3-c]pyrazole])) are described in Scheme F:

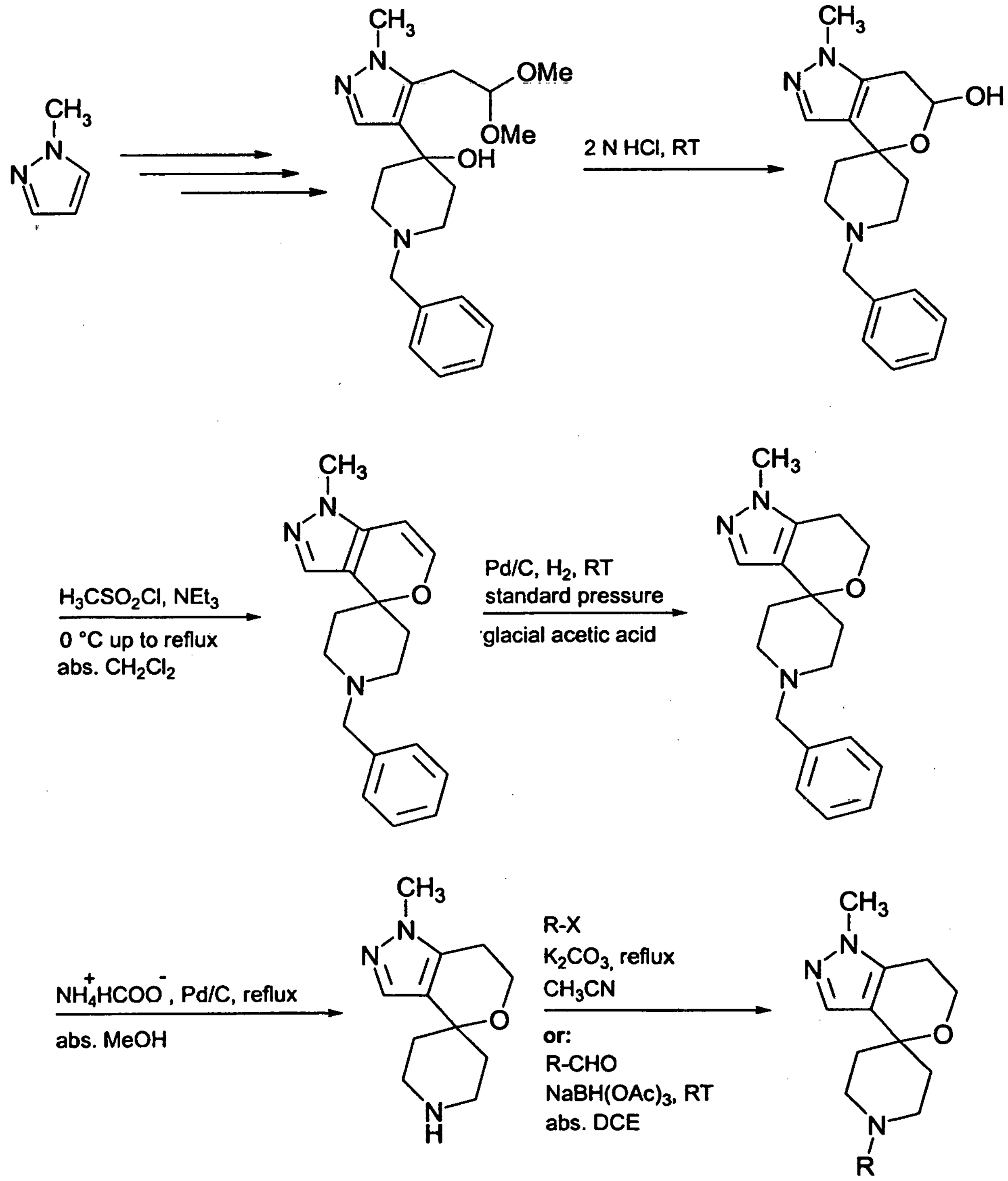


with R being here either:

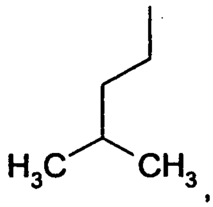,

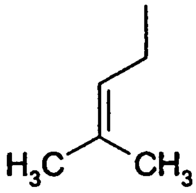

or 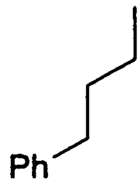.

(Example 39), (Example 40) or (Example 41)

BIOLOGICAL ACTIVITY

**A) In-Vitro**

Some representative compounds of the invention were tested for their activity as sigma (sigma-1 and sigma-2) inhibitors. The following protocols were followed:

**Sigma-1 (Version A)**

Brain membrane preparation and binding assays for the σ1-receptor were performed as described (DeHaven-Hudkins et al., 1992) with some modifications. In brief, guinea pig brains were homogenized in 10 vols. (w/v) of Tris-HCl 50 mM 0.32 M sucrose, pH 7.4, with a Kinematica Polytron PT 3000 at 15000 r.p.m. for 30 s. The homogenate was centrifuged at 1000g for 10 min at 4°C and the supernatants collected and centrifuged again at 48000g for 15 min at 4°C. The pellet was resuspended in 10 volumes of Tris-HCl buffer (50 mM, pH 7.4), incubated at 37°C for 30 min, and centrifuged at 48000g for 20 min at 4°C. Following this, the pellet was resuspended in fresh Tris-HCl buffer (50 mM, pH 7.4) and stored on ice until use.

Each assay tube contained 10 μL of $[^3H]$(+)-pentazocine (final concentration of 0.5 nM), 900 μL of the tissue suspension to a final assay volume of 1 mL and a final tissue concentration of approximately 30 mg tissue net weight/mL. Non-specific binding was defined by addition of a final concentration of 1 μM haloperidol. All tubes were incubated at 37°C for 150 min before termination of the reaction by rapid filtration over Schleicher & Schuell GF 3362 glass fibre filters [previously soaked in a solution of 0,5% polyethylenimine for at least 1 h]. Filters were then washed with four times with 4 mL of cold Tris-HCl buffer (50 mM, pH 7.4). Following addition of scintillation cocktail, the samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

**Sigma 1 (Version B)**

In brief the $\sigma_1$-receptor preparation was prepared from guinea pig brain. The brains were homogenized in 5 to 6 times of volume sucrose solution (0.32M) and homogenized. The homogenate was centrifuged at 2900 rpm, 4°C, 10 min). the supernatant was centrifuged again (23500 x g, 4°C, 20 min). The pellet was resuspended in Tris-buffer, incubated for 30 min at room temperature and centrifuged f (23500 x g, 4°C, 20 min). The pellet was resuspended in cold TRIS-buffer and homogenized. Then the protein content was measured (approx. 1.5 mg/mL) and the homogenate frozen at -80°C for later use.

The radioligand used was $[^3H]$-(+)-Pentazocin in TRIS-buffer. In a volume of 200 μL 50 μL TRIS-buffer, 50 μL compound solution of varying concentration, 50 μL radioligand- solution (8 nM; resulting in 2 nM in the assay) and finally 50 μL of receptor preparation (approx. 1.5 mg /mL) were given into a well of a microplate equipped with a filter. The plate was closed and stirred for 2.5 h at 37 °C and 500 rpm. Following that the solvents were removed by a harvester through the filter. After rinsing with $H_2O$ the filter was measured in a scintillation counter ($[^3H]$-protocol).

**Sigma-2 (Version A)**

Binding studies for σ2-receptor were performed as described (Radesca et al., 1991) with some modifications. In brief, brains from sigma receptor type I (σ1) knockout mice were homogenized in a volume of 10 mUg tissue net weight of ice-cold 10 mM Tris-HCl, pH 7.4, containing 320 mM sucrose (Tris-sucrose buffer) with a Potter-Elvehjem homogenizer (10 strokes at 500 r.p.m.) The homogenates were then centrifuged at 1000g for 10 min at 4°C, and the supernatants were saved. The pellets were resuspended by vortexing in 2 mUg ice-cold Tris-sucrose buffer and centrifuged again at 1000g for 10 min. The combined 1000g supernatants were centrifuged at 31000g for 15 min at 4°C. The

pellets were resuspended by vortexing in 3 mUg 10 mM Tris-HCl, pH 7.4, and the suspension was kept at 25°C for 15 min. Following centrifugation at 31000g for 15 min, the pellets were resuspended by gentle Potter Elvehjem homogenization to a volume of 1.53 mUg in 10 mM Tris-HCl pH 7.4.

The assay tubes contained 10 μL of [$^3$H]-DTG (final concentration of 3 nM), 400 μL of the tissue suspension (5.3 mUg in 50 mM Tris-HCl, pH 8.0) to a final assay volume of 0.5 mL. Non-specific binding was defined by addition of a final concentration of 1 μM haloperidol. All tubes were incubated at 25°C for 120 min before termination of the reaction by rapid filtration over Schleicher & Schuell GF 3362 glass fibre filters [previously soaked in a solution of 0,5% polyethylenimine for at least 1 h]. Filters were washed with three times with 5 mL volumes of cold Tris-HCl buffer (10 mM, pH 8.0). Following addition of scintillation cocktail samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

**References**

DeHaven-Hudkins, D. L., L.C. Fleissner, and F. Y. Ford-Rice, 1992, "Characterization of the binding of [3H](+) pentazocine to σ recognition sites in guinea pig brain", Eur. J. Pharmacol. 227, 371-378.

Radesca, L., W.D. Bowen, and L. Di Paolo, B.R. de Costa, 1991, Synthesis and Receptor Binding of Enantiomeric N-Substituted cis-N-[2-(3,4-Dichlorophenyl)ethyl]-2-(1-pyrrolidinyl)cyclohexylamines as High-Affinity σ Receptor Ligands, J. Med. Chem. 34, 3065-3074.

Langa, F., Codony X., Tovar V., Lavado A., Giménez E., Cozar P., Cantero M., Dordal A., Hernández E., Pérez R., Monroy X., Zamanillo D., Guitart X., Montoliu Ll., 2003, Generation and phenotypic analysis of sigma receptor type I (Sigma1) knockout mice, European Journal of Neuroscience, Vol. 18, 2188-2196.

Lowry, O.H., N.J. Rosebrough, A.L. Farr, and R.J. Randall, 1951, Protein measurement with the Folin phenol reagent, J. Biol. Chem, 193, 265.

**SIGMA 2 (Version B)**

In brief the $\sigma_2$-Receptor preparation was prepared from rat liver. The livers were homogenized in 5 to 6 times of volume sucrose solution (0.32M) and homogenized.

The homogenate was centrifuged at 2900 rpm, 4°C, 10 min). the supernatant was centrifuged again (31000 x g, 4°C, 20 min). The pellet was resuspended in TRIS-buffer, incubated for 30 min at room temperature while stirring and centrifuged f (31000 x g, 4°C, 20 min). The pellet was resuspended in cold TRIS-buffer pH 8 and homogenized. Then the protein content was measured (approx. 2 mg/mL) and the homogenate frozen at -80°C for later use.

The radioligand used was [$^3$H]-Ditolylguanidin in TRIS-buffer pH 8. The $\sigma_1$-receptor binding sites were masked through (+)-Pentazocin-solution in TRIS-Puffer pH 8.

In a volume of 200 μL 50 μL compound solution of varying concentration, 50 μL (+)-Pentazocin- solution (2 μM; resulting in 500 nM in the assay), 50 μL radioligand-solution (12 nM; resulting in 3 nM in the assay) and finally 50 μL of receptor preparation (approx. 2 mg /mL) were given into a well of a microplate equipped with a filter. The plate was closed and stirred for 2 h at room temperature and 500 rpm. Following that the solvents were removed by a harvester through the filter. After rinsing with $H_2O$ the filter was measured in a scintillation counter ([$^3$H]-protocol).

Some of the results obtained (according to the versions B) are shown in table (I).

**Table (I)**

| Example | Binding σ1 Ki [nM] | Binding σ2 Ki [nM] or [Inhibition at 1μM] |
|---|---|---|
| **2** | 1200 | [12% Inhibition at 1μM] |
| **3** | 0.49 +/- 0.15 (n=3) | 1750 |
| **4** | 1.28 +/- 0.14 (n=3) | 1050 |
| **5** | 1.46 +/- 0.08 (n=3) | [22% Inhibition at 1μM] |

(continued)

| Example | Binding σ1 Ki [nM] | Binding σ2 Ki [nM] or [Inhibition at 1μM] |
|---|---|---|
| 7 | 33.3 +/- 4.28 (n=3) | 3030 |
| 8 | 210 +/- 35.1 (n=3) | 3210 |
| 9 | 8.04 +/- 2.20 (n=3) | 752 |
| 10 | 6.32 +/- 1.05 (n=3) | 933 |
| 11 | 0.82 +/- 0.06 (n=3) | 340 +/- 32.5 |
| 12 | 0.97 +/- 0.16 (n=3) | 316 +/- 53.3 |
| 13 | 1.64 +/- 0.33 (n=4) | [3% Inhibition at 1μM] |
| 14 | 2.86 +/- 0.47 (n=3) | 209 +/- 21.8 |
| 15 | 0.55 +/- 0.17 (n=3) | 109 |
| 16 | 2.73 +/- 0.54 (n=3) | 570 |
| 17 | 3.24 +/- 0.70 (n=4) | 833 |
| 18 | 3.22 +/- 0.29 (n=5) | 428 |
| 19 | 2.21 +/- 0.37 (n=3) | 5220 |
| 20 | 1.54 +/- 0.29 (n=3) | 925 |
| 21 | 0.94 +/- 0.21 (n=4) | 687 |
| 22 | 21.4 +/- 2.33 (n=4) | [0% Inhibition at 1μM] |
| 24 | 92.7 +/- 18.9 (n=5) | 1730 |
| 25 | 21.1 +/- 6.29 (n=3) | [46 % Inhibition at 1μM] |
| 26 | 17.8 +/- 6.32 (n=3) | [22% Inhibition at 1μM] |
| 27 | 2980 | [0% Inhibition at 1μM] |
| 28 | 190 +/- 9.50 (n=2) | [21% Inhibition at 1 μM] |
| 29 | 11.8 +/- 3.68 (n=3) | 429 |
| 30 | 9.18 +/- 2.76 (n=3) | 191 |
| 32 | 27.1 +/- 6.72 (n=3) | [41% Inhibition at 1 μM] |
| 33 | 1.48 +/- 0.27 (n=3) | 557 |
| 34 | 1.71 +/- 0.08 (n=3) | 773 |
| 35 | 0.98 +/- 0.17 (n=3) | 83.3 +/- 22.0 |
| 36 | 0.97 +/- 0.07 (n=3) | 269 |
| 37 | 0.81 +/- 0.15 (n=3) | 102 |
| 38 | 0.43 +/- 0.09 | 42.8 +/- 4.84 |
| 39 | 29.9 +/- 11.1 (n=3) | [27% Inhibition at 1μM] |
| 40 | 14.0 +/-2.86 (n=3) | [49% Inhibition at 1μM] |
| 41 | 17.4 +/- 10.8 (n=3) | 1070 |

Some of the results obtained for Sigma 1 according to the version A are shown in table (II).

Table (II)

| Example | Binding σ1 Ki [nM] |
|---|---|
| **3** | 2.7 +/- 2.1 |
| **4** | 1.2 +/- 0.4 |
| **8** | > 100 |
| **10** | 9,4 |
| **12** | 2.5 |
| **13** | 7.3 +/- 0.7 |
| **14** | 41.0 +/- 8.4 |
| **15** | 7.9 +/- 1.0 |
| **18** | 15.7 |
| **19** | 3.8 +/- 0.1 |
| **21** | 7.7 |

**B) In-Vivo**

**EFFECT ON CAPSAICIN IN DEVELOPMENT OF MECHANICAL ALLODYNIA**

This model uses the von-Frey Filaments and is a model to test the effects or symptoms of neuropathic pain, allodynia etc.

Interest of the model:

- The injection of 1 μg of capsaicin to experimental animals produces acute pain followed by hyperalgesia/allodynia
- The mechanisms involved in capsaicin-induced acute pain and hyperalgesia are relatively well known (mainly activation of peripheral nociceptors and sensitization of spinal cord neurons, respectively)

The test protocol for all tests with of Frey filaments: After habituation mice were first treated with the test-compound (or solvent in controls). Then 1 μg capsaicin (1% DMSO) is injected into their paw resulting in developing pain in the effected paw. The effected paw is then treated with a mechanical stimulus and the latency time before the paw is withdrawn is measured.

## Claims

**1.** Compound of general formula Ia,

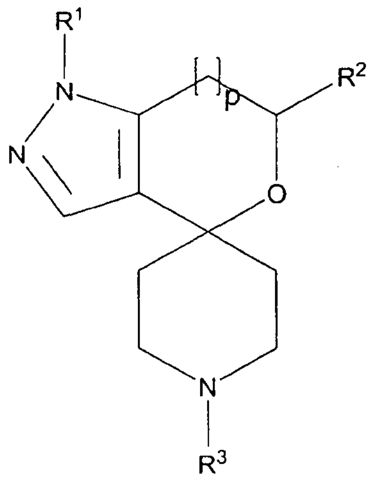

Ia

wherein

p is selected from 0 or 1;
$R^1$ is selected from linear or branched $C_{1-4}$-alkyl; or optionally at least monosubstituted aryl;
$R^2$ is selected from H; OH or a linear or branched $OC_{1-4}$-alkyl group;
$R^3$ is selected from hydrogen; $C_{1-10}$-alkyl, linear or branched; $C_{3-8}$-alkenyl, linear or branched; optionally at least mono-substituted $C_{1-6}$-alkyl-aryl; optionally at least mono-substituted $C_{1-6}$-alkyl-heterocyclyl; or optionally at least mono-substituted $C_{1-6}$-alkyl-$C_{4-8}$-cycloalkyl, wherein the cycloalkyl group can be saturated or unsaturated, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof,
wherein aryl or alkyl-aryl, cycloalkyl or alkyl-cycloalkyl, heterocyclyl or alkyl-heterocyclyl groups, are substituted on the ring-system of the aryl or alkyl-aryl, cycloalkyl or alkyl-cycloalkyl, heterocyclyl or alkyl-heterocyclyl, by substituents selected from OH, SH, =O, halogen (F, Cl, Br, I), CN, $NO_2$, ,COOH; $NR_xR_y$, with $R_x$ and $R_y$ independently being either H or a saturated or unsaturated, linear or branched, substituted or unsubstituted $C_{1-6}$-alkyl; a saturated or unsaturated, linear or branched, substituted or unsubstituted -O-$C_{1-6}$-alkyl (alkoxy); a saturated or unsaturated, linear or branched, substituted or unsubstituted -S-$C_{16}$-alkyl; a saturated or unsaturated, linear or branched, substituted or unsubstituted C(O)- $C_{1-6}$-alkyl-group; a saturated or unsaturated, linear or branched, substituted or unsubstituted C(O)-O-$C_{1-6}$-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl, and
wherein alkyls or alkenyls can be substituted or unsubstituted, wherein substitutents of alkyls or alkenyls are selected from the group consisting of F, Cl, Br, I, $NH_2$, SH or OH.

**2.** Compound according to claim 1, **characterized in that** the compound is selected from

- 6'-Methoxy-1'-phenyl-4',6'-dihydro-1'H-spiro[piperidin-4,4'-furo[3,4-c]pyrazole];
- 1-Benzyl-6'-methoxy-1'-phenyl-4',6'-dihydro-1'H-spiro[piperidin-4,4'-furo[3,4-c]pyrazole];
- 1-Butyl-6'-methoxy-1'-phenyl-4',6'-dihydro-1'H-spiro[piperidin-4,4'-furo[3,4-c]pyrazole];
- 1-Benzyl-6'-methoxy-1'-phenyl-6',7'-dihydro-1'H-spiro[piperidin-4,4'-pyrano[4,3-c]pyrazole];
- 6'-Methoxy-1'-phenyl-1-propyl-6',7'-dihydro-1'H-spiro[piperidin-4,4'-pyrano[4,3-c]pyrazole];
- 1-Isopropyl-6'-methoxy-1'-phenyl-6',7'-dihydro-1'H-spiro[piperidin-4,4'-pyrano[4,3-c]pyrazole];
- 1-Butyl-6'-methoxy-1'-phenyl-6',7'-dihydro-1'H-spiro[piperidin-4,4'-pyrano[4,3-c]pyrazole];
- 1-Isobutyl-6'-methoxy-1'-phenyl-6',7'-dihydro-1'H-spiro[piperidin-4,4'-pyrano[4,3-c]pyrazole];
- 6'-Methoxy-1-pentyl-1'-phenyl-6',7'-dihydro-1'H-spiro[piperidin-4,4'-pyrano[4,3-c]pyrazole];
- 1-Isopentyl-6'-methoxy-1'-phenyl-6',7'-dihydro-1'H-spiro[piperidin-4,4'-pyrano[4,3-c]pyrazole];
- 6'-Methoxy-1-(3-methylbut-2-en-1-yl)-1'-phenyl-6',7'-dihydro-1'H-spiro[piperidin-4,4'-pyrano[4,3-c]pyrazole];
- 6'-Methoxy-1-octyl-1'-phenyl-6',7'-dihydro-1'H-spiro[piperidin-4,4'-pyrano[4,3-c]pyrazole];

- 1-(Cyclohexan-1-ylmethyl)-6'-methoxy-1'-phenyl-6',7'-dihydro-1'H-spiro[piperidin-4,4'-pyrano[4,3-c]pyrazole];
- 6'-Methoxy-1'-phenyl-1-(2-phenylethyl)-6',7'-dihydro-1'H-spiro[piperidin-4,4'-pyrano[4,3-c]pyrazole];
- 6'-Methoxy-1'-phenyl-1-(3-phenylpropyl)-6',7'-dihydro-1'H-spiro[piperidin-4,4'-pyrano[4,3-c]pyrazole];
- 6'-Methoxy-1'-phenyl-1-(4-phenylbutyl)-6',7'-dihydro-1'H-spiro[piperidin-4,4'-pyrano[4,3-c]pyrazole];
- 1-(Furan-2-ylmethyl)-6'-methoxy-1'-phenyl-6',7'-dihydro-1'H-spiro[piperidin-4,4'-pyrano[4,3-c]pyrazole];
- 6'-Methoxy-1-(4-methoxybenzyl)-1'-phenyl-6',7'-dihydro-1'H-spiro[piperidin-4,4'-pyrano[4,3-c]pyrazole];
- 1-(4-Fluorbenzyl)-6'-methoxy-1'-phenyl-6',7'-dihydro-1'H-spiro[piperidin-4,4'-pyrano[4,3-c]pyrazole];
- 1-Benzyl-6'-methoxy-1'-methyl-6',7'-dihydro-1'H-spiro[piperidin-4,4'-pyrano[4,3-c]pyrazole];
- 6'-Methoxy-1'-methyl-6',7'-dihydro-1'H-spiro[piperidin-4,4'-pyrano[4,3-c]pyrazole];
- 6'-Methoxy-1'-methyl-1-(3-phenylpropyl)-6',7'-dihydro-1'H-spiro[piperidin-4,4'-pyrano[4,3-c]pyrazole];
- 1-(4-Fluorbenzyl)-6'-methoxy-1'-methyl-6',7'-dihydro-1'H-spiro[piperidin-4,4'-pyrano[4,3-c]pyrazole];
- 1-Isopentyl-6'-methoxy-1'-methyl-6',7'-dihydro-1'H-spiro[piperidin-4,4'-pyrano[4,3-c]pyrazole];
- 6'-Methoxy-1'-methyl-1-propyl-6',7'-dihydro-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]-6'-ol;
- 1-Benzyl-1'-methyl-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]; or
- 1'-Methyl-6',7'-dihydro-1'H-spiro[piperidin-4,4'-pyrano[4,3-c]pyrazole];
- 1-Benzyl-6'-hydroxy-1'-phenyl-6',7'-dihydro-1'H-spiro[piperidin-4,4'-pyrano[4,3-c]pyrazole];
- 1-Benzyl-1'-phenyl-6',7'-dihydro-1'H-spiro[piperidin-4,4'-pyrano[4,3-c]pyrazole];
- 1'-phenyl-6',7'-dihydro-1'H-spiro[piperidin-4,4'-pyrano[4,3-c]pyrazole];
- 1-Isopentyl-1'-phenyl-6',7'-dihydro-1'H-spiro[piperidin-4,4'-pyrano[4,3-c]pyrazole];
- 1-(3-methylbut-2-en-1-yl)-1'-phenyl-6',7'-dihydro-1'H-spiro[piperidin-4,4'-pyrano[4,3-c]pyrazole];
- 1'-phenyl-1-(3-phenylpropyl)-6',7'-dihydro-1'H-spiro[piperidin-4,4'-pyrano[4,3-c]pyrazole];
- 1-Isopentyl-1'-methyl-6',7'-dihydro-1'H-spiro[piperidin-4,4'-pyrano[4,3-c]pyrazole];
- 1-(3-methylbut-2-en-1-yl)-1'-methyl-6',7'-dihydro-1'H-spiro[piperidin-4,4'-pyrano[4,3-c]pyrazole];
- 1'-methyl-1-(3-phenylpropyl)-6',7'-dihydro-1'H-spiro[piperidin-4,4'-pyrano[4,3-c]pyrazole]; or
- 1-(Cyclohexan-1-ylmethyl)-1'-phenyl-6',7'-dihydro-1'H-spiro[piperidin-4,4'-pyrano[4,3-c]pyrazole]

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**3.** Compound according to claim 1, **characterized in that** the compound is selected from

- 6'-Methoxy-1'-phenyl-4',6'-dihydro-1'H-spiro[piperidin-4,4'-furo[3,4-c]pyrazole];
- 1-Benzyl-6'-methoxy-1'-phenyl-4',6'-dihydro-1'H-spiro[piperidin-4,4'-furo[3,4-c]pyrazole];
- 1-Butyl-6'-methoxy-1'-phenyl-4',6'-dihydro-1'H-spiro[piperidin-4,4'-furo[3,4-c]pyrazole];
- 1-Benzyl-6'-methoxy-1'-phenyl-6',7'-dihydro-1'H-spiro[piperidin-4,4'-pyrano[4,3-c]pyrazole];
- 6'-Methoxy-1'-phenyl-1-propyl-6',7'-dihydro-1'H-spiro[piperidin-4,4'-pyrano[4,3-c]pyrazole];
- 1-Isopropyl-6'-methoxy-1'-phenyl-6',7'-dihydro-1'H-spiro[piperidin-4,4'-pyrano[4,3-c]pyrazole];
- 1-Butyl-6'-methoxy-1'-phenyl-6',7'-dihydro-1'H-spiro[piperidin-4,4'-pyrano[4,3-c]pyrazole];
- 1-Isobutyl-6'-methoxy-1'-phenyl-6',7'-dihydro-1'H-spiro[piperidin-4,4'-pyrano[4,3-c]pyrazole];
- 6'-Methoxy-1-pentyl-1'-phenyl-6',7'-dihydro-1'H-spiro[piperidin-4,4'-pyrano[4,3-c]pyrazole];
- 1-Isopentyl-6'-methoxy-1'-phenyl-6',7'-dihydro-1'H-spiro[piperidin-4,4'-pyrano[4,3-c]pyrazole];
- 6'-Methoxy-1-(3-methylbut-2-en-1-yl)-1'-phenyl-6',7'-dihydro-1'H-spiro[piperidin-4,4'-pyrano[4,3-c]pyrazole];
- 6'-Methoxy-1-octyl-1'-phenyl-6',7'-dihydro-1'H-spiro[piperidin-4,4'-pyrano[4,3-c]pyrazole];
- 1-(Cyclohexan-1-ylmethyl)-6'-methoxy-1'-phenyl-6',7'-dihydro-1'H-spiro[piperidin-4,4'-pyrano[4,3-c]pyrazole];
- 6'-Methoxy-1'-phenyl-1-(2-phenylethyl)-6',7'-dihydro-1'H-spiro[piperidin-4,4'-pyrano[4,3-c]pyrazole];
- 6'-Methoxy-1'-phenyl-1-(3-phenylpropyl)-6',7'-dihydro-1'H-spiro[piperidin-4,4'-pyrano[4,3-c]pyrazole];
- 6'-Methoxy-1'-phenyl-1-(4-phenylbutyl)-6',7'-dihydro-1'H-spiro[piperidin-4,4'-pyrano[4,3-c]pyrazole];
- 1-(Furan-2-ylmethyl)-6'-methoxy-1'-phenyl-6',7'-dihydro-1'H-spiro[piperidin-4,4'-pyrano[4,3-c]pyrazole];
- 6'-Methoxy-1-(4-methoxybenzyl)-1'-phenyl-6',7'-dihydro-1'H-spiro[piperidin-4,4'-pyrano[4,3-c]pyrazole];
- 1-(4-Fluorbenzyl)-6'-methoxy-1'-phenyl-6',7'-dihydro-1'H-spiro[piperidin-4,4'-pyrano[4,3-c]pyrazole];
- 1-Benzyl-6'-methoxy-1'-methyl-6',7'-dihydro-1'H-spiro[piperidin-4,4'-pyrano[4,3-c]pyrazole];
- 6'-Methoxy-1'-methyl-6',7'-dihydro-1'H-spiro[piperidin-4,4'-pyrano[4,3-c]pyrazole];
- 6'-Methoxy-1'-methyl-1-(3-phenylpropyl)-6',7'-dihydro-1'H-spiro[piperidin-4,4'-pyrano[4,3-c]pyrazole];

- 1-(4-Fluorbenzyl)-6'-methoxy-1'-methyl-6',7'-dihydro-1'H-spiro[piperidin-4,4'-pyrano[4,3-c]pyrazole];
- 1-Isopentyl-6'-methoxy-1'-methyl-6',7'-dihydro-1'H-spiro[piperidin-4,4'-pyrano[4,3-c]pyrazole];
- 6'-Methoxy-1'-methyl-1-propyl-6',7'-dihydro-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]-6'-ol;
- 1-Benzyl-1'-methyl-1'H-spiro[piperidin-4,4'-pyrano[4,3-c]pyrazole];
- 1-Benzyl-1'-methyl-6',7'-dihydro-1'H-spiro[piperidin-4,4'-pyrano[4,3-c]pyrazole]; or
- 1'-Methyl-6',7'-dihydro-1'H-spiro[piperidin-4,4'-pyrano[4,3-c]pyrazole];

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**4.** Compound according to claim 1, **characterized in that** the compound is
1-Benzyl-1'-phenyl-1'*H*-spiro[piperidine-4,4'-pyrano[4,3-*c*]pyrazole]
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**5.** Process for the production of a compound according to formula Ia, wherein a compound of formula IIIa

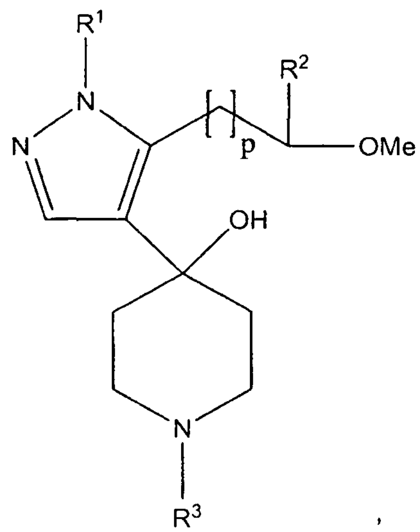

,

wherein $R^1$, $R^2$, $R^3$ and p are as defined in claim 1 is reacted with acid to form a compound according the formula Ia.

**6.** Process according to claim 5, wherein as a next step a compound according to formula Ia in which p is 0 and $R^3$ is C(O)OR' is reacted with a KOH solution in water to form a compound according to formula Ia with $R^3$ being H
or
wherein as a next step a compound according to formula Ia in which p is 1 and $R^3$ is benzyl is reacted with $NH_4^+$ $HCOO^-$ with a Pd/C catalysator to form a compound according to formula Ia with $R^3$ being H, preferably
wherein as a next step a compound according to formula Ia in which $R^3$ is hydrogen is reacted with a compound $R^3$-X with X being a leaving group and $K_2CO_3$ under reflux to form a compound according to formula Ia with $R^3$ being as defined in claim 1, except H.

**7.** A pharmaceutical composition which comprises a compound as defined in claim 1 or a pharmaceutically acceptable salt or solvate thereof, and a pharmaceutically acceptable carrier, adjuvant or vehicle.

**8.** Use of a compound as defined in any of claims 1-4 for the production of a medicament for the treatment or prophylaxis of a sigma receptor mediated disease,
wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer, or wherein the disease is pain, especially neuropathic pain, inflammatory pain or other pain conditions, allodynia and/or hyperalgesia, especially mechanical allodynia.

**9.** A compound as defined in any of claims 1-4 for use in the treatment of a sigma receptor mediated disease, wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer, or wherein the disease is pain, especially neuropathic pain, inflammatory pain or other pain conditions, allodynia and/or hyperalgesia, especially mechanical allodynia.

## Patentansprüche

**1.** Verbindung gemäß der allgemeinen Formel Ia,

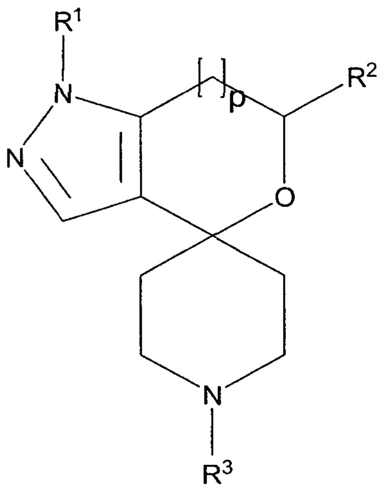

Ia

wobei

p aus 0 oder 1 ausgewählt ist;

$R^1$ aus einer linearen oder verzweigten $C_{1-4}$-Alkylgruppe; oder aus einer wahlweise zumindest monosubstituierten Arylgruppe ausgewählt ist;

$R^2$ aus H; OH oder aus einer linearen oder verzweigten $OC_{1-4}$-Alkylgruppe ausgewählt ist;

$R^3$ aus Wasserstoff; einer linearen oder verzweigten $C_{1-10}$-Alkylgruppe; einer linearen oder verzweigten $C_{3-8}$-Alkenylgruppe; aus einer wahlweise zumindest mono-substitutierten $C_{1-6}$-Alkyl-Arylgruppe; aus einer wahlweise zumindest mono-substitutierten $C_{1-6}$-Alkyl-Heterozyklylgruppe; oder aus einer wahlweise zumindest mono-substitutierten $C_{1-6}$-Alkyl-$C_{4-8}$-Zykloalkylgruppe ausgewählt ist, wobei die Zykloalkylgruppe gesättigt oder ungesättigt sein kann,

wahlweise in Form eines der Stereoisomere, bevorzugt Enantiomere oder Diastereomere, eines Razemats oder in Form eines Gemischs von mindestens zwei der Stereoisomere, bevorzugt Enantiomere und/oder Diastereomere, in jeglichem Mischungsverhältnis, oder einem entsprechenden Salz davon, oder einem entsprechenden Solvat davon,

wobei die Aryl- oder Alkyl-Aryl-, Zykloalkyl- oder Alkyl-Zykloalkyl-, Heterozyklyl-oder Alkyl-Heterozyklyl Gruppen, an dem Ringsystem der Aryl- oder Alkyl-Aryl-, Zykloalkyl- oder Alkyl-Zykloalkyl-, Heterozyklyl- oder Alkyl-Heterozyklyl Gruppen mit Substituenten ausgewählt aus OH, SH, =O, Halogen (F, Cl, Br, I), CN, $NO_2$, COOH; $NR_xR_y$ substitutiert sind, wobei $R_x$ und $R_y$ unabhängig voneinander entweder H oder eine gesättigte oder ungesättigte, lineare or verzweigte, substituierte oder unsubstitutierte $C_{1-6}$-Alkylgruppe; eine gesättigte oder ungesättigte, lineare oder verzweigte, substituierte oder unsubstitutierte -O-$C_{1-6}$-Alkyl (Alkoxy)gruppe; eine gesättigte oder ungesättigte, lineare or verzweigte, substituierte oder unsubstitutierte -S-$C_{1-6}$-Alkylgruppe; eine gesättigte oder ungesättigte, lineare or verzweigte, substituierte oder unsubstitutierte C(O)- $C_{1-6}$-Alkylgruppe; eine gesättigte oder ungesättigte, lineare oder verzweigte, substituierte oder unsubstitutierte C(O)-O-$C_{1-6}$-Alkylgruppe; eine subsituierte oder unsubsituierte Aryl- oder Alkyl-Arylgruppe; eine subsituierte oder unsubsituierte Zykloalkyl- oder Alkyl-Zykloalkylgruppe; eine subsituierte oder unsubsituierte Heterozyklyl- oder Alkyl-Heterozyklylgruppe sind,
und
wobei Alkyl- oder Alkenylgruppen subsituiert oder unsubsituiert sein können, wobei die Substitutenten der Alkyl- oder Alkenylgruppen ausgewählt sind aus der Gruppe bestehend aus F, Cl, Br, I, $NH_2$, SH oder OH.

**2.** Verbindung gemäß Anspruch1, dadurch charakterisiert, dass die Verbindung ausgewählt ist aus

- 6'-Methoxy-1'-phenyl-4',6'-dihydro-1'H-spiro[piperidin-4,4'-furo[3,4-c]pyrazol];
- 1-Benzyl-6'-methoxy-1'-phenyl-4',6'-dihydro-1'H-spiro[piperidin-4,4'-furo[3,4-c]pyrazol];
- 1-Butyl-6'-methoxy-1'-phenyl-4',6'-dihydro-1'H-spiro[piperidin-4,4'-furo[3,4-c]pyrazol];
- 1-Benzyl-6'-methoxy-1'-phenyl-6',7'-dihydro-1'H-spiro[piperidin-4,4'-pyrano[4,3-c]pyrazol];
- 6'-Methoxy-1'-phenyl-1-propyl-6',7'-dihydro-1'H-spiro[piperidin-4,4'-pyrano[4,3-c]pyrazol];
- 1-Isopropyl-6'-methoxy-1'-phenyl-6',7'-dihydro-1'H-spiro[piperidin-4,4'-pyrano[4,3-c]pyrazol];
- 1-Butyl-6'-methoxy-1'-phenyl-6',7'-dihydro-1'H-spiro[piperidin-4,4'-pyrano[4,3-c]pyrazol];
- 1-Isobutyl-6'-methoxy-1'-phenyl-6',7'-dihydro-1'H-spiro[piperidin-4,4'-pyrano[4,3-c]pyrazol];
- 6'-Methoxy-1-pentyl-1'-phenyl-6',7'-dihydro-1'H-spiro[piperidin-4,4'-pyrano[4,3-c]pyrazol];
- 1-Isopentyl-6'-methoxy-1'-phenyl-6',7'-dihydro-1'H-spiro[piperidin-4,4'-pyrano[4,3-c]pyrazol];
- 6'-Methoxy-1-(3-methylbut-2-en-1-yl)-1'-phenyl-6',7'-dihydro-1'H-spiro[piperidin-4,4'-pyrano[4,3-c]pyrazol];
- 6'-Methoxy-1-octyl-1'-phenyl-6',7'-dihydro-1'H-spiro[piperidin-4,4'-pyrano[4,3-c]pyrazol];
- 1-(Cyclohexan-1-ylmethyl)-6'-methoxy-1'-phenyl-6',7'-dihydro-1'H-spiro[piperidin-4,4'-pyrano[4,3-c]pyrazol];
- 6'-Methoxy-1'-phenyl-1-(2-phenylethyl)-6',7'-dihydro-1'H-spiro[piperidin-4,4'-pyrano[4,3-c]pyrazol];
- 6'-Methoxy-1'-phenyl-1-(3-phenylpropyl)-6',7'-dihydro-1'H-spiro[piperidin-4,4'-pyrano[4,3-c]pyrazol];
- 6'-Methoxy-1'-phenyl-1-(4-phenylbutyl)-6',7'-dihydro-1'H-spiro[piperidin-4,4'-pyrano[4,3-c]pyrazol];
- 1-(Furan-2-ylmethyl)-6'-methoxy-1'-phenyl-6',7'-dihydro-1'H-spiro[piperidin-4,4'-pyrano[4,3-c]pyrazol];
- 6'-Methoxy-1-(4-methoxybenzyl)-1'-phenyl-6',7'-dihydro-1'H-spiro[piperidin-4,4'-pyrano[4,3-c]pyrazol];
- 1-(4-Fluorbenzyl)-6'-methoxy-1'-phenyl-6',7'-dihydro-1'H-spiro[piperidin-4,4'-pyrano[4,3-c]pyrazol];
- 1-Benzyl-6'-methoxy-1'-methyl-6',7'-dihydro-1'H-spiro[piperidin-4,4'-pyrano[4,3-c]pyrazol];
- 6'-Methoxy-1'-methyl-6',7'-dihydro-1'H-spiro[piperidin-4,4'-pyrano[4,3-c]pyrazol];
- 6'-Methoxy-1'-methyl-1-(3-phenylpropyl)-6',7'-dihydro-1'H-spiro[piperidin-4,4'-pyrano[4,3-c]pyrazol];
- 1-(4-Fluorbenzyl)-6'-methoxy-1'-methyl-6',7'-dihydro-1'H-spiro[piperidin-4,4'-pyrano[4,3-c]pyrazol];
- 1-Isopentyl-6'-methoxy-1'-methyl-6',7'-dihydro-1'H-spiro[piperidin-4,4'-pyrano[4,3-c]pyrazol];
- 6'-Methoxy-1'-methyl-1-propyl-6',7'-dihydro-1'H-spiro[piperidin-4,4'-pyrano[4,3-c]pyrazol];
- 1-Benzyl-1'-methyl-6',7'-dihydro-1'H-spiro[piperidin-4,4'-pyrano[4,3-c]pyrazol]-6'-ol;
- 1-Benzyl-1'-methyl-1'H-spiro[piperidin-4,4'-pyrano[4,3-c]pyrazol];
- 1-Benzyl-1'-methyl-6',7'-dihydro-1'H-spiro[piperidin-4,4'-pyrano[4,3-c]pyrazol]; oder
- 1'-Methyl-6',7'-dihydro-1'H-spiro[piperidin-4,4'-pyrano[4,3-c]pyrazol];
- 1-Benzyl-6'-hydroxy-1'-phenyl-6',7'-dihydro-1'H-spiro[piperidin-4,4'-pyrano[4,3-c]pyrazol];
- 1-Benzyl-1'-phenyl-6',7'-dihydro-1'H-spiro[piperidin-4,4'-pyrano[4,3-c]pyrazol];
- 1'-Phenyl-6',7'-dihydro-1'H-spiro[piperidin-4,4'-pyrano[4,3-c]pyrazol];
- 1-Isopentyl-1'-phenyl-6',7'-dihydro-1'H-spiro[piperidin-4,4'-pyrano[4,3-c]pyrazol];
- 1-(3-methylbut-2-en-1-yl)-1'-phenyl-6',7'-dihydro-1'H-spiro[piperidin-4,4'-pyrano[4,3-c]pyrazol];
- 1'-Phenyl-1-(3-phenylpropyl)-6',7'-dihydro-1'H-spiro[piperidin-4,4'-pyrano[4,3-c]pyrazol];
- 1-Isopentyl-1'-methyl-6',7'-dihydro-1'H-spiro[piperidin-4,4'-pyrano[4,3-c]pyrazol];
- 1-(3-methylbut-2-en-1-yl)-1'-methyl-6',7'-dihydro-1'H-spiro[piperidin-4,4'-pyrano[4,3-c]pyrazol];
- 1'-Methyl-1-(3-phenylpropyl)-6',7'-dihydro-1'H-spiro[piperidin-4,4'-pyrano[4,3-c]pyrazol]; oder
- 1-(Cyclohexan-1-ylmethyl)-1'-phenyl-6',7'-dihydro-1'H-spiro[piperidin-4,4'-pyrano[4,3-c]pyrazol]

wahlweise in Form eines der Stereoisomere, bevorzugt Enantiomere oder Diastereomere, eines Razemats oder in Form eines Gemischs von mindestens zwei der Stereoisomere, bevorzugt Enantiomere und/oder Diastereomere, in jeglichem Mischungsverhältnis, oder einem entsprechenden Salz davon, oder einem entsprechenden Solvat davon.

**3.** Verbindung gemäß Anspruch1, dadurch charakterisiert, dass die Verbindung ausgewählt ist aus

- 6'-Methoxy-1'-phenyl-4',6'-dihydro-1'H-spiro[piperidin-4,4'-furo[3,4-c]pyrazol];
- 1-Benzyl-6'-methoxy-1'-phenyl-4',6'-dihydro-1'H-spiro[piperidin-4,4'-furo[3,4-c]pyrazol];
- 1-Butyl-6'-methoxy-1'-phenyl-4',6'-dihydro-1'H-spiro[piperidin-4,4'-furo[3,4-c]pyrazol];
- 1-Benzyl-6'-methoxy-1'-phenyl-6',7'-dihydro-1'H-spiro[piperidin-4,4'-pyrano[4,3-c]pyrazol];
- 6'-Methoxy-1'-phenyl-1-propyl-6',7'-dihydro-1'H-spiro[piperidin-4,4'-pyrano[4,3-c]pyrazol];
- 1-Isopropyl-6'-methoxy-1'-phenyl-6',7'-dihydro-1'H-spiro[piperidin-4,4'-pyrano[4,3-c]pyrazol];
- 1-Butyl-6'-methoxy-1'-phenyl-6',7'-dihydro-1'H-spiro[piperidin-4,4'-pyrano[4,3-c]pyrazol];
- 1-Isobutyl-6'-methoxy-1'-phenyl-6',7'-dihydro-1'H-spiro[piperidin-4,4'-pyrano[4,3-c]pyrazol];
- 6'-Methoxy-1-pentyl-1'-phenyl-6',7'-dihydro-1'H-spiro[piperidin-4,4'-pyrano[4,3-c]pyrazol];
- 1-Isopentyl-6'-methoxy-1'-phenyl-6',7'-dihydro-1'H-spiro[piperidin-4,4'-pyrano[4,3-c]pyrazol];
- 6'-Methoxy-1-(3-methylbut-2-en-1-yl)-1'-phenyl-6',7'-dihydro-1'H-spiro[piperidin-4,4'-pyrano[4,3-c]pyrazol];
- 6'-Methoxy-1-octyl-1'-phenyl-6',7'-dihydro-1'H-spiro[piperidin-4,4'-pyrano[4,3-c]pyrazol];
- 1-(Cyclohexan-1-ylmethyl)-6'-methoxy-1'-phenyl-6',7'-dihydro-1'H-spiro[piperidin-4,4'-pyrano[4,3-c]pyrazol];
- 6'-Methoxy-1'-phenyl-1-(2-phenylethyl)-6',7'-dihydro-1'H-spiro[piperidin-4,4'-pyrano[4,3-c]pyrazol];
- 6'-Methoxy-1'-phenyl-1-(3-phenylpropyl)-6',7'-dihydro-1'H-spiro[piperidin-4,4'-pyrano[4,3-c]pyrazol];
- 6'-Methoxy-1'-phenyl-1-(4-phenylbutyl)-6',7'-dihydro-1'H-spiro[piperidin-4,4'-pyrano[4,3-c]pyrazol];
- 1-(Furan-2-ylmethyl)-6'-methoxy-1'-phenyl-6',7'-dihydro-1'H-spiro[piperidin-4,4'-pyrano[4,3-c]pyrazol];
- 6'-Methoxy-1-(4-methoxybenzyl)-1'-phenyl-6',7'-dihydro-1'H-spiro[piperidin-4,4'-pyrano[4,3-c]pyrazol];
- 1-(4-Fluorbenzyl)-6'-methoxy-1'-phenyl-6',7'-dihydro-1'H-spiro[piperidin-4,4'-pyrano[4,3-c]pyrazol];
- 1-Benzyl-6'-methoxy-1'-methyl-6',7'-dihydro-1'H-spiro[piperidin-4,4'-pyrano[4,3-c]pyrazol];
- 6'-Methoxy-1'-methyl-6',7'-dihydro-1'H-spiro[piperidin-4,4'-pyrano[4,3-c]pyrazol];
- 6'-Methoxy-1'-methyl-1-(3-phenylpropyl)-6',7'-dihydro-1'H-spiro[piperidin-4,4'-pyrano[4,3-c]pyrazol];
- 1-(4-Fluorbenzyl)-6'-methoxy-1'-methyl-6',7'-dihydro-1'H-spiro[piperidin-4,4'-pyrano[4,3-c]pyrazol];
- 1-Isopentyl-6'-methoxy-1'-methyl-6',7'-dihydro-1'H-spiro[piperidin-4,4'-pyrano[4,3-c]pyrazol];
- 6'-Methoxy-1'-methyl-1-propyl-6',7'-dihydro-1'H-spiro[piperidin-4,4'-pyrano[4,3-c]pyrazol];
- 1-Benzyl-1'-methyl-6',7'-dihydro-1'H-spiro[piperidin-4,4'-pyrano[4,3-c]pyrazol]-6'-ol;
- 1-Benzyl-1'-methyl-1'H-spiro[piperidin-4,4'-pyrano[4,3-c]pyrazol];
- 1-Benzyl-1'-methyl-6',7'-dihydro-1'H-spiro[piperidin-4,4'-pyrano[4,3-c]pyrazol]; oder
- 1'-Methyl-6',7'-dihydro-1'H-spiro[piperidin-4,4'-pyrano[4,3-c]pyrazol];

wahlweise in Form eines der Stereoisomere, bevorzugt Enantiomere oder Diastereomere, eines Razemats oder in Form eines Gemischs von mindestens zwei der Stereoisomere, bevorzugt Enantiomere und/oder Diastereomere, in jeglichem Mischungsverhältnis, oder einem entsprechenden Salz davon, oder einem entsprechenden Solvat davon.

**4.** Verbindung nach Anspruch 1, charakterisiert dadurch, dass es sich bei der Verbindung um 1-Benzyl-1'-phenyl-1'*H*-spiro[piperidine-4,4'-pyrano[4,3-c]pyrazol] handelt,
wahlweise in Form eines der Stereoisomere, bevorzugt Enantiomere oder Diastereomere, eines Razemats oder in Form eines Gemischs von mindestens zwei der Stereoisomere, bevorzugt Enantiomere und/oder Diastereomere, in jeglichem Mischungsverhältnis, oder einem entsprechenden Salz davon, oder einem entsprechenden Solvat davon.

**5.** Verfahren zur Herstellung einer Verbindung gemäß Formel Ia, wobei eine Verbindung gemäß Formel IIIa

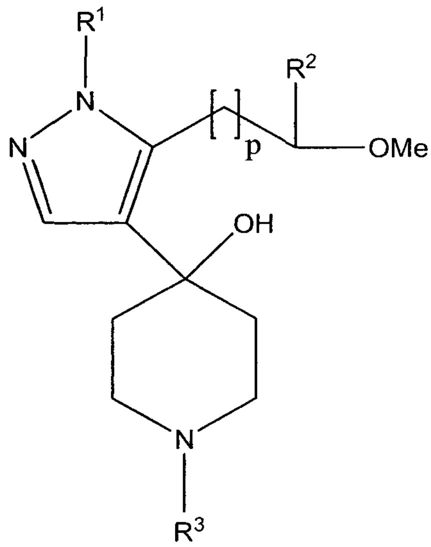

,

wobei $R^1$, $R^2$, $R^3$ und p wie in Anspruch 1 definiert vorliegen, mit einer Säure reagiert wird um eine Verbindung gemäß Formel Ia zu erhalten.

6. Verfahren gemäß Anspruch 5, wobei als nächster Schritt eine Verbindung gemäß Formel Ia, in der p 0 ist und $R^3$ C(O)OR' ist, mit einer wässrigen KOH-Lösung reagiert wird, um eine Verbindung gemäß Formel Ia in der $R^3$ H ist, zu erhalten
oder
wobei als nächster Schritt eine Verbindung gemäß Formel Ia in der p 1 ist und in der $R^3$ Benzyl ist, mit $NH_4^+$ $HCOO^-$ mit einem Pd/C Katalysator reagiert wird, um eine Verbindung gemäß Formel Ia in der $R^3$ H ist, zu erhalten, bevorzugt wobei als nächster Schritt eine Verbindung gemäß Formel Ia in der $R^3$ Wasserstoff ist, mit einer Verbindung $R^3$-X, wobei X eine Abgangsgruppe ist, und $K_2CO_3$ unter Rückfluss reagiert wird, um eine Verbindung gemäß Formel Ia in der $R^3$ wie in Anspruch 1 definiert ist, mit Ausnahme von H, zu erhalten.

7. Pharmazeutische Zusammensetzung umfassend eine Verbindung wie in Anspruch 1 definiert oder ein pharmazeutisch akzeptables Salz oder Solvat davon, sowie einen pharmazeutisch akzeptablen Trägerstoff, Adjuvant oder Vehikel.

8. Verwendung einer Verbindung wie in Ansprüchen 1-4 definiert zur Herstellung eines Medikaments zur Behandlung oder Vorsorge einer Sigma-Rezeptor vermittelten Krankheit,
wobei die Krankheit Diarrhoe, Lipoproteinstörungen, Metabolisches Syndrom, Behandlung erhöhter Triglyzeridspiegel, Chylomikronämie, Hyperlipoproteinämie; Hyperlipidämie, insbesondere gemischte Hyperlipidämie; Hypercholesterinämie, Dysbetalipoproteinämie, Hypertriglyzeridämie beinhaltend sowohl die sporadische als familiäre Störung (vererbliche Hypertriglyzeridämie), Migräne, Fettleibigkeit, Arthritis, Bluthochdruck, Arrhythmie, Magengeschwür, Lern-, Gedächtnis- und Aufmerksamkeitsstörungen, Kognitionsstörungen, neurodegenerative Erkrankungen, demyelinisierende Erkrankungen, Abhängigkeit von Drogen und chemischen Substanzen einschließlich Kokain, Amphetamin, Ethanol und Nikotin, tardive Dyskinesie, ischämischer Schlaganfall, Epilepsie, Schlaganfall, Depression, Stress, psychotische Störungen, Schizophrenie; Entzündungen, Autoimmunerkrankungen oder Krebs ist, oder
wobei die Erkrankung Schmerz, insbesondere neuropathischer Schmerz, Entzündungsschmerz oder andere Schmerzzustände, Allodynie und/oder Hyperalgesie, inbesondere mechanische Allodynie ist.

9. Verbindung wie in Ansprüchen 1-4 definiert zur Behandlung einer Sigma-Rezeptor vermittelten Krankheit,
wobei die Krankheit Diarrhoe, Lipoproteinstörungen, Metabolisches Syndrom, Behandlung erhöhter Triglyzeridspiegel, Chylomikronämie, Hyperlipoproteinämie; Hyperlipidämie, insbesondere gemischte Hyperlipidämie; Hypercholesterinämie, Dysbetalipoproteinämie, Hypertriglyzeridämie beinhaltend sowohl die sporadische als familiäre Störung (vererbliche Hypertriglyzeridämie), Migräne, Fettleibigkeit, Arthritis, Bluthochdruck, Arrhythmie, Magengeschwür, Lern-, Gedächtnis- und Aufmerksamkeitsstörungen, Kognitionsstörungen, neurodegenerative Erkrankungen, demyelinisierende Erkrankungen, Abhängigkeit von Drogen und chemischen Substanzen einschließlich Kokain, Amphetamin, Ethanol und Nikotin, tardive Dyskinesie, ischämischer Schlaganfall, Epilepsie, Schlaganfall, Depression, Stress, psychotische Störungen, Schizophrenie; Entzündungen, Autoimmunerkrankungen oder Krebs ist, oder

wobei die Erkrankung Schmerz, insbesondere neuropathischer Schmerz, Entzündungsschmerz oder andere Schmerzzustände, Allodynie und/oder Hyperalgesie, inbesondere mechanische Allodynie ist.

**Revendications**

**1.** Composé de formule générale Ia,

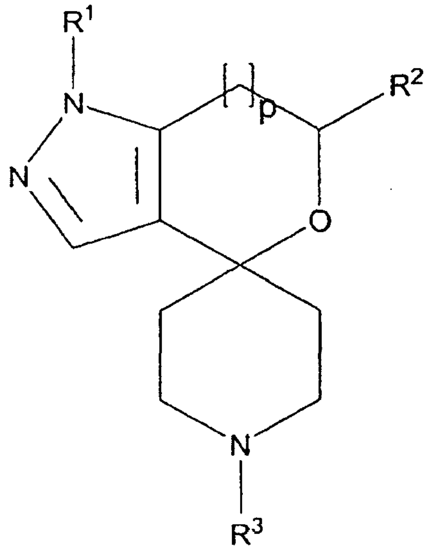

Ia

dans laquelle

p est choisi parmi 0 ou 1 ;

$R^1$ est choisi parmi un groupe alkyle en $C_{1-4}$ linéaire ou ramifié ; ou un groupe aryle facultativement au moins monosubstitué ;

$R^2$ est choisi parmi H ; OH ou un groupe alkyle en $OC_{1-4}$ linéaire ou ramifié ;

$R^3$ est choisi parmi l'hydrogène ; un groupe alkyle en $C_{1-10}$ linéaire ou ramifié ; un groupe alcényle en $C_{3-8}$ linéaire ou ramifié ; un groupe alkylaryle en $C_{1-6}$ facultativement au moins monosubstitué ; un groupe alkylhétérocyclyle en $C_{1-6}$ facultativement au moins monosubstitué ; ou un groupe alkyle en $C_{1-6}$-cycloalkyle en $C_{4-8}$ facultativement au moins monosubstitué, dans lequel le groupe cycloalkyle peut être saturé ou insaturé,

facultativement sous la forme de l'un des stéréoisomères, de préférence énantiomères ou diastéréomères, d'un racémate ou sous la forme d'un mélange d'au moins deux des stéréoisomères, de préférence énantiomères et/ou diastéréomères, selon un quelconque rapport de mélange, ou l'un de ses sels correspondants ou de ses solvates correspondants,

dans lequel les groupes aryle ou alkylaryle, cycloalkyle ou alkylcycloalkyle, hétérocyclyle ou alkylhétérocyclyle, sont substitués sur le système cyclique des groupes aryle ou alkylaryle, cycloalkyle ou alkylcycloalkyle, hétérocyclyle ou alkylhétérocyclyle, par des substituants choisis parmi OH, SH, =O, l'halogène (F, Cl, Br, I), CN, $NO_2$, COOH ; $NR_xR_y$, $R_x$ et $R_y$ étant indépendamment H ou un groupe alkyle en $C_{1-6}$ saturé ou insaturé, linéaire ou ramifié, substitué ou non substitué ; un groupe -O-alkyle (alcoxy) en $C_{1-6}$ saturé ou insaturé, linéaire ou ramifié, substitué ou non substitué ; un groupe -S-alkyle en $C_{1-6}$ saturé ou insaturé, linéaire ou ramifié, substitué ou non substitué ; un groupe C(O)-alkyle en $C_{1-6}$ saturé ou insaturé, linéaire ou ramifié, substitué ou non substitué ; un groupe C(O)-O-alkyle en $C_{1-6}$ saturé ou insaturé, linéaire ou ramifié, substitué ou non substitué ; un groupe aryle ou alkylaryle substitué ou non substitué ; un groupe cycloalkyle ou alkylcycloalkyle substitué ou non substitué ,un groupe hétérocyclyle ou alkylhétérocyclyle substitué ou non substitué,

et

dans lequel les groupes alkyle ou alcényle peuvent être substitués ou non substitués, les substituants des groupes alkyle ou alcényle étant choisis dans le groupe constitué par F, Cl, Br, I, $NH_2$, SH ou OH.

**2.** Composé selon la revendication 1, **caractérisé en ce que** le composé est choisi parmi :

- le 6'-Méthoxy-1'-phényl-4',6'-dihydro-1'H-spiro[pipéridin-4,4'-furo[3,4-c]pyrazole] ;
- le 1-Benzyl-6'-méthoxy-1'-phényl-4',6'-dihydro-1'H-spiro[pipéridin-4,4'-furo[3,4-c]pyrazole] ;
- le 1-Butyl-6'-méthoxy-1'-phényl-4',6'-dihydro-1'H-spiro[pipéridin-4,4'-furo[3,4-c]pyrazole] ;
- le 1-Benzyl-6'-méthoxy-1'-phényl-6',7'-dihydro-1'H-spiro[pipéridin-4,4'-pyrano[4,3-c]pyrazole] ;
- le 6'-Méthoxy-1'-phényl-1-propyl-6',7'-dihydro-1'H-spiro[pipéridin-4,4'-pyrano[4,3-c]pyrazole] ;
- le 1-Isopropyl-6'-méthoxy-1'-phényl-6',7'-dihydro-1'H-spiro[pipéridin-4,4'-pyrano[4,3-c]pyrazole] ;
- le 1-Butyl-6'-méthoxy-1'-phényl-6',7'-dihydro-1'H-spiro[pipéridin-4,4'-pyrano[4,3-c]pyrazole] ;
- le 1-Isobutyl-6'-méthoxy-1'-phényl-6',7'-dihydro-1'H-spiro[pipéridin-4,4'-pyrano[4,3-c]pyrazole] ;
- le 6'-Méthoxy-1-pentyl-1'-phényl-6',7'-dihydro-1'H-spiro[pipéridin-4,4'-pyrano[4,3-c]pyrazole] ;
- le 1-Isopentyl-6'-méthoxy-1'-phényl-6',7'-dihydro-1'H-spiro[pipéridin-4,4'-pyrano[4,3-c]pyrazole] ;
- le 6'-Méthoxy-1-(3-méthylbut-2-en-1-yl)-1'-phényl-6',7'-dihydro-1'H-spiro[pipéridin-4,4'-pyrano[4,3-c]pyrazole] ;
- le 6'-Méthoxy-1-octyl-1'-phényl-6',7'-dihydro-1'H-spiro[pipéridin-4,4'-pyrano[4,3-c]pyrazole] ;
- le 1-(Cyclohexan-1-ylméthyl)-6'-méthoxy-1'-phényl-6',7'-dihydro-1'H-spiro[pipéridin-4,4'-pyrano[4,3-c]pyrazole] ;
- le 6'-Méthoxy-1'-phényl-1-(2-phényléthyl)-6',7'-dihydro-1'H-spiro[pipéridin-4,4'-pyrano[4,3-c]pyrazole] ;
- le 6'-Méthoxy-1'-phényl-1-(3-phénylpropyl)-6',7'-dihydro-1'H-spiro[pipéridin-4,4'-pyrano[4,3-c]pyrazole] ;
- le 6'-Méthoxy-1'-phényl-1-(4-phénylbutyl)-6',7'-dihydro-1'H-spiro[pipéridin-4,4'-pyrano[4,3-c]pyrazole] ;
- le 1-(Furan-2-ylméthyl)-6'-méthoxy-1'-phényl-6',7'-dihydro-1'H-spiro[pipéridin-4,4'-pyrano[4,3-c]pyrazole] ;
- le 6'-Méthoxy-1-(4-méthoxybenzyl)-1'-phényl-6',7'-dihydro-1'H-spiro[pipéridin-4,4'-pyrano[4,3-c]pyrazole] ;
- le 1-(4-Fluorbenzyl)-6'-méthoxy-1'-phényl-6',7'-dihydro-1'H-spiro[pipéridin-4,4'-pyrano[4,3-c]pyrazole] ;
- le 1-Benzyl-6'-méthoxy-1'-méthyl-6',7'-dihydro-1'H-spiro[pipéridin-4,4'-pyrano[4,3-c]pyrazole] ;
- le 6'-Méthoxy-1'-méthyl-6',7'-dihydro-1'H-spiro[pipéridin-4,4'-pyrano[4,3-c]pyrazole] ;
- le 6'-Méthoxy-1'-méthyl-1-(3-phénylpropyl)-6',7'-dihydro-1'H-spiro[pipéridin-4,4'-pyrano[4,3-c]pyrazole] ;
- le 1-(4-Fluorbenzyl)-6'-méthoxy-1'-methyl-6',7'-dihydro-1'H-spiro[pipéridin-4,4'-pyrano[4,3-c]pyrazole] ;
- le 1-Isopentyl-6'-méthoxy-1'-méthyl-6',7'-dihydro-1'H-spiro[pipéridin-4,4'-pyrano[4,3-c]pyrazole] ;
- le 6'-Méthoxy-1'-méthyl-1-propyl-6',7'-dihydro-1'H-spiro[pipéridin-4,4'-pyrano[4,3-c]pyrazole] ;
- le 1-Benzyl-1'-méthyl-6',7'-dihydro-1'H-spiro[pipéridin-4,4'-pyrano[4,3-c]pyrazole]-6'-ol ;
- le 1-Benzyl-1'-méthyl-1'H-spiro[pipéridin-4,4'-pyrano[4,3-c]pyrazole] ;
- le 1-Benzyl-1'-méthyl-6',7'-dihydro-1'H-spiro[pipéridin-4,4'-pyrano[4,3-c]pyrazole] ; ou
- le 1'-Méthyl-6',7'-dihydro-1'H-spiro[pipéridin-4,4'-pyrano[4,3-c]pyrazole] ;
- le 1-Benzyl-6'-hydroxy-1'-phényl-6',7'-dihydro-1'H-spiro[pipéridin-4,4'-pyrano[4,3-c]pyrazole] ;
- le 1-Benzyl-1'-phényl-6',7'-dihydro-1'H-spiro[pipéridin-4,4'-pyrano[4,3-c]pyrazole] ;
- le 1'-phényl-6',7'-dihydro-1'H-spiro[pipéridin-4,4'-pyrano[4,3-c]pyrazole] ;
- le 1-Isopentyl-1'-phényl-6',7'-dihydro-1'H-spiro[pipéridin-4,4'-pyrano[4,3-c]pyrazole] ;
- le 1-(3-méthylbut-2-en-1-yl)-1'-phényl-6',7'-dihydro-1'H-spiro[pipéridin-4,4'-pyrano[4,3-c]pyrazole] ;
- le 1'-phényl-1-(3-phénylpropyl)-6',7'-dihydro-1'H-spiro[pipéridin-4,4'-pyrano[4,3-c]pyrazole] ;
- le 1-Isopentyl-1'-méthyl-6',7'-dihydro-1'H-spiro[pipéridin-4,4'-pyrano[4,3-c]pyrazole] ;
- le 1-(3-méthylbut-2-en-1-yl)-1'-méthyl-6',7'-dihydro-1'H-spiro[pipéridin-4,4'-pyrano[4,3-c]pyrazole] ;
- le 1'-méthyl-1-(3-phénylpropyl)-6',7'-dihydro-1'H-spiro[pipéridin-4,4'-pyrano[4,3-c]pyrazole] ; ou
- le 1-(Cyclohexan-1-ylméthyl)-1'-phényl-6',7'-dihydro-1'H-spiro[pipéridin-4,4'-pyrano[4,3-c]pyrazole]

facultativement sous la forme de l'un des stéréoisomères, de préférence énantiomères ou diastéréomères, d'un racémate ou sous la forme d'un mélange d'au moins deux des stéréoisomères, de préférence énantiomères et/ou diastéréomères, selon un quelconque rapport de mélange, ou l'un de ses sels correspondants ou de ses solvates correspondants.

**3.** Composé selon la revendication 1, **caractérisé en ce que** le composé est choisi parmi :

- le 6'-Méthoxy-1'-phényl-4',6'-dihydro-1'H-spiro[pipéridin-4,4'-furo[3,4-c]pyrazole] ;
- le 1-Benzyl-6'-méthoxy-1'-phényl-4',6'-dihydro-1'H-spiro[pipéridin-4,4'-furo[3,4-c]pyrazole] ;
- le 1-Butyl-6'-méthoxy-1'-phényl-4',6'-dihydro-1'H-spiro[pipéridin-4,4'-furo[3,4-c]pyrazole] ;
- le 1-Benzyl-6'-méthoxy-1'-phényl-6',7'-dihydro-1'H-spiro[pipéridin-4,4'-pyrano[4,3-c]pyrazole] ;

- le 6'-Méthoxy-1'-phényl-1-propyl-6',7'-dihydro-1'H-spiro[pipéridin-4,4'-pyrano[4,3-c]pyrazole] ;
- le 1-Isopropyl-6'-méthoxy-1'-phényl-6',7'-dihydro-1'H-spiro[pipéridin-4,4'-pyrano[4,3-c]pyrazole] ;
- le 1-Butyl-6'-méthoxy-1'-phényl-6',7'-dihydro-1'H-spiro[pipéridin-4,4'-pyrano[4,3-c]pyrazole] ;
- le 1-Isobutyl-6'-méthoxy-1'-phényl-6',7'-dihydro-1'H-spiro[pipéridin-4,4'-pyrano[4,3-c]pyrazole] ;
- le 6'-Méthoxy-1-pentyl-1'-phényl-6',7'-dihydro-1'H-spiro[pipéridin-4,4'-pyrano[4,3-c]pyrazole] ;
- le 1-Isopentyl-6'-méthoxy-1'-phényl-6',7'-dihydro-1'H-spiro[pipéridin-4,4'-pyrano[4,3-c]pyrazole] ;
- le 6'-Méthoxy-1-(3-méthylbut-2-en-1-yl)-1'-phényl-6',7'-dihydro-1'H-spiro[pipéridin-4,4'-pyrano[4,3-c]pyrazole] ;
- le 6'-Méthoxy-1-octyl-1'-phényl-6',7'-dihydro-1'H-spiro[pipéridin-4,4'-pyrano[4,3-c]pyrazole] ;
- le 1-(Cyclohexan-1-ylméthyl)-6'-méthoxy-1'-phényl-6',7'-dihydro-1'H-spiro[pipéridin-4,4'-pyrano[4,3-c]pyrazole] ;
- le 6'-Méthoxy-1'-phenyl-1-(2-phényléthyl)-6',7'-dihydro-1'H-spiro[pipéridin-4,4'-pyrano[4,3-c]pyrazole] ;
- le 6'-Méthoxy-1'-phényl-1-(3-phénylpropyl)-6',7'-dihydro-1'H-spiro[pipéridin-4,4'-pyrano[4,3-c]pyrazole] ;
- le 6'-Méthoxy-1'-phenyl-1-(4-phénylbutyl)-6',7'-dihydro-1'H-spiro[pipéridin-4,4'-pyrano[4,3-c]pyrazole] ;
- le 1-(Furan-2-ylméthyl)-6'-méthoxy-1'-phényl-6',7'-dihydro-1'H-spiro[pipéridin-4,4'-pyrano[4,3-c]pyrazole] ;
- le 6-Méthoxy-1-(4-méthoxybenzyl)-1'-phényl-6',7'-dihydro-1'H-spiro[pipéridin-4,4'-pyrano[4,3-c]pyrazole] ;
- le 1-(4-Fluorbenzyl)-6'-méthoxy-1'-phényl-6',7'-dihydro-1'H-spiro[pipéridin-4,4'-pyrano[4,3-c]pyrazole] ;
- le 1-Benzyl-6'-méthoxy-1'-méthyl-6',7'-dihydro-1'H-spiro[pipéridin-4,4'-pyrano[4,3-c]pyrazole] ;
- le 6'-Methoxy-1'-methyl-6',7'-dihydro-1'H-spiro[piperidin-4,4'-pyrano[4,3-c]pyrazole] ;
- le 6'-Méthoxy-1'-méthyl-1-(3-phénylpropyl)-6',7'-dihydro-1'H-spiro[pipéridin-4,4'-pyrano[4,3-c]pyrazole] ;
- le 1-(4-Fluorbenzyl)-6'-méthoxy-1'-méthyl-6',7'-dihydro-1'H-spiro[pipéridin-4,4'-pyrano[4,3-c]pyrazole] ;
- le 1-Isopentyl-6'-méthoxy-1'-méthyl-6',7'-dihydro-1'H-spiro[pipéridin-4,4'-pyrano[4,3-c]pyrazole] ;
- le 6'-Méthoxy-1'-méthyl-1-propyl-6',7'-dihydro-1'H-spiro[pipéridin-4,4'-pyrano[4,3-c]pyrazole] ;
- le 1-Benzyl-1'-méthyl-6',7'-dihydro-1'H-spiro[pipéridin-4,4'-pyrano[4,3-c]pyrazole]-6'-ol ;
- le 1-Benzyl-1'-méthyl-1'H-spiro[pipéridin-4,4'-pyrano[4,3-c]pyrazole] ;
- le 1-Benzyl-1'-méthyl-6',7'-dihydro-1'H-spiro[pipéridin-4,4'-pyrano[4,3-c]pyrazole] ; ou
- le 1'-Méthyl-6',7'-dihydro-1'H-spiro[pipéridin-4,4'-pyrano[4,3-c]pyrazole] ;

facultativement sous la forme de l'un des stéréoisomères, de préférence énantiomères ou diastéréomères, d'un racémate ou sous la forme d'un mélange d'au moins deux des stéréoisomères, de préférence énantiomères et/ou diastéréomères, selon un quelconque rapport de mélange, ou l'un de ses sels correspondants ou de ses solvates correspondants.

**4.** Composé selon la revendication 1, **caractérisé en ce que** le composé est
le 1-Benzyl-1'-phényl-1'H-spiro[pipéridin-4,4'-pyrano[4,3-c]pyrazole] ;
facultativement sous la forme de l'un des stéréoisomères, de préférence énantiomères ou diastéréomères, d'un racémate ou sous la forme d'un mélange d'au moins deux des stéréoisomères, de préférence énantiomères et/ou diastéréomères, selon un quelconque rapport de mélange, ou l'un de ses sels correspondants ou de ses solvates correspondants.

**5.** Procédé de production d'un composé de formule Ia, dans lequel un composé de formule IIIa

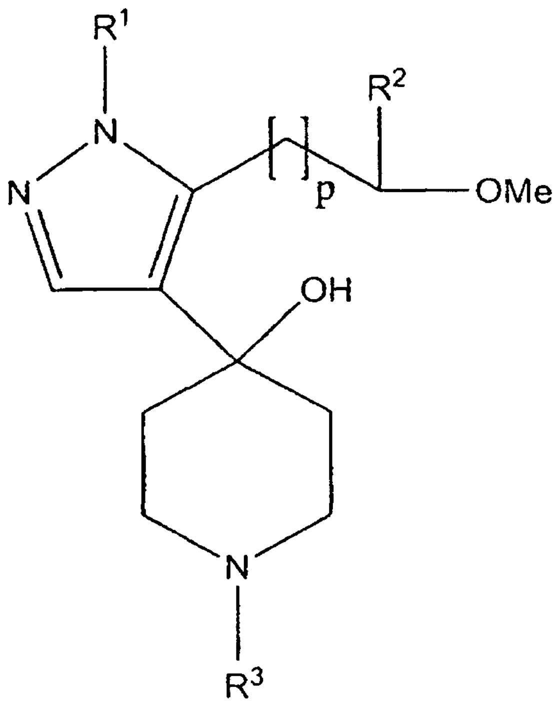

dans laquelle $R^1$, $R^2$, $R^3$ et p sont tels que définis dans la revendication 1, est mis à réagir avec de l'acide pour former un composé de formule Ia.

**6.** Procédé selon la revendication 5, dans lequel, lors d'une étape ultérieure, un composé de formule Ia, dans laquelle p vaut 0 et $R^3$ est C(O)OR', est mis à réagir avec une solution de KOH dans de l'eau pour former un composé de formule Ia, $R^3$ étant H
ou
lors d'une étape ultérieure, un composé de formule Ia, dans laquelle p vaut 1 et $R^3$ est du benzyle, est mis à réagir avec $NH_4^+HCOO^-$ avec un catalyseur Pd/C pour former un composé de formule Ia, $R^3$ étant H, de préférence lors d'une étape ultérieure, un composé de formule Ia, dans laquelle $R^3$ est de l'hydrogène, est mis à réagir avec un composé $R^3$-X, X étant un groupe partant et $K_2CO_3$ sous reflux pour former un composé de formule Ia, $R^3$ étant tel que défini dans la revendication 1, sauf H.

**7.** Composition pharmaceutique qui comprend un composé selon la revendication 1 ou l'un de ses sels ou solvates pharmaceutiquement acceptables, et un support, adjuvant ou véhicule pharmaceutiquement acceptable.

**8.** Utilisation d'un composé selon l'une quelconque des revendications 1 à 4 pour la production d'un médicament pour le traitement ou la prophylaxie d'une maladie médiée par le récepteur sigma,
dans laquelle la maladie est la diarrhée, les troubles lipoprotéiques, le syndrome métabolique, le traitement des niveaux de triglycérides élevés, la chylomicronémie, l'hyperlipoprotéinémie ; l'hyperlipidémie, notamment l'hyperlipidémie mixte ; l'hypercholestérolémie, la dysbêtalipoprotéinémie, l'hypertriglycéridémie, notamment le trouble sporadique et familial (hypertriglycéridémie héréditaire), la migraine, l'obésité, l'arthrite, l'hypertension, l'arythmie, l'ulcère, les troubles de l'apprentissage, de la mémoire et de l'attention, les troubles cognitifs, les maladies neurodégénératives, les maladies démyélinisantes, l'addiction aux médicaments et aux substances chimiques, notamment la cocaïne, l'amphétamine, l'éthanol et la nicotine, la diskinésie tardive, l'accident ischémique cérébral, l'épilepsie, l'accident vasculaire cérébral, la dépression, le stress, la psychose, la schizophrénie ; l'inflammation, les maladies auto-immunes ou le cancer, ou
dans laquelle la maladie est la douleur, notamment la douleur neuropathique, la douleur inflammatoire ou d'autres conditions douloureuses, l'allodynie et/ou l'hyperalgésie, notamment l'allodynie mécanique.

**9.** Composé selon l'une quelconque des revendications 1 à 4 pour une utilisation dans le traitement d'une maladie médiée par le récepteur sigma,
dans lequel la maladie est la diarrhée, les troubles lipoprotéiques, le syndrome métabolique, le traitement des niveaux de triglycérides élevés, la chylomicronémie, l'hyperlipoprotéinémie ; l'hyperlipidémie, notamment l'hyperlipidémie mixte ; l'hypercholestérolémie, la dysbêtalipoprotéinémie, l'hypertriglycéridémie, notamment le trouble sporadique et familial (hypertriglycéridémie héréditaire), la migraine, l'obésité, l'arthrite, l'hypertension, l'arythmie, l'ulcère, les troubles de l'apprentissage, de la mémoire et de l'attention, les troubles cognitifs, les maladies neurodégénératives, les maladies démyélinisantes, l'addiction aux médicaments et aux substances chimiques, notamment la cocaïne, l'amphétamine, l'éthanol et la nicotine, la diskinésie tardive, l'accident ischémique cérébral, l'épilepsie, l'accident vasculaire cérébral, la dépression, le stress, la psychose, la schizophrénie ; l'inflammation, les maladies auto-immunes ou le cancer, ou

dans laquelle la maladie est la douleur, notamment la douleur neuropathique, la douleur inflammatoire ou d'autres conditions douloureuses, l'allodynie et/ou l'hyperalgésie, notamment l'allodynie mécanique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003035065 A
- WO 2007001939 A
- FR 2875230


### Non-patent literature cited in the description


- **WALKER, J.M. et al.** *Pharmacological Reviews,* 1990, vol. 42, 355
- **SNYDER, S.H. ; LARGENT, B.L.** *J. Neuropsychiatry,* 1989, vol. 1, 7
- **QUIRION, R. et al.** *Trends Pharmacol. Sci.,* 1992, vol. 13, 85-86
- **HANNER, M. et al.** *Proc. Natl. Acad. Sci.,* 1996, vol. 93, 8072-8077
- **MAIER et al.** *J Med Chem,* 2002, vol. 45, 438-448
- **MAIER et al.** *J Med Chem,* 2002, vol. 45, 4923-4930
- **DEHAVEN-HUDKINS, D. L. ; L.C. FLEISSNER ; F. Y. FORD-RICE.** Characterization of the binding of [3H](+)pentazocine to σ recognition sites in guinea pig brain. *Eur. J. Pharmacol.,* 1992, vol. 227, 371-378
- **RADESCA, L. ; W.D. BOWEN ; L. DI PAOLO ; B.R. DE COSTA.** Synthesis and Receptor Binding of Enantiomeric N-Substituted cis-N-[2-(3,4-Dichlorophenyl)ethyl]-2-(1-pyrrolidinyl)cyclohexylamines as High-Affinity σ Receptor Ligands. *J. Med. Chem.,* 1991, vol. 34, 3065-3074
- **LANGA, F. ; CODONY X. ; TOVAR V. ; LAVADO A. ; GIMÉNEZ E. ; COZAR P. ; CANTERO M. ; DORDAL A. ; HERNÁNDEZ E. ; PÉREZ R.** Generation and phenotypic analysis of sigma receptor type I (Sigma1) knockout mice. *European Journal of Neuroscience,* 2003, vol. 18, 2188-2196
- **LOWRY, O.H. ; N.J. ROSEBROUGH ; A.L. FARR ; R.J. RANDALL.** Protein measurement with the Folin phenol reagent. *J. Biol. Chem,* 1951, vol. 193, 265